# FASCICULE DE BREVET EUROPEEN

(11) **EP 4 341 397 B1**
(45) Date de publication et mention de la délivrance du brevet: **26.11.2025**
(21) Numéro de dépôt: 22737844.5
(22) Date de dépôt: 24.06.2022
(51) Int. Cl.: C12N 9/22, C12N 15/10, C12N 15/90

(54) **COMPOSITION ET MÉTHODE PERMETTANT L'ÉDITION GÉNOMIQUE**
ZUSAMMENSETZUNG UND VERFAHREN ZUR GENOMEDITIERUNG
COMPOSITION AND METHOD FOR GENOME EDITING

(30) Priorité: 25.06.2021 FR 2106868
(43) Date de publication de la demande: 27.03.2024
(73) Titulaire: Quidditas SA, 4000 Liège (Sart-Tilman) (BE)
(72) Inventeur: CHERBONNEAU, François, 91310 MONTLHÉRY (FR)
(74) Mandataire: LLR
(86) Numéro de dépôt international: PCT/EP2022/067351
(87) Numéro de publication internationale: WO 2022/269037

(56) Documents cités:
- WO-A1-2019/046636
- WO-A1-2021/061832
- KLOMPE SANNE E ET AL: "Transposon-encoded CRISPR-Cas systems direct RNA-guided DNA integration", NATURE, NATURE PUBLISHING GROUP UK, LONDON, vol. 571, no. 7764, 12 June 2019 (2019-06-12), pages 219 - 225, XP036831898, ISSN: 0028-0836, [retrieved on 20190612], DOI: 10.1038/S41586-019-1323-Z
- MORIARITY BRANDEN S. ET AL: "Modular assembly of transposon integratable multigene vectors using RecWay assembly", vol. 41, no. 8, 1 April 2013 (2013-04-01), GB, pages e92 - e92, XP055919256, ISSN: 0305-1048, Retrieved from the Internet <URL:https://watermark.silverchair.com/gkt115.pdf?token=AQECAHi208BE49Ooan9kkhW_Ercy7Dm3ZL_9Cf3qfKAc485ysgAAAtAwggLMBgkqhkiG9w0BBwagggK9MIICuQIBADCCArIGCSqGSIb3DQEHATAeBglghkgBZQMEAS4wEQQMghSykix3GnbnsRg3AgEQgIICgxhhEjk4CGu901FL7BLEZFR874_GYKchEUcU6fiEKuYmo8NIDpvKm6FPsGcoI_TSV_hwU4FIq81bnEeIxMEwHKDsOGEI0> DOI: 10.1093/nar/gkt115
- E. L. ARONOVICH ET AL: "The Sleeping Beauty transposon system: a non-viral vector for gene therapy", HUMAN MOLECULAR GENETICS, vol. 20, no. R1, 15 April 2011 (2011-04-15), pages R14 - R20, XP055177523, ISSN: 0964-6906, DOI: 10.1093/hmg/ddr140
- MIYAMOTO TATSUO ET AL: "Updated summary of genome editing technology in human cultured cells linked to human genetics studies", JOURNAL OF HUMAN GENETICS, SPRINGER SINGAPORE, SINGAPORE, vol. 63, no. 2, 11 October 2017 (2017-10-11), pages 133 - 143, XP036428428, ISSN: 1434-5161, [retrieved on 20171011], DOI: 10.1038/S10038-017-0349-Z

## Description

L'invention concerne une composition et une méthode permettant l'édition génomique.

La volonté de comprendre les effets de modifications de l'information génétique des cellules vivantes remonte aux premiers pas de la génétique.

Tout d'abord la génétique classique a tenté de comprendre les modifications génétiques, et le phénotype qui en découle, en sélectionnant des sites génétiques déterminés.

Par la suite, les biochimistes ont utilisé des rayonnements et des agents mutagènes chimiques pour augmenter la probabilité de mutations génétiques dans les organismes expérimentaux. Bien que très utiles, ces méthodes sont couteuses, et ne permettent pas de contrôler simplement les modifications introduites dans le matériel génétique.

La biologie moléculaire et la connaissance des mécanismes moléculaires de réparation ou de défense des cellules contre des organismes hôte a permis de développer de nombreuses technologies, permettant le développement d'une génétique dite inverse, dont l'objectif est à l'opposé des criblages génétiques dits classiques.

La génétique inverse a pour but d'introduire des mutations au sein des matériels génétiques dans le but de mesurer et d'analyser les effets phénotypiques qui en découlent.

Les stratégies d'édition du génome ont évolué au cours des trois dernières décennies, et la plus récente innovation et révolution dans le cadre de la modification génique ciblée correspond au Système CRISPR/CRISPR associé à la protéine 9 (Cas9) (CRISPR/Cas-9). A ce titre on peut citer les deux brevets EP 3 144 390 B1 et US 8 697 359 B1, qui décrivent tous deux cette technologie.

Le système CRISPR/Cas-9 s'est dès lors imposé comme l'outil de référence pour les modifications génétiques. Néanmoins cette révolution CRISPR s'apparente à des ciseaux moléculaires, ce qui n'est pas suffisant pour permettre une recombinaison totale d'un exon entier.

Plus récemment, la transposase encodée par le système CRISPR (Transposon-encoded CRISPR-Cas systems) est une demi-réponse aux manques de la technologie CRISPR. En effet, cette technologie utilise à la fois une intégration ciblée par l'intermédiaire du transposon Tn7 puis un ajout de séquence dans le génome par la technologie CRISPR. Mais en aucun cas il ne s'agit d'une recombinaison.

La technologie du *Prime Editing* est également une possibilité de remplacement génétique associant l'outil CRISPR avec une reverse transcriptase. Cette dernière évolution dans l'édition du génome permet un remplacement, dépendant de la taille, d'un des deux brins d'ADN, ce qui reste une limitation forte inhérente et tributaire du système d'intégration et de réparation cellulaire.

Aussi, l'invention a-t-elle pour but de pallier ces inconvénients de l'art antérieur.

Un des buts de l'invention est de fournir un outil de recombinaison permettant l'édition du génome.

Un autre but de l'invention est que ce nouvel outil ne soit pas dépendant de la taille de la séquence manipulée, et que ce système soit contrôlé et contrôlable par l'utilisateur.

Encore un autre but de l'invention est de fournir un outil qui permette, au bon vouloir de l'utilisateur un remplacement simple brin ou double brin d'une molécule d'intérêt, et en faisant appel au système de réparation de l'ADN de manière contrôlée et limitée.

L'invention concerne une première molécule d'acides nucléiques simple brin comprenant ou constituée essentiellement d'une séquence A permettant l'insertion d'une séquence complémentaire d'un acide nucléique d'intérêt,

ladite séquence A étant liée en 5' à une première séquence riches en T de 40 à 60 nucléotides de long et en 3' à une deuxième séquence riches en T de 40 à 60 nucléotides de long, lesdites première et deuxième séquence riches en T comprenant respectivement un premier et un deuxième domaine de 6 à 12 nucléotides riches en G/C, la séquence du premier domaine étant complémentaire de la séquence du deuxième domaine, ledit premier et deuxième domaine étant positionnés de 15 à 52 nucléotides de ladite séquence A, ladite première molécule comprenant en son extrémité 5' une première séquence orientés 5'-3' de reconnaissance d'une transposase et en son extrémité 3' au moins une deuxième séquence de reconnaissance de ladite transposase

Aussi l'invention concerne-t-elle un complexe moléculaire comprenant :
- une première molécule d'acides nucléiques simple brin comprenant ou constituée essentiellement d'une séquence A permettant l'insertion d'une séquence complémentaire d'un acide nucléique d'intérêt,
   ladite séquence A étant liée en 5' à une première séquence riches en A/T, notamment riches en T, de 40 à 60 nucléotides de long et en 3' à une deuxième séquence riches en A/T, notamment riches en T, de 40 à 60 nucléotides de long, lesdites première et deuxième séquence riches en A/T, notamment riches en T, comprenant respectivement un premier et un deuxième domaine de 6 à 12 nucléotides riches en G/C, la séquence du premier domaine étant complémentaire de la séquence du deuxième domaine, ledit premier et deuxième domaine étant positionnés de 15 à 52 nucléotides de ladite séquence A, ladite première molécule comprenant en son extrémité 5' une première séquence orientés 5'-3' de reconnaissance d'une transposase et en son extrémité 3' au moins une deuxième séquence de reconnaissance de ladite transposase ; et
- une deuxième molécule d'acides nucléiques simple brin comprenant ou constituée essentiellement en son extrémité 5' d'au moins une séquence complémentaire de ladite deuxième séquence de reconnaissance de ladite transposase,
   ledit complexe étant tel que les première et deuxième molécules d'acides nucléiques simple brin sont appariées selon la complémentarité des bases définie par Watson et Crick de sorte à définir deux sites double brin de liaison de ladite transposase.

Cela signifie que l'invention concerne un complexe moléculaire comprenant une première molécule d'acides nucléiques simple brin et une deuxième molécule d'acides nucléiques simple brin, ladite deuxième molécule d'acides nucléiques simple brin comprenant ou constituée essentiellement en son extrémité 5' d'au moins une séquence complémentaire de ladite deuxième séquence de reconnaissance de ladite transposase,
ledit complexe étant tel que les première et deuxième molécules d'acides nucléiques simple brin sont appariées selon la complémentarité des bases définie par Watson et Crick de sorte à définir deux sites double brin de liaison de ladite transposase.

L'invention repose sur l'observation inattendue faite par l'inventeur que l'utilisation de guides simples brins spécifiques capables de cibler une région d'un acide nucléique d'intérêt permettent de mobiliser de manière contrôlée et « site spécifique » des transposases, et ainsi utiliser les propriétés de recombinaison desdites transposases pour réaliser du remplacement de séquences dans des molécules d'intérêt.

Le complexe moléculaire susmentionné est en fait l'unité de base de la technologie définie dans l'invention. Cette unité de base sert à guider les recombinases sur un site spécifique où doit avoir lieu la recombinaison, et donc le remplacement de séquence. Contrairement au système CRISPR/Cas9 qui requiert la présence de séquences de type PAM (NGG), l'outil moléculaire défini ici peut être utilisé sur n'importe quelle séquence cible, quelle qu'en soit sa séquence.

Le complexe moléculaire susmentionné est donc l'unité de base qui devra être complétée par :
- une région homologie de la séquence cible et
- une région de remplacement de la séquence cible.

Il s'agit donc ici d'un produit intermédiaire de l'outil tel que décrit ci-après.

Le complexe moléculaire est constitué de deux molécules d'acides nucléiques simples brins, qui peuvent être des molécules d'ADN, des molécules d'ARN ou des molécules mixtes d'ARN et d'ADN.

Ces deux molécules sont partiellement complémentaires entre elle, selon la complémentarité des bases d'acides nucléiques définie par Watson et Crick, c'est-à-dire qu'une Adénine s'apparie à un Thymidine ou un Uracile, et une Cytosine s'apparie à une Guanine, et réciproquement.

Plus particulièrement les deux molécules formant le complexe susmentionné comprennent chacune la séquence d'un des brins d'une molécule double brin correspondant à la séquence de fixation d'une transposase. Aussi, chaque molécule simple brin comprend donc une « demie séquence » de fixation de transposase et ne peut donc pas permettre une interaction avec ladite transposase correspondante. En revanche, lors que les deux molécules du complexe interagissent ensemble, par appariement des bases tel que défini ci-dessus, une molécule double brin est ainsi formée, reconstituant un site de liaison double brin de ladite transposase, celle-ci pouvant ainsi interagir avec la molécule formée.

### La première molécule.

La première molécule du complexe est la molécule qui comprendra, une fois modifiée, une séquence d'acide nucléique qui permettra de cibler spécifiquement une région d'intérêt d'une molécule d'acide nucléique d'intérêt. Cette séquence d'intérêt sera choisie par l'utilisateur du système selon la cible choisie. Cette séquence d'intérêt sera insérée dans la première molécule dudit complexe au niveau de la région A. Cette région A correspond *a minima* à deux acides nucléiques entre lesquels sera inséré la séquence permettant de cibler la molécule cible. Au vu de la structure orientée des acides nucléiques (sens 5'-3'), il est important que la séquence permettant de cibler la région d'intérêt soit positionnée dans le bon sens, afin que l'appariement soit possible avec la séquence cible.

Aussi, avantageusement, la région A comprend un ou plusieurs sites reconnaissant des enzymes de restrictions afin de favoriser une insertion orientée. Un ou plusieurs des sites suivants peuvent être présents dans la région A :

**[Tableaux1]**

| | | | |
|---|---|---|---|
| SEQ ID NO : | 29 | AA/CGTT | AclI |
| SEQ ID NO : | 30 | A/AGCTT | HindIII |
| SEQ ID NO : | 31 | AAT/ATT | SspI |
| SEQ ID NO : | 32 | /AATT | MluCI |
| SEQ ID NO : | 33 | A/CATGT | PciI |
| SEQ ID NO : | 34 | A/CCGGT | AgeI |
| SEQ ID NO : | 35 | ACCTGC(4/8) | BfuAI BspM I |
| SEQ ID NO : | 36 | A/CCWGGT | SexAI |
| SEQ ID NO : | 37 | A/CGCGT | MluI |
| SEQ ID NO : | 38 | ACGGC(12/14) | BceAI |
| SEQ ID NO : | 39 | A/CGT | HpyCH4IV |
| SEQ ID NO : | 40 | ACN/GT | HpyCH4III |
| SEQ ID NO : | 41 | (10/15)ACNNNNGTAYC(12/7) | BaeI |
| SEQ ID NO : | 42 | (9/12)ACNNNNNCTCC(10/7) | BsaXI |
| SEQ ID NO : | 43 | A/CRYGT | AflIII |
| SEQ ID NO : | 44 | A/CTAGT | SpeI |
| SEQ ID NO : | 45 | ACTGG(1/-1) | BsrI |
| SEQ ID NO : | 46 | ACTGGG(5/4) | BmrI |
| SEQ ID NO : | 47 | A/GATCT | BglII |
| SEQ ID NO : | 48 | AGC/GCT | AfeI |
| SEQ ID NO : | 49 | AG/CT | AluI |
| SEQ ID NO : | 50 | AGG/CCT | StuI |
| SEQ ID NO : | 51 | AGT/ACT | ScaI- |
| SEQ ID NO : | 52 | AT/CGAT | ClaI BspDI |
| SEQ ID NO : | 53 | ATCTATGTCGGGTGCGGAGAAAGAGG TAAT(-15/-19) | PI-SceI |
| SEQ ID NO : | 54 | ATGCA/T | NsiI |
| SEQ ID NO : | 55 | AT/TAAT | AseI |
| SEQ ID NO : | 56 | ATTT/AAAT | SwaI |
| SEQ ID NO : | 57 | (11/13)CAANNNNNGTGG(12/10) | CspCI |
| SEQ ID NO : | 58 | C/AATTG | MfeI |
| SEQ ID NO : | 59 | CACCTGC(4/8) | PaqCI |
| SEQ ID NO : | 60 | CACGAG | Nb.BssSI |
| SEQ ID NO : | 61 | CACGAG(-5/-1) | BssSI-v2 |
| SEQ ID NO : | 62 | CACGTC(-3/-3) | BmgBI |
| SEQ ID NO : | 63 | CAC/GTG | PmlI |
| SEQ ID NO : | 64 | CACNNN/GTG | DraIII |
| SEQ ID NO : | 65 | CACNN/NNGTG | AleI-v2 |
| SEQ ID NO : | 66 | CAGCAG(25/27) | EcoP15I |
| SEQ ID NO : | 67 | CAG/CTG | PvuII |
| SEQ ID NO : | 68 | CAGNNN/CTG | AlwNI |
| SEQ ID NO : | 69 | CAGTG(2/0) | BtsIMutI |
| SEQ ID NO : | 70 | CA/TATG | NdeI |
| SEQ ID NO : | 71 | CATG/ | NlaIII |
| SEQ ID NO : | 72 | /CATG | FatI |
| SEQ ID NO : | 73 | C/ATG | CviAII |
| SEQ ID NO : | 74 | CAYNN/NNRTG | MslI |
| | | CC(12/16) | FspEI |
| SEQ ID NO : | 75 | CCANNNNN/NNNNTGG | XcmI |
| SEQ ID NO : | 76 | CCANNNNN/NTGG | BstXI |
| SEQ ID NO : | 77 | CCANNNN/NTGG | PflMI |
| SEQ ID NO : | 78 | CCATC(4/5) | BccI |
| SEQ ID NO : | 79 | C/CATGG | NcoI |
| SEQ ID NO : | 80 | CCCAGC(-5/-1) | BseYI |
| SEQ ID NO : | 81 | CCCGC(4/6) | FauI |
| SEQ ID NO : | 82 | CCC/GGG | SmaI |
| SEQ ID NO : | 83 | C/CCGGG(0/-1)CCD | TspMI XmaI Nt.CviPII |
| SEQ ID NO : | 84 | CCDG(10/14) | LpnPI |
| SEQ ID NO : | 85 | CCGC(-3/-1) | AciI |
| SEQ ID NO : | 86 | CCGC/GG | SacII |
| SEQ ID NO : | 87 | CCGCTC(-3/-3) | BsrBI |
| SEQ ID NO : | 88 | C/CGG | MspI HpaII |
| SEQ ID NO : | 89 | CC/NGG | ScrFI |
| SEQ ID NO : | 90 | /CCNGG | StyD4I |
| SEQ ID NO : | 91 | C/CNNGG | BsaJI |
| SEQ ID NO : | 92 | CCNNNNN/NNGG | BslI |
| SEQ ID NO : | 93 | C/CRYGG | BtgI |
| SEQ ID NO : | 94 | CC/SGG | NciI |
| SEQ ID NO : | 95 | C/CTAGG | AvrII |
| SEQ ID NO : | 96 | CCTC(7/6) | MnlI |
| SEQ ID NO : | 97 | CCTCAGC | Nb.BbvCI |
| SEQ ID NO : | 98 | CCTCAGC(-5/-7) | Nt.BbvCI |
| SEQ ID NO : | 99 | CCTCAGC(-5/-2) | BbvCI |
| SEQ ID NO : | 100 | CCTGCA/GG | SbfI |
| SEQ ID NO : | 101 | CCTNAGC(-5/-2) | Bpu10I |
| SEQ ID NO : | 102 | CC/TNAGG | Bsu36I |
| SEQ ID NO : | 103 | CCTNN/NNNAGG | EcoNI |
| SEQ ID NO : | 104 | CCTTC(6/5) | HpyAV |
| SEQ ID NO : | 105 | /CCWGG | PspGI |
| SEQ ID NO : | 106 | CC/WGG | BstNI |
| SEQ ID NO : | 107 | C/CWWGG | StyI |
| SEQ ID NO : | 108 | (10/12)CGANNNNNNTGC(12/10) | BcgI |
| SEQ ID NO : | 109 | CGAT/CG | PvuI |
| SEQ ID NO : | 110 | CG/CG | BstUI |
| SEQ ID NO : | 111 | C/GGCCG | EagI |
| SEQ ID NO : | 112 | CG/GWCCG | RsrII |
| SEQ ID NO : | 113 | CGRY/CG | BsiEI |
| SEQ ID NO : | 114 | C/GTACG | BsiWI |
| SEQ ID NO : | 115 | CGTCTC | BsmBI-v2 |
| SEQ ID NO : | 116 | CGTCTC(1/5) | Esp3I |
| SEQ ID NO : | 117 | CGWCG/ | Hpy99I |
| SEQ ID NO : | 118 | CMG/CKG | MspA1I |
| SEQ ID NO : | 119 | CNNNNNNNNNNN/NNNNNNNNNG | AbaSI |
| SEQ ID NO : | 120 | CNNR(9/13) | MspJI |
| SEQ ID NO : | 121 | CR/CCGGYG | SgrAI |
| SEQ ID NO : | 122 | C/TAG | BfaI |
| SEQ ID NO : | 123 | CTCAG(9/7) | BspCNI |
| SEQ ID NO : | 124 | C/TCGAG | XhoI PaeR7I |
| SEQ ID NO : | 125 | CTCTTC(1/4) | EarI |
| SEQ ID NO : | 126 | CTGAAG(16/14) | AcuI |
| SEQ ID NO : | 127 | CTGCA/G | PstI |
| SEQ ID NO : | 128 | CTGGAG(16/14) | BpmI |
| SEQ ID NO : | 129 | C/TNAG | DdeI |
| SEQ ID NO : | 130 | C/TRYAG | SfcI |
| SEQ ID NO : | 131 | C/TTAAG | AflII |
| SEQ ID NO : | 132 | CTTGAG(16/14) | BpuEI |
| SEQ ID NO : | 133 | C/TYRAG | SmlI |
| SEQ ID NO : | 134 | C/YCGRG | BsoBI AvaI |
| SEQ ID NO : | 135 | GAAGA(8/7) | MboII |
| SEQ ID NO : | 136 | GAAGAC(2/6) | BbsI |
| SEQ ID NO : | 137 | GAANN/NNTTC | XmnI |
| SEQ ID NO : | 138 | GAATGC(1/-1) | BsmI |
| SEQ ID NO : | 139 | GAATGC | Nb.BsmI |
| SEQ ID NO : | 140 | G/AATTC | EcoRI |
| SEQ ID NO : | 141 | GACGC(5/10) | HgaI |
| SEQ ID NO : | 142 | GACGT/C | AatII |
| SEQ ID NO : | 143 | GAC/GTC | ZraI |
| SEQ ID NO : | 144 | GACN/NNGTC | PflFI Tth111 I |
| SEQ ID NO : | 145 | GACNN/NNGTC | PshAI |
| SEQ ID NO : | 146 | GACNNN/NNGTC | AhdI |
| SEQ ID NO : | 147 | GACNNNN/NNGTC | DrdI |
| SEQ ID NO : | 148 | GAG/CTC | Eco53kI |
| SEQ ID NO : | 149 | GAGCT/C | SacI |
| SEQ ID NO : | 150 | GAGGAG(10/8) | BseRI |
| SEQ ID NO : | 151 | GAGTC(4/-5) | Nt.BstNBI |
| SEQ ID NO : | 152 | GAGTC(4/5) | PleI |
| SEQ ID NO : | 153 | GAGTC(5/5) | MlyI |
| SEQ ID NO : | 154 | G/ANTC | HinfI |
| SEQ ID NO : | 155 | GAT/ATC | EcoRV |
| SEQ ID NO : | 156 | GA/TC | DpnI |
| SEQ ID NO : | 157 | /GATC | Sau3AI Dpn II MboI |
| SEQ ID NO : | 158 | GATNN/NNATC | BsaBI |
| SEQ ID NO : | 159 | G/AWTC | TfiI |
| SEQ ID NO : | 160 | GCAATG | Nb.BsrDI |
| SEQ ID NO : | 161 | GCAATG(2/0) | BsrDI |
| SEQ ID NO : | 162 | GCAGC(8/12) | BbvI |
| SEQ ID NO : | 163 | GCAGTG(2/0) | BtsI-v2 |
| SEQ ID NO : | 164 | GCAGTG | Nb.BtsI |
| SEQ ID NO : | 165 | GCANNNN/NTGC | BstAPI |
| SEQ ID NO : | 166 | GCATC(5/9) | SfaNI |
| SEQ ID NO : | 167 | GCATG/C | SphI |
| SEQ ID NO : | 168 | GCCC/GGGC | SrfI |
| SEQ ID NO : | 169 | GCCGAG(21/19) | NmeAIII |
| SEQ ID NO : | 170 | G/CCGGC | NgoMIV |
| SEQ ID NO : | 171 | GCC/GGC | NaeI |
| SEQ ID NO : | 172 | GCCNNNN/NGGC | BglI |
| SEQ ID NO : | 173 | GCGAT/CGC | AsiSI |
| SEQ ID NO : | 174 | GCGATG(10/14) | BtgZI |
| SEQ ID NO : | 175 | GCG/C | HhaI |
| SEQ ID NO : | 176 | G/CGC | HinP1I |
| SEQ ID NO : | 177 | G/CGCGC | BssHII |
| SEQ ID NO : | 178 | GC/GGCCGC | NotI |
| SEQ ID NO : | 179 | GC/NGC | Fnu4HI |
| SEQ ID NO : | 180 | GCN/NGC | Cac8I |
| SEQ ID NO : | 181 | GCNNNNN/NNGC | MwoI |
| SEQ ID NO : | 182 | G/CTAGC | NheI |
| SEQ ID NO : | 183 | GCTAG/C | BmtI |
| SEQ ID NO : | 184 | GCTCTTC(1/-7) | Nt.BspQI |
| SEQ ID NO : | 185 | GCTCTTC(1/4) | SapI BspQI |
| SEQ ID NO : | 186 | GC/TNAGC | BlpI |
| SEQ ID NO : | 187 | G/CWGC | ApeKI TseI |
| SEQ ID NO : | 188 | GDGCH/C | Bsp1286I |
| SEQ ID NO : | 189 | GGATC(4/5) | AlwI |
| SEQ ID NO : | 190 | GGATC(4/-5) | Nt.AlwI |
| SEQ ID NO : | 191 | G/GATCC | BamHI |
| SEQ ID NO : | 192 | GGATG(9/13) | FokI |
| SEQ ID NO : | 193 | GGATG(2/0) | BtsCI |
| SEQ ID NO : | 194 | GG/CC | HaeIII |
| SEQ ID NO : | 195 | GGCCGG/CC | FseI |
| SEQ ID NO : | 196 | GGCCNNNN/NGGCC | SfiI |
| SEQ ID NO : | 197 | G/GCGCC | KasI |
| SEQ ID NO : | 198 | GG/CGCC | NarI |
| SEQ ID NO : | 199 | GGCGC/C | PluTI |
| SEQ ID NO : | 200 | GGC/GCC | SfoI |
| SEQ ID NO : | 201 | GG/CGCGCC | AscI |
| SEQ ID NO : | 202 | GGCGGA(11/9) | EciI |
| SEQ ID NO : | 203 | GGGAC(10/14) | BsmFI |
| SEQ ID NO : | 204 | GGGCC/C | ApaI |
| SEQ ID NO : | 205 | G/GGCCC | PspOMI |
| SEQ ID NO : | 206 | G/GNCC | Sau96I |
| SEQ ID NO : | 207 | GGN/NCC | NlaIV |
| SEQ ID NO : | 208 | G/GTACC | Acc65I |
| SEQ ID NO : | 209 | GGTAC/C | KpnI |
| SEQ ID NO : | 210 | GGTCTC(1/5) | BsaI v2 |
| SEQ ID NO : | 211 | GGTGA(8/7) | HphI |
| SEQ ID NO : | 212 | G/GTNACC | BstEII |
| SEQ ID NO : | 213 | G/GWCC | AvaII |
| SEQ ID NO : | 214 | G/GYRCC | BanI |
| SEQ ID NO : | 215 | GKGCM/C | BaeGI |
| SEQ ID NO : | 216 | GR/CGYC | BsaHI |
| SEQ ID NO : | 217 | GRGCY/C | BanII |
| SEQ ID NO : | 218 | GT/AC | RsaI |
| SEQ ID NO : | 219 | G/TAC | CviQI |
| SEQ ID NO : | 220 | GTATAC | BstZ17I |
| SEQ ID NO : | 221 | GTATCC(6/5) | BciVI |
| SEQ ID NO : | 222 | G/TCGAC | SalI |
| SEQ ID NO : | 223 | GTCTC(1/5) | BsmAI Bco DI |
| SEQ ID NO : | 224 | GTCTC(1/-5) | Nt.BsmAI |
| SEQ ID NO : | 225 | G/TGCAC | ApaLI |
| SEQ ID NO : | 226 | GTGCAG(16/14) | BsgI |
| SEQ ID NO : | 227 | GT/MKAC | AccI |
| SEQ ID NO : | 228 | GTN/NAC | Hpy166II |
| SEQ ID NO : | 229 | /GTSAC | Tsp45I |
| SEQ ID NO : | 230 | GTT/AAC | HpaI |
| SEQ ID NO : | 231 | GTTT/AAAC | PmeI |
| SEQ ID NO : | 232 | GTY/RAC | HincII |
| SEQ ID NO : | 233 | GWGCW/C | BsiHKAI |
| SEQ ID NO : | 234 | NNCASTGNN/ | TspRI |
| SEQ ID NO : | 235 | R/AATTY | ApoI |
| SEQ ID NO : | 236 | RCATG/Y | NspI |
| SEQ ID NO : | 237 | R/CCGGY | BsrFI-v2 |
| SEQ ID NO : | 238 | R/GATCY | BstYI |
| SEQ ID NO : | 239 | RGCGC/Y | HaeII |
| SEQ ID NO : | 240 | RG/CY | CviKI-1 |
| SEQ ID NO : | 241 | RG/GNCCY | EcoO109I |
| SEQ ID NO : | 242 | RG/GWCCY | PpuMI |
| SEQ ID NO : | 243 | TAACTATAACGGTCCTAAGGTAGCGAA (-9/-13) | I-CeuI |
| SEQ ID NO : | 244 | TAC/GTA | SnaBI |
| SEQ ID NO : | 245 | TAGGGATAACAGGGTAAT(-9/-13) | I-SceI |
| SEQ ID NO : | 246 | T/CATGA | BspHI |
| SEQ ID NO : | 247 | T/CCGGA | BspEI |
| SEQ ID NO : | 248 | TCCRAC(20/18) | MmeI |
| SEQ ID NO : | 249 | T/CGA | TaqI-v2 |
| SEQ ID NO : | 250 | TCG/CGA | NruI |
| SEQ ID NO : | 251 | TCN/GA | Hpy188I |
| SEQ ID NO : | 252 | TC/NNGA | Hpy188III |
| SEQ ID NO : | 253 | T/CTAGA | XbaI |
| SEQ ID NO : | 254 | T/GATCA | BclI |
| SEQ ID NO : | 255 | TG/CA | HpyCH4V |
| SEQ ID NO : | 256 | TGC/GCA | FspI |
| SEQ ID NO : | 257 | TGGCAAACAGCTATTATGGGTATTATG GGT(-13/-17) | PI-PspI |
| SEQ ID NO : | 258 | TGG/CCA | MscI |
| SEQ ID NO : | 259 | T/GTACA | BsrGI |
| SEQ ID NO : | 260 | T/TAA | MseI |
| SEQ ID NO : | 261 | TTAAT/TAA | PacI |
| SEQ ID NO : | 262 | TTA/TAA | PsiI-v2 |
| SEQ ID NO : | 263 | TT/CGAA | BstBI |
| SEQ ID NO : | 264 | TTT/AAA | DraI |
| SEQ ID NO : | 265 | VC/TCGAGB | PspXI |
| SEQ ID NO : | 266 | W/CCGGW | BsaWI |
| SEQ ID NO : | 267 | YAC/GTR | BsaAI |
| SEQ ID NO : | 268 | Y/GGCCR | EaeI |

Évidemment, dans le cadre d'une synthèse chimique de la première molécule, il n'est pas nécessaire de disposer de sites de clonage (ou d'insertion) de la séquence permettant de cibler la région cible, mais de bien prendre la précaution de donner une séquence correctement orientée. Cela est bien évidemment toutefois possible.

La première molécule est en outre constituée de part et d'autre de la région A, de séquences riches en A/T, ou s'il s'agit d'ARN en A/U, afin de permettre une certaine flexibilité de la structure. Par riche en A/T, ou en A/U, on entend dans l'invention une séquence qui comprend plus de 50% de A ou T, ou U, de préférence plus de 50% de T ou de U, par rapport au nombre total de nucléotides constituant la séquence. Ces séquences de part et d'autre de la région A ont une taille en nucléotides variant de de 10 nucléotides à 60 nucléotides.

La flexibilité de ces séquences flanquantes de la région A, du fait de la présence de nombreux A, T ou U, peut avoir pour effet de permettre une recombinaison par l'intermédiaire des recombinases trop peu contrôlée, voire même lors que le complexe n'a pas encore reconnu la molécule cible.

Aussi, afin de pallier ce problème, des séquences riches en GC sont introduites dans chacune des séquences riches en A/T, notamment riches en T, ou A/U, bordant la région A. Ces régions riches en G/C sont constituée de 6 à 12 nucléotides, dont la quantité de C et/ou de G est supérieur à 50% des nucléotides contenus dans ladite séquence riche en G/C.

Pour stabiliser la structure de la première molécule, et comme décrit ci-dessus éviter une recombinaison intempestive, les régions riches en G/C sont positionnées de 15 à 52 nucléotides par rapport à l'extrémité de la région A.

Pour plus de clarté, si la région A consiste en 3 nucléotides, le nucléotide central correspondant à la position 0, la région riche en A/T ou A/U débutera à gauche en position -2, et à droite en position +2. Dès lors, à gauche, la région riche en G/C sera positionnée de la position -17 à la position -54, et du côté droit de la position +17 à la position +54.

Autre élément important, la séquence riche en G/C à droite (ou 5') de la région A est nécessairement complémentaires (selon le principe d'appariement de Watson et Crick) de la région riche en G/C de la région à droite (ou 3') de la région A. Aussi, la première molécule simple brin s'apparie-t-elle avec elle-même au niveau des régions riches en G/C, ce qui empêche toute recombinaison par les transposases, tant qu'il n'y aura pas d'interaction avec la séquence cible complémentaire de la région qui sera insérée dans la région A de la première molécule.

Enfin la première molécule comprend à son extrémité 5' une séquence correspondant à un premier site de liaison à une transposase, et à son extrémité 3' un deuxième site de liaison à ladite transposase.

Le premier site de liaison et le deuxième site de liaison sont avantageusement les mêmes, et surtout correspondent tous les deux au même brin du site de liaison double brin de ladite transposase. Cela signifie que le premier site de liaison à la transposase présent dans la région 5' de la première molécule ne peut s'apparier intégralement, et donc de manière stable avec le site de liaison à la transposase présent dans la région 3'.

Le premier site de liaison et le deuxième site de liaison sont avantageusement les mêmes, mais correspondent chacun à un brin différent du site double brin de liaison à la transposase. Aussi, par exemple, si le premier site de liaison à la transposase correspond au brin sens, le deuxième site de liaison à la transposase correspond à la séquence du brin complémentaire. Il est alors possible d'avoir deux configurations : i) soit le deuxième site de liaison qui correspond au brin complémentaire est orienté dans le sens 3'-5', dans ce cas il pourra s'apparier avec le premier site de liaison à la transposase et former le site double brin, ii) soit le deuxième site de liaison qui correspond au brin complémentaire est orienté dans le sens 5'-3', et dans ce cas ne pourra pas s'apparier avec la première séquence de liaison à la transposase, les séquences, du fait de leur orientation n'étant pas complémentaires. Dans le cas i) susmentionné, si la première molécule simple brin s'auto-apparie au niveau des premier et deuxième site de liaison, il ne sera pas possible de former le complexe susmentionné, car il n'y aura plus de région complémentaire simple brin disponible pour s'apparier avec la deuxième molécule de sorte à former deux sites de liaison double brin à la transposase.

Aussi, la première molécule, lorsqu'elle est dépourvue de séquence complémentaire de la région cible dans la partie A, ou lorsqu'elle contient une telle séquence cible mais que cette dernière n'interagit pas (ne s'apparie pas) avec ladite séquence cible, forme une structure tridimensionnelle où toute la molécule est simple brin à l'exception de la région correspondant aux régions riches en G/C qui s'apparient entre elles.

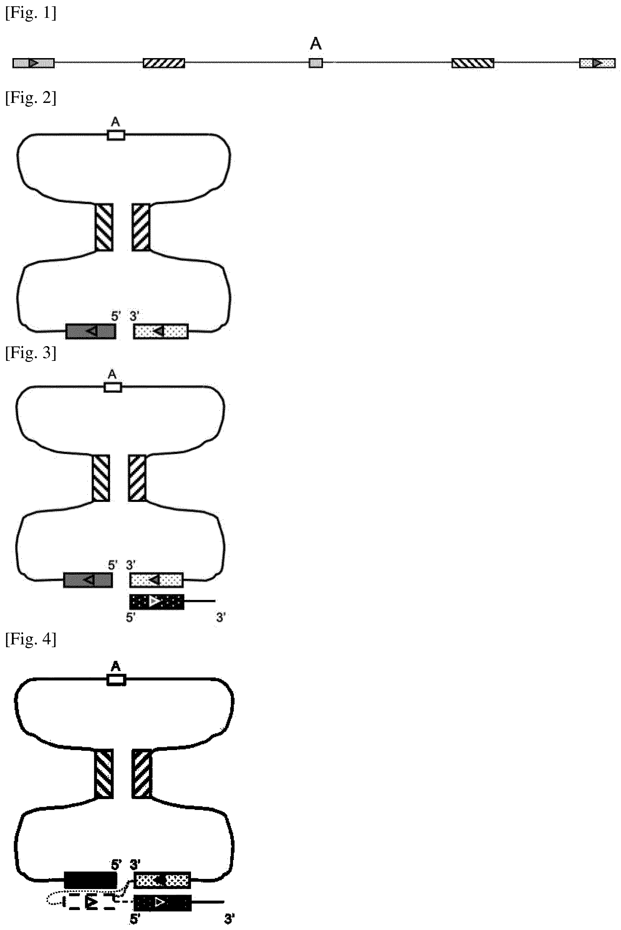

Une représentation schématique linéaire de la première molécule est représentée en [Fig.1], et une représentation schématique de sous forme appariée est représentée en [Fig.2].

### La deuxième molécule.

La deuxième molécule du complexe susmentionné est plus simple que la première. Elle comprend, dans sa partie 5', un site de liaison à la transposase qui est complémentaire du site de liaison à ladite transposase présent dans la partie 3' de la première molécule. De ce fait, lorsque le complexe sera formé, le ½ site de liaison à la transposase (simple brin) localisé en 3' de la première molécule pourrait s'apparier avec le ½ site de liaison (simple brin) à la transposase localisé en 5' de la deuxième molécule de sorte à former un site de liaison double brin à la transposase, site double brin sur lequel la transposase pourra venir se fixer.

Dans la partie 3' de la deuxième molécule se trouve une région similaire à la région A de la première molécule, cette région permettant de recevoir une séquence spécifique, qui correspond à la séquence que l'on souhaite insérer à la place de la molécule cible d'intérêt. Par la suite, il sera décrit plus en détail comment préparer une deuxième molécule permettant cette substitution.

### Le complexe

Le complexe formé de la première molécule et de la deuxième molécule est représenté schématiquement en [Fig.3].

Le complexe est tel que lorsque la première et la deuxième molécule sont appariées, par l'intermédiaire des demi-sites de liaison à la transposasse, le complexe est capable de fixer un dimère de transposase, dimère fonctionnel qui permettra la recombinaison.

Aussi, l'une ou l'autre des première ou deuxième molécule comprend en outre une séquence complémentaire du demi-site de liaison à la transposase localisé en 5' de la première molécule. Cette région complémentaire du site de liaison de la transposase localisé en 5' de la première molécule peut être localisé en 5' ou en 3' de la première molécule, ou encore en 5' de la deuxième molécule, de préférence en 5' de la séquence complémentaire du site de liaison à la transposase localisé en 3' de la première molécule.

Dans l'invention « ledit complexe étant tel que les première et deuxième molécules d'acides nucléiques simple brin sont appariées selon la complémentarité des bases définie par Watson et Crick de sorte à définir deux sites double brin de liaison de ladite transposase ». Comme la première molécule comprend au moins un demi-site de liaison à la transposase dans sa régions 5' et au moins un demi site de liaison à la transposase dans sa région 3' et que la deuxième molécule comprend elle aussi au moins un demi site de liaison à la transposase, cela signifie que lors de l'appariement entre la première et la deuxième molécule, deux sites complets seront formés car
- soit
   * la première molécule comprend dans sa région 5' une première séquence d'un premier site de liaison à la transposase et la séquence complémentaire de la première séquence du premier site de liaison à la transposase, et dans sa partie 3' une deuxième séquence d'un deuxième site de liaison à la transposase, et
   * la deuxième molécule comprend la séquence complémentaire de la deuxième séquence du deuxième site de liaison à la transposase,
- soit
   * la première molécule comprend dans sa région 5' une première séquence d'un premier site de liaison à la transposase, et dans sa partie 3' une deuxième séquence d'un deuxième site de liaison à la transposase et la séquence complémentaire de la première séquence du premier site de liaison à la transposase, et
   * la deuxième molécule comprend la séquence complémentaire de la deuxième séquence du deuxième site de liaison à la transposase,
- soit
   * la première molécule comprend dans sa région 5' une première séquence d'un premier site de liaison à la transposase, et dans sa partie 3' une deuxième séquence d'un deuxième site de liaison à la transposase, et
   * la deuxième molécule comprend la séquence complémentaire de la première séquence du premier site de liaison à la transposase, et la séquence complémentaire de la deuxième séquence du deuxième site de liaison à la transposase.

La terminologie utilisée « au moins », et le fait que la molécule « comprenne » des séquences formant des sites de reconnaissance de la transposase permettent à l'homme du métier de choisir la position des demi-séquences formant un site de liaison, de sorte que *in fine*, lorsque le complexe est formé deux sites entiers soient reconstitués.

Trois options, dont deux sont détaillées ci-après sont représentées schématiquement en [Fig.4].

De manière avantageuse, l'invention concerne le complexe susmentionné, où ladite séquence A comprend une séquence complémentaire d'un acide nucléique d'intérêt.

Comme mentionné précédemment, la région A peut contenir une séquence complémentaire d'un acide nucléique d'intérêt. Plus particulièrement, la séquence contenue dans la région A de la première molécule du complexe susmentionnée est complémentaire d'une séquence en 5' ou en 3' d'une séquence d'une molécule d'intérêt, de sorte que la complexe permet la reconnaissance spécifique de cette région de la molécule d'acide nucléique, et permettre au complexe de remplacer une région adjacente à la région complémentaire de la région complémentaire de la séquence contenue dans la région A.

En d'autres termes, l'invention concerna avantageusement le complexe susmentionné, ledit complexe comprenant :
- une première molécule d'acides nucléiques simple brin comprenant ou constituée essentiellement d'une séquence A comprenant une séquence complémentaire d'un acide nucléique d'intérêt, ladite séquence complémentaire étant liée en 5' à une première séquence riches en A/T, notamment riches en T, de 40 à 60 nucléotides de long et en 3' à une deuxième séquence riches en A/T, notamment riches en T, de 40 à 60 nucléotides de long, lesdites première et deuxième séquence riches en A/T, notamment riches en T, comprenant respectivement un premier et un deuxième domaine de 6 à 12 nucléotides riches en G/C, la séquence du premier domaine étant complémentaire de la séquence du deuxième domaine, ledit premier et deuxième domaine étant positionnés de 15 à 52 nucléotides de ladite séquence A, ladite première molécule comprenant en son extrémité 5' une première séquence orientés 5'-3' de reconnaissance d'une transposase et en son extrémité 3' au moins une deuxième séquence de reconnaissance de ladite transposase ; et
- une deuxième molécule d'acides nucléiques simple brin comprenant ou constituée essentiellement en son extrémité 5' au moins une séquence complémentaire de ladite deuxième séquence de reconnaissance de ladite transposase,
les première et deuxième molécules d'acides nucléiques simple brin étant appariées selon la complémentarité des bases définie par Watson et Crick de sorte à définir deux sites double brin de liaison de ladite transposase.

Dans un mode de réalisation avantageux, l'invention concerne un complexe susmentionné, où ladite première molécule comprend en son extrémité 5' une première séquence orientée 5'-3' de reconnaissance d'une transposase et en son extrémité 3' une deuxième séquence orientée 5'-3' de reconnaissance de ladite transposase et
où la deuxième molécule comprend son extrémité 5' une première séquence complémentaire de ladite première séquence de reconnaissance de ladite transposase suivi d'une deuxième séquence complémentaire de ladite deuxième séquence de reconnaissance de ladite transposase.

Dans ce mode avantageux du complexe de l'invention, la première molécule comprend une première séquence de reconnaissance de la transposase en position 5' et une deuxième séquence de reconnaissance de la transposase en position 3'. La deuxième molécule comprend quant à elle en position 5' une première séquence complémentaire de la première séquence reconnaissance de la transposase de la première molécule, suivie d'une deuxième séquence complémentaire de la deuxième séquence reconnaissance de la transposase de la première molécule. Aussi, chaque molécule du complexe comprend deux demi-sites de liaison à la transposase, de sorte que lors que le complexe est formé, c'est-à-dire lorsque la première molécule s'apparie avec la deuxième molécule, deux sites double brin adjacents de fixation de la transposase sont reformés, et un dimère de transposase peut alors s'y fixer.

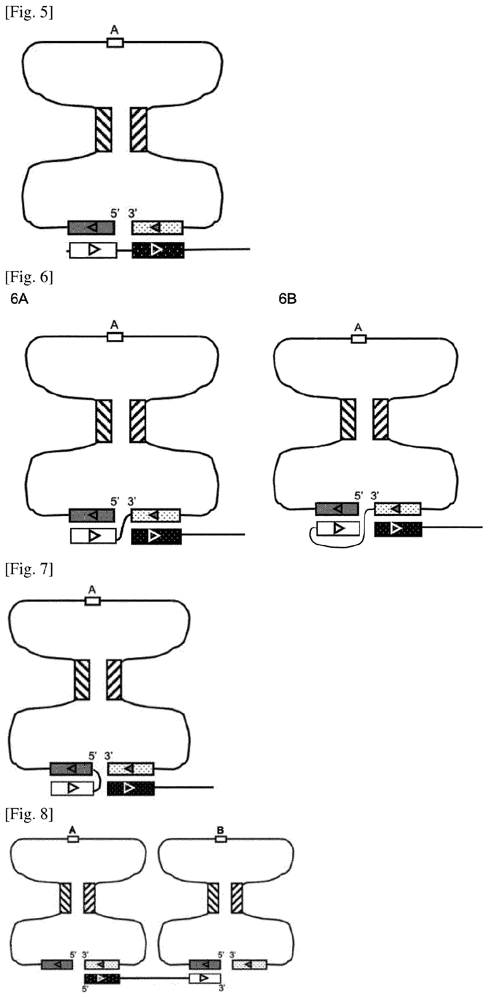

La [Fig.5] représente schématiquement ce mode de réalisation.

De manière avantageuse l'invention concerne le complexe susmentionné, où ladite première molécule comprend en son extrémité 5' une première séquence orientée 5'-3' de reconnaissance d'une transposase et en son extrémité 3' une deuxième séquence de reconnaissance de ladite transposase, suivie d'une première séquence complémentaire de ladite première séquence de reconnaissance de ladite transposase et
où la deuxième molécule comprend son extrémité 5' une séquence complémentaire de ladite deuxième séquence de reconnaissance de ladite transposase.

Dans ce mode avantageux du complexe de l'invention, la première molécule comprend une première séquence de reconnaissance de la transposase en position 5' et une deuxième séquence de reconnaissance de la transposase en position 3' cette dernière étant suivie immédiatement par une première séquence complémentaire de la séquence de reconnaissance de la transposase localisée dans en 5' de la première molécule. La deuxième molécule comprend quant à elle en position 5' une deuxième séquence complémentaire de la deuxième séquence reconnaissance de la transposase de la première molécule.

Aussi, dans ce mode de réalisation, la première molécule peut reformer un site de liaison double brin de reconnaissance de la transposase, par appariement de la première séquence de reconnaissance en 5' de la première molécule et la première séquence de reconnaissance en 3' de la molécule. La deuxième molécule quant à elle devra s'apparier avec la première molécule pour reconstituer le deuxième site de liaison double brin de la transposase à l'aide de la deuxième séquence de reconnaissance en 3' de la première molécule et la deuxième séquence complémentaire de reconnaissance de la transposase localisée en 5' de deuxième molécule.

La Figure 6B représente schématiquement ce mode de réalisation.

Il est également possible de d'envisager un autre mode de réalisation avantageux du complexe selon l'invention dans lequel la première molécule comprend en 5' une première séquence complémentaire de d'un premier site de reconnaissance de la transposase, suivi par premier site de reconnaissance de la transposase. En outre, en 3', la première molécule comprend un deuxième site de reconnaissance de la transposase. La deuxième molécule quant à elle reste inchangée par rapport au mode de réalisation précédemment décrit.

Ici, la partie 5' de la première molécule se repliera sur elle-même de sorte à reconstituer, par appariement, un site double brin de reconnaissance de la transposase à l'aide du premier site de reconnaissance et de la séquence complémentaire immédiatement adjacente. Ce mode de réalisation est présenté en [Fig.7].

Un mode réalisation similaire additionnel existe où la première molécule comprend en 5' la première séquence complémentaire du premier site de la transposase suivi immédiatement par le premier site de reconnaissance de la première transposase.

De manière avantageuse, la transposase susmentionné est une transposase de type bactérienne choisie parmi la transposase du transposon Tn5, la transposase du transposon Tn9, la transposase du transposon Tn10, Tn903, Tn602 ou encore la transposase du transposon Tc1, ou plus généralement de la superfamille des transposons mariner.

D'autres exemples de transposases qui peuvent être utilisés dans le cadre de l'invention sont : la transposase de Vibrio harveyi (transposase caractérisée par Agilent et utilisé dans le produit SureSelect QXT), la transposase MuA et un site de reconnaissance de la transposase Mu comprenant les séquences terminales R1 et R2, la transposase du transposon Tn552 de Staphylococcus aureus, la transposase du transposon Tn7, la transposase Tn/O et IS10, la transposase du transposon Tn3.

La transposase de Tn5 est la plus connue. Elle est codée par le gène Tnp du transposon Tn5. La transposase initie la transposition en formant un dimère de transposase qui se fixe sur ses séquences cibles. Dans le contexte de ce complexe, la transposase catalyse ensuite quatre réactions de transfert de phosphoryle (coupure d'ADN, formation en épingle à cheveux d'ADN, résolution en épingle à cheveux et transfert de brin dans l'ADN cible) entraînant l'intégration du transposon dans son nouveau site d'ADN : il s'agit de la tagmentation.

C'est sur le principe de cette tagmentation que l'invention repose. En utilisant les propriétés de tagmentation des transposases, il est possible d'insérer une séquence dans une autre de manière ciblée, grâce au complexe susmentionné.

Aussi dans le cadre de l'invention, lorsqu'il est fait référence à une transposase, il est fait référence à l'une des transposases susmentionnées, à savoir les transposases des transposons Tn5, Tn9, Tn10 ou Tc1/mariner (ou transposases mutées afin d'augmenter leur activité de transposition ou de tagmentation).

Dans l'invention, lorsque plusieurs transposases sont utilisées simultanément, l'une se liant au complexe formé par la première molécule et à la troisième molécule, et l'autre se liant au complexe formé par la deuxième molécule et la troisième molécule, on privilégiera les couples de transposases issus de transposons Tn5 et Tn10.

Dans un mode de réalisation avantageux, l'invention concerne un kit comprenant un vecteur permettant l'expression de la première molécule du complexe susmentionné, et un vecteur permettant l'expression de la deuxième molécule susmentionnée.

Dans le cadre de ce kit, les vecteurs sont des molécules préférentiellement circulaires, d'ADN double brin qui possèdent tous les éléments permettant leur réplication dans des cellules hôtes (procaryotes et ou eucaryotes) et qui disposent d'éléments permettant l'expression de la première ou de la deuxième molécule du complexe susmentionnés.

Dans l'hypothèse où une première molécule et une deuxième molécule doivent être sous la forme de molécules d'ADN simple brin, la séquence de chacune desdites première et deuxième molécules sont sous contrôle d'une permettant la synthèse d'ADN simple brin à partir d'ADN double brin. C'est le cas par exemple de la séquence d'origine de réplication du bactériophage f1 contenue dans les vecteurs de type phagemide. E présence d'un phase auxiliaire M13, qui porte tous les gènes nécessaires pour l'activation de la séquence f1, le vecteur va donc produire de l'ADN simple brin à partir de l'ADN de plasmide double brin.

Le kit peut donc contenir soit deux vecteurs indépendant contenant chacun la séquence de l'une ou l'autre des première et deuxième molécules formant le complexe susmentionné, soit un seul vecteur comprenant les deux séquences, mais isolées génétiquement entre elles.

Le kit susmentionné peut également contenir d'autres éléments tels qu'une transposase permettant la transposition.

De manière avantageuse, le complexe susmentionné est tel que la première et la deuxième séquence de reconnaissance de la transposase est une séquence de reconnaissance de la transposase de Tn5 ayant l'une des séquences suivantes :
- CTGtCTCTTataCAcAtcT (SEQ ID NO : 1),
- CTGACTCTTataCACAagT (SEQ ID NO : 3), et
- CTGtCTCTTgatCAgATCT (SEQ ID NO : 5).

En conséquence les séquences complémentaires correspondantes sont les suivantes :
- AgaTgTGtatAAGAGaCAG (SEQ ID NO : 2),
- ActTGTGtatAAGAGTCAG (SEQ ID NO : 4), et
- AGATcTGatcAAGAGaCAG (SEQ ID NO : 6).

D'autres séquences de reconnaissance d'une transposase sont les suivantes :
Tn5MErev,
   5'-[phos]CTGTCTCTTATACACATCT-3' (SEQ ID NO : 11)
Tn5ME-A (Illumina FC-121-1030),
   5'-TCGTCGGCAGCGTCAGATGTGTATAAGAGACAG-3'; (SEQ ID NO : 12)
et Tn5ME-B (Illumina FC-121-1031),
   5'-GTCTCGTGGGCTCGGAGATGTGTATAAGAGACAG-3' (SEQ ID NO : 13)

Encore d'autres séquences sont les suivantes
- séquence sens SEQ ID NO : i
- séquence antisens SEQ ID NO : i+1,
où i varie de 269 à 424.

Cela signifie par exemple que les couples de séquence sens et antisens respectifs suivants sont envisagés : SEQ ID NO : 269 et SEQ ID NO : 270 ; SEQ ID NO : 271 et SEQ ID NO : 272 ; SEQ ID NO : 273 et SEQ ID NO : 274 ; SEQ ID NO : 275 et SEQ ID NO : 276 ; SEQ ID NO : 277 et SEQ ID NO : 278 ; SEQ ID NO : 279 et SEQ ID NO : 280 ; SEQ ID NO : 281 et SEQ ID NO : 282 ; SEQ ID NO : 283 et SEQ ID NO : 284 ; SEQ ID NO : 285 et SEQ ID NO : 286 ; SEQ ID NO : 287 et SEQ ID NO : 288 ; SEQ ID NO : 289 et SEQ ID NO : 290 ; SEQ ID NO : 291 et SEQ ID NO : 292 ; SEQ ID NO : 293 et SEQ ID NO : 294 ; SEQ ID NO : 295 et SEQ ID NO : 296 ; SEQ ID NO : 297 et SEQ ID NO : 298 ; SEQ ID NO : 299 et SEQ ID NO : 300 ; SEQ ID NO : 301 et SEQ ID NO : 302 ; SEQ ID NO : 303 et SEQ ID NO : 304 ; SEQ ID NO : 305 et SEQ ID NO : 306 ; SEQ ID NO : 307 et SEQ ID NO : 308 ; SEQ ID NO : 309 et SEQ ID NO : 310 ; SEQ ID NO : 311 et SEQ ID NO : 312 ; SEQ ID NO : 313 et SEQ ID NO : 314 ; SEQ ID NO : 315 et SEQ ID NO : 316 ; SEQ ID NO : 317 et SEQ ID NO : 318 ; SEQ ID NO : 319 et SEQ ID NO : 320 ; SEQ ID NO : 321 et SEQ ID NO : 322 ; SEQ ID NO : 323 et SEQ ID NO : 324 ; SEQ ID NO : 325 et SEQ ID NO : 326 ; SEQ ID NO : 327 et SEQ ID NO : 328 ; SEQ ID NO : 329 et SEQ ID NO : 330 ; SEQ ID NO : 331 et SEQ ID NO : 332 ; SEQ ID NO : 333 et SEQ ID NO : 334 ; SEQ ID NO : 335 et SEQ ID NO : 336 ; SEQ ID NO : 337 et SEQ ID NO : 338 ; SEQ ID NO : 339 et SEQ ID NO : 340 ; SEQ ID NO : 341 et SEQ ID NO : 342 ; SEQ ID NO : 343 et SEQ ID NO : 344 ; SEQ ID NO : 345 et SEQ ID NO : 346 ; SEQ ID NO : 347 et SEQ ID NO : 348 ; SEQ ID NO : 349 et SEQ ID NO : 350 ; SEQ ID NO : 351 et SEQ ID NO : 352 ; SEQ ID NO : 353 et SEQ ID NO : 354 ; SEQ ID NO : 355 et SEQ ID NO : 356 ; SEQ ID NO : 357 et SEQ ID NO : 358 ; SEQ ID NO : 359 et SEQ ID NO : 360 ; SEQ ID NO : 361 et SEQ ID NO : 362 ; SEQ ID NO : 363 et SEQ ID NO : 364 ; SEQ ID NO : 365 et SEQ ID NO : 366 ; SEQ ID NO : 367 et SEQ ID NO : 368 ; SEQ ID NO : 369 et SEQ ID NO : 370 ; SEQ ID NO : 371 et SEQ ID NO : 372 ; SEQ ID NO : 373 et SEQ ID NO : 374 ; SEQ ID NO : 375 et SEQ ID NO : 376 ; SEQ ID NO : 377 et SEQ ID NO : 378 ; SEQ ID NO : 379 et SEQ ID NO : 380 ; SEQ ID NO : 381 et SEQ ID NO : 382 ; SEQ ID NO : 383 et SEQ ID NO : 384 ; SEQ ID NO : 385 et SEQ ID NO : 386 ; SEQ ID NO : 387 et SEQ ID NO : 388 ; SEQ ID NO : 389 et SEQ ID NO : 390 ; SEQ ID NO : 391 et SEQ ID NO : 392 ; SEQ ID NO : 393 et SEQ ID NO : 394 ; SEQ ID NO : 395 et SEQ ID NO : 396 ; SEQ ID NO : 397 et SEQ ID NO : 398 ; SEQ ID NO : 399 et SEQ ID NO : 400 ; SEQ ID NO : 401 et SEQ ID NO : 402 ; SEQ ID NO : 403 et SEQ ID NO : 404 ; SEQ ID NO : 405 et SEQ ID NO : 406 ; SEQ ID NO : 407 et SEQ ID NO : 408 ; SEQ ID NO : 409 et SEQ ID NO : 410 ; SEQ ID NO : 411 et SEQ ID NO : 412 ; SEQ ID NO : 413 et SEQ ID NO : 414 ; SEQ ID NO : 415 et SEQ ID NO : 416 ; SEQ ID NO : 417 et SEQ ID NO : 418 ; SEQ ID NO : 419 et SEQ ID NO : 420 ; SEQ ID NO : 421 et SEQ ID NO : 422 ; SEQ ID NO : 423 et SEQ ID NO : 424 ;

De manière avantageuse, le premier domaine riche en G/C de la première molécule correspond à la séquence suivante GGCGATCGC (SEQ ID NO : 425) de sorte que le deuxième domaine riche en G/C sera le même. En effet, du fait du repliement de la molécule sur elle-même, le deuxième domaine riche en G/C sera dans une orientation complémentaire et antiparallèle par rapport à au premier domaine riche en G/C, et l'interaction se fera au niveau de la région palindromique (souligné dans la séquence ci-dessus).

Le premier et le deuxième domaines riches en G/C peut également être la séquence suivante GCGGCGATCGGC (SEQ ID NO : 426). Les explications ci-dessus s'appliquent *mutatis mutandis.*

D'autres séquences des domaines riches en G/C, peuvent être les suivantes :
- premier domaine riche en G/C de séquence GGTCGC (SEQ ID NO : 427) et le second domaine riche en C/C de séquence GCGACC (SEQ ID NO : 428).

Ces exemples sont donnés à titre illustratif seulement et ne sauraient limiter la portée de l'invention.

Dans un mode de réalisation avantageux, les séquences riches en A/T de la première molécule dudit complexe sont constituées essentiellement, ou constituées de A ou de T.

De manière encore plus avantageuse, la séquence riche en A/T de la première molécule dudit complexe est constituée de T.

De manière encore plus avantageuse, le complexe susmentionné est tel qu'il comprend la séquence suivante correspondant à la première molécule : 
5’-TGCAGCTGCTGTCTCTTATACACATCTTTTTTTTTTTTTTTTTTTTTTTTTT
TTTGGCGATCGCTTTTTTTTTTTTTTTTTTTTXTTTTTTTTTTTTTTTTTTTTGC
GATCGCCTTTTTTTTTTTTTTTTGATACATGTTTAGATGTGTATAAGAGACA
GCTGTAAGC-3’ (SEQ ID NO : 7),
 où X représente aucun nucléotide, deux nucléotides ou au moins un site de restriction.

En encadré est représentée le premier site de liaison à la transposase et le deuxième site de liaison à la transposase est représenté souligné.

De manière encore plus avantageuse, le complexe susmentionné est tel qu'il comprend la séquence suivante correspondant à la deuxième molécule : 
5'-AGATGTGTATAAGAGACAGCAGCTGCAGACAAAGCTTACAGCTGTCTC
TTATACACATCTTTTTTTTTTTTTTTTTTTTTTTcatatgccaagtY-3' (SEQ ID NO :
8)
 où Y représente aucun nucléotide, deux nucléotides ou au moins un site de restriction.

En encadré est représentée le premier site de liaison à la transposase et le deuxième site de liaison à la transposase est représenté souligné.

De manière avantageuse, le complexe susmentionné est tel qu'il comprend la séquence suivante correspondant à la première molécule : 
5'-ATCATCCTGTCTCTTATACACATCTTTTTTTTTTTTTTTTTTGATAGTAGC
TGTCTCTTATACACATCTTTTTTTTTTTTTTTTTTTTTTGGCGATCGCTTTTT
TTTTTTTTTTTXTTTTTTTTTTTTTTTTTGCGATCGCCTTTTTTTTTTTTTTTGATAC
ATTTAGATGTGTATAAGAGACAGGATGAT-3' (SEQ ID NO : 9)
 où X représente aucun nucléotide, deux nucléotides ou au moins un site de restriction.

En encadré est représentée la séquence complémentaire du premier site de liaison transposase et le deuxième site de liaison à la transposase est représenté souligné, et en italique souligné le premier site de liaison à la transposase.

Dans ce mode de réalisation, le complexe susmentionné est tel qu'il comprend la séquence suivante correspondant à la deuxième molécule : 
5’-YacttggTTAATTAATTTTTTTTTTTTTTTTTTTTTTTTAGATGTGTATAAGAGA
CAGCTACTATC-3’ (SEQ ID NO : 10)
 où Y représente aucun nucléotide, deux nucléotides ou au moins un site de restriction.

La séquence complémentaire du deuxième site de liaison à la transposase est représenté souligné.

De manière avantageuse, l'invention concerne les complexes suivants :
- une première molécule de séquence

5’-TGCAGCTGR1TTTTTTTTTTTTTTTTTTTTTTTTTTTGGCGATCGCTTTTTT
TTTTTTTTTTTTTTTTXTTTTTTTTTTTTTTTTTTTTGCGATCGCCTTTTTTTTTTTTT
TTTTGATACATGTTTR2CTGTAAGC-3’ (SEQ ID NO : 436)

Où R1 est 5'-CTGtCTCTTataCAcAtcT (SEQ ID NO : 1),
R2 est 5'-AgaTgTGtatAAGAGaCAG (SEQ ID NO : 2),
X correspond à la séquence permettant la reconnaissance de la région cible
   et
- une deuxième molécule comprenant la séquence suivante :

5’-R2CAGCTGCAGACAAAGCTTACAGR1TTTTTTTTTTTTTTTTTTTTcatat
gccaagtY-3’ SEQ ID NO : 437)

Où R1 est 5'-CTGtCTCTTataCAcAtcT(SEQ ID NO : 1),
R2 est 5'-AgaTgTGtatAAGAGaCAG (SEQ ID NO : 2),
Y correspond à aucun nucléotide, ou à la séquence de remplacement de la région cible.

Cela signifie que le complexe est constitué des molécules de séquence suivante 
5’-TGCAGCTGCTGtCTCTTataCAcAtcTTTTTTTTTTTTTTTTTTTTTTTTTT
GGCGATCGCTTTTTTTTTTTTTTTTTTTTXTTTTTTTTTTTTTTTTTTTTGCGAT
CGCCTTTTTTTTTTTTTTTTTGATACATGTTTAgaTgTGtatAAGAGaCAG
CTGTAAGC-3’ (SEQ ID NO : 438)
 Et

5'- AgaTgTGtatAAGAGaCAGCAGCTGCAGACAAAGCTTACAGCTGtCTCTTa-
taCAcAtcTTTTTTTTTTTTTTTTTTTTTcatatgccaagtY-3' (SEQ ID NO : 439)

Ou X et Y sont tels que définis ci-dessus.

De manière avantageuse, l'invention concerne les complexes suivants :
- une première molécule de séquence

5’-ATCATCR1TTTTTTTTTTTTTTTTGATAGTAGR1TTTTTTTTTTTTTTTTTT
TTTTTTGGCGATCGCTTTTTTTTTTTTTTTTTXTTTTTTTTTTTTTTTTTGCGATCG
CCTTTTTTTTTTTTTTGATACATTTR2GATGAT-3’ (SEQ ID NO : 641)

Où R1 est 5'-CTGtCTCTTataCAcAtcT (SEQ ID NO : 1),
R2 est 5'-AgaTgTGtatAAGAGaCAG (SEQ ID NO : 2),
X correspond à la séquence permettant la reconnaissance de la région cible
   et
   - une deuxième molécule comprenant la séquence suivante :
      5'-**Y**acttggTTAATTAATTTTTTTTTTTTTTTTTTTTTTR2CTACTATC-3' (SEQ ID NO : 642)

Où R1 est 5'-CTGtCTCTTataCAcAtcT(SEQ ID NO : 1),
R2 est 5'-AgaTgTGtatAAGAGaCAG (SEQ ID NO : 2),
Y correspond à aucun nucléotide, ou à la séquence de remplacement de la région cible.

De manière avantageuse, l'invention concerne les complexes suivants :
- une première molécule de séquence

5’-TGCAGCTGR2TTTTTTTTTTTTTTTTTTTTTTTTTTTTTTTTTGGCGATCGCTTTTTT
TTTTTTTTTTTTTTTTXTTTTTTTTTTTTTTTTTTTTGCGATCGCCTTTTTTTTTTTTTTTTT
TTTTGATACATGTTTR2CTGTAAGC-3’ (SEQ ID NO : 643)

Où R1 est 5'-CTGtCTCTTataCAcAtcT (SEQ ID NO : 1),
R2 est 5'-AgaTgTGtatAAGAGaCAG (SEQ ID NO : 2),
X correspond à la séquence permettant la reconnaissance de la région cible
   et
   - une deuxième molécule comprenant la séquence suivante :

5’-R1CAGCTGCAGACAAAGCTTACAGR1TTTTTTTTTTTTTTTTTTTTTTTTTTTTTTcatat
gccaagtY-3’ SEQ ID NO : 644)

Où R1 est 5'-CTGtCTCTTataCAcAtcT(SEQ ID NO : 1),
R2 est 5'-AgaTgTGtatAAGAGaCAG (SEQ ID NO : 2),
Y correspond à aucun nucléotide, ou à la séquence de remplacement de la région cible.

De manière avantageuse, l'invention concerne les complexes suivants :
- une première molécule de séquence

5’-ATCATCR2TTTTTTTTTTTTTTTTTGATAGTAGR2TTTTTTTTTTTTTTTTT
TTTTTTGGCGATCGCTTTTTTTTTTTTTTTTTXTTTTTTTTTTTTTTTTTGCGATCG
CCTTTTTTTTTTTTTTGATACATTTR1GATGAT-3’ (SEQ ID NO : 645)

Où R1 est 5'-CTGtCTCTTataCAcAtcT (SEQ ID NO : 1),
R2 est 5'-AgaTgTGtatAAGAGaCAG (SEQ ID NO : 2),
X correspond à la séquence permettant la reconnaissance de la région cible
   et
   - une deuxième molécule comprenant la séquence suivante :
      5'-**Y**acttggTTAATTAATTTTTTTTTTTTTTTTTTTTTTR1CTACTATC-3' (SEQ ID NO : 646)

Où R1 est 5'-CTGtCTCTTataCAcAtcT (SEQ ID NO : 1),
R2 est 5'-AgaTgTGtatAAGAGaCAG (SEQ ID NO : 2),
Y correspond à aucun nucléotide, ou à la séquence de remplacement de la région cible.

De manière avantageuse, le complexe susmentionné est constitué des couples de séquences suivants :

**[Tableaux2]**

| Molécule 1 | Molécule 2 | R1 | R2 |
|---|---|---|---|
| 5'-ATCATCR1TTTTTTT TTTTTTTTTGATAGTA GR1TTTTTTTTTTTTTT TTTTTTTTGGCGATCGC TTTTTTTTTTTTTTTTXT TTTTTTTTTTTTTTTTGC GATCGCCTTTTTTTTT TTTGATACATTTR2GAT GAT-3' (SEQ ID NO : 641) | 5'-YacttggTTAATT AATTTTTTTTTTT TTTTTTTTTTTTR2 CTACTATC-3' (SEQ ID NO : 642) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5’-ATCATCR1TTTTTTT TTTTTTTTTGATAGTA GR1TTTTTTTTTTTTTTT TTTTTTTTGGCGATCGC TTTTTTTTTTTTTTTTXT TTTTTTTTTTTTTTTTGC GATCGCCTTTTTTTTT TTTGATACATTTR2GAT GAT-3’ (SEQ ID NO : 641) | 5'-YacttggTTAATT AATTTTTTTTTTT TTTTTTTTTTTTR2 CTACTATC-3' (SEQ ID NO : 642) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5’-TGCAGCTGR2TTTTT TTTTTTTTTTTTTTTTTT TTTTGGCGATCGCTTTT TTTTTTTTTTTTTTTTXT TTTTTTTTTTTTTTTTTT TGCGATCGCCTTTTTTT TTTTTTTTTGATACATG TTTR2CTGTAAGC-3’ (SEQ ID NO : 643) | 5'-R1CAGCTGCA GACAAAGCTTAC AGR1TTTTTTTTT TTTTTTTTTTTTTc atatgccaagtY-3' (SEQ ID NO : 644) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-TGCAGCTGR2TTTTT TTTTTTTTTTTTTTTTTTTT TTTTGGCGATCGCTTTT TTTTTTTTTTTTTTTTTTXT TTTTTTTTTTTTTTTTTTTT | 5'-**R1**CAGCTGCA GACAAAGCTTAC AGR1TTTTTTTTT TTTTTTTTTTTTTc atatgccaagt**Y**-3' | SEQ ID NO : n n étant un nombre impair variant de 1 à 6 et de 269 à 424 | SEQ ID NO : n+1 n étant le même nombre impair que R1 variant de 1 à 6 et de 269 à |
| TGCGATCGCCTTTTTTT TTTTTTTTTGATACATG TTTR2CTGTAAGC-3' (SEQ ID NO : 643) | (**SEQ ID NO : 644**) | | 424 |
| 5'-ATCATCR2TTTTTTT TTTTTTTTTGATAGTA GR2TTTTTTTTTTTTTT TTTTTTTTGGCGATCGC TTTTTTTTTTTTTTTTXT TTTTTTTTTTTTTTTGC GATCGCCTTTTTTTTT TTTGATACATTTR1GAT GAT-3' (SEQ ID NO : 645) | 5'-YacttggTTAATT AATTTTTTTTTTT TTTTTTTTTTTR1 CTACTATC-3' (SEQ ID NO : 646) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATCR2TTTTTTT TTTTTTTTTGATAGTA GR2TTTTTTTTTTTTTTT TTTTTTTTGGCGATCGC TTTTTTTTTTTTTTTTXT TTTTTTTTTTTTTTTTGC GATCGCCTTTTTTTTTT TTTGATACATTTR1GAT GAT-3' (SEQ ID NO : 645) | 5'-YacttggTTAATT AATTTTTTTTTTT TTTTTTTTTTTTR1 CTACTATC-3' (SEQ ID NO : 646) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 5' - modèle 1 | | | |
|---|---|---|---|
| 5'-TGCAGCTG**R1**TTTTT TTTTTTTTTTTTTTTTTTT TTTTGGTCGCTTTTTTT TTTTTTTTTTTTTT**X**TTTT TTTTTTTTTTTTTTTTTGC GACCTTTTTTTTTTTTT TTTGATACATGTTT**R2**C TGTAAGC-3' (**SEQ ID NO : 440**) | 5’-R2CAGCTGCA GACAAAGCTTAC AGR1TTTTTTTTT TTTTTTTTTTTTTT Yccaagt-3’ (SEQ ID NO : 441) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-TGCAGCTGR1TTTTT TTTTTTTTTTTTTTTTTTT | 5'-R2CAGCTGCA GACAAAGCTTAC | SEQ ID NO : n n étant un | SEQ ID NO : n+1 n étant le même |
| TTTTGGTCGCTTTTTTT TTTTTTTTTTTTTXTTTT TTTTTTTTTTTTTTTTTGC GACCTTTTTTTTTTTTT TTTGATACATGTTTR2C TGTAAGC-3’ (SEQ ID NO : 440) | AGR1TTTTTTTTTT TTTTTTTTTTTTTT Yccaagt-3’ (SEQ ID NO : 441) | nombre impair variant de 1 à 6 et de 269 à 424 | nombre impair que R1 variant de 1 à 6 et de 269 à 424 |
| 5'-TGCAGCTGR1TTTTT TTTTTTTTTTTTTTTTTT TTTTGGTCGCTTTTTTT TTTTTTTTTTTTTXTTTT TTTTTTTTTTTTTTTTGC GACCTTTTTTTTTTTTT TTTGATACATGTTTR2C TGTAAGC-3' (SEQ ID NO : 440) | 5'-**R2**CAGCTGCA GACAAAGCTTAC AG**R1**TTTTTTTTT TTTTTTTTTTTTTc caagt**Y**-3' (**SEQ ID NO : 442**) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5’-TGCAGCTGR1TTTTT TTTTTTTTTTTTTTTTTTT TTTTGGTCGCTTTTTTT TTTTTTTTTTTTTTXTTTT TTTTTTTTTTTTTTTTTGC GACCTTTTTTTTTTTTT TTTGATACATGTTTR2C TGTAAGC-3’ (SEQ ID NO : 440) | 5'-R2CAGCTGCA GACAAAGCTTAC AGR1TTTTTTTTT TTTTTTTTTTTTTc caagtY-3' (SEQ ID NO : 442) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 5' - modèle 2 | | | |
|---|---|---|---|
| 5'-TGCAGCTGR1TTTTT TTTTTTTTTTTTTTTTTT TTTTGCGGCGATCGGC TTTTTTTTTTTTTTTTTT TTXTTTTTTTTTTTTTT TTTTTGCCGATCGCCG CTTTTTTTTTTTTTTTTG ATACATGTTTR2CTGTA AGC-3' (SEQ ID NO : 443) | 5'-R2CAGCTGCA GACAAAGCTTAC AGR1TTTTTTTTT TTTTTTTTTTTTT Yccaagt-3' (SEQ ID NO : 441) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-TGCAGCTGR1TTTTT TTTTTTTTTTTTTTTT TTTTGCGGCGATCGGC TTTTTTTTTTTTTTTT TTXTTTTTTTTTTTTT TTTTTGCCGATCGCCG CTTTTTTTTTTTTTTG ATACATGTTTR2CTGTA AGC-3' (SEQ ID NO : 443) | 5'-**R2**CAGCTGCA GACAAAGCTTAC AG**R1**TTTTTTTTT TTTTTTTTTTTTTT **Y**ccaagt-3' (**SEQ ID NO : 441**) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-TGCAGCTGR1TTTTT TTTTTTTTTTTTTTTTT TTTTGCGGCGATCGGC TTTTTTTTTTTTTTTTT TTXTTTTTTTTTTTTTT TTTTTGCCGATCGCCG CTTTTTTTTTTTTTTTG ATACATGTTTR2CTGTA AGC-3' (SEQ ID NO : 443) | 5'-R2CAGCTGCA GACAAAGCTTAC AGR1TTTTTTTTT TTTTTTTTTTTTTc caagtY-3' (SEQ ID NO : 442) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-TGCAGCTGR1TTTTT TTTTTTTTTTTTTTTTTT TTTTGCGGCGATCGGC TTTTTTTTTTTTTTTTTT TTXTTTTTTTTTTTTTT TTTTTGCCGATCGCCG CTTTTTTTTTTTTTTTTG ATACATGTTTR2CTGTA AGC-3' (SEQ ID NO : 443) | 5'-**R2**CAGCTGCA GACAAAGCTTAC AG**R1**TTTTTTTTT TTTTTTTTTTTTTc caagt**Y**-3' (**SEQ ID NO : 442**) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 3' - modèle 1 | | | |
|---|---|---|---|
| 5'-GTGCCCAGR1TTTCT CGATCATTTTTTTTTT TTTTTGGTCGCTTTTTT TTTTTTTTTTTTTTTXTTT TTTTTTTTTTTTTTTTTTG CGACCTTTTTTTTTTT | 5'-YtacaagTCCGG ATAATAATTTTT TTTTTTTTTTTT TTTTR2CTGGGC ACGCGTATAAGC AGR1-3' | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTTTTTTTTTTR2C TGCTTAT-3' (SEQ ID NO : 444) | (SEQ ID NO : 445) | | |
| 5’-GTGCCCAGR1TTTCT CGATCATTTTTTTTTT TTTTTGGTCGCTTTTTT TTTTTTTTTTTTTTXTTT TTTTTTTTTTTTTTTTTG CGACCTTTTTTTTTTT TTTTTTTTTTTTTTTR2C TGCTTAT-3' (SEQ ID NO : 444) | 5'-YtacaagTCCGG ATAATAATTTTT TTTTTTTTTTTTT TTTTR2CTGGGC ACGCGTATAAGC AGR1-3' (SEQ ID NO : 445) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GTGCCCAGR1TTTCT CGATCATTTTTTTTTT TTTTTGGTCGCTTTTTT TTTTTTTTTTTTTTXTTT TTTTTTTTTTTTTTTTTG CGACCTTTTTTTTTTT TTTTTTTTTTTTTTTR2C TGCTTAT-3' (SEQ ID NO : 444) | 5'-tacaagYTAATA ATTTTTTTTTTT TTTTTTTTTTR2C TGGGCACGCGTA TAAGCAGR1-3' (SEQ ID NO : 446) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5’-GTGCCCAGR1TTTCT CGATCATTTTTTTTTT TTTTTGGTCGCTTTTTT TTTTTTTTTTTTTTXTTT TTTTTTTTTTTTTTTTTG CGACCTTTTTTTTTTT TTTTTTTTTTTTTTTR2C TGCTTAT-3' (SEQ ID NO : 444) | 5'-tacaagYTAATA ATTTTTTTTTTT TTTTTTTTTTR2C TGGGCACGCGTA TAAGCAGR1-3' (SEQ ID NO : 446) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5’-GTGCCCAGR1TTTCT CGATCATTTTTTTTTTTT TTTTTGGTCGCTTTTTTT TTTTTTTTTTTTTTTXTTT TTTTTTTTTTTTTTTTTTG CGACCTTTTTTTTTTTTT TTTTTTTTTTTTTTTTR2C | 5'-YtacaagTAATA ATTTTTTTTTTT TTTTTTTTTTR2C TGGGCACGCGTA TAAGCAGR1-3' (SEQ ID NO : 447) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TGCTTAT-3' (SEQ ID NO : 444) | | | |
| 5’-GTGCCCAGR1TTTCT CGATCATTTTTTTTTT TTTTTGGTCGCTTTTTT TTTTTTTTTTTTTTXTTT TTTTTTTTTTTTTTTTTG CGACCTTTTTTTTTTT TTTTTTTTTTTTTTTR2C TGCTTAT-3' (SEQ ID NO : 444) | 5’-YtacaagTAATA ATTTTTTTTTTT TTTTTTTTTTR2C TGGGCACGCGTA TAAGCAGR1-3' (SEQ ID NO : 447) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 3' - modèle 2 | | | |
|---|---|---|---|
| 5'-GTGCCCAGR1TTTCT CGATCATTTTTTTTTT TTTTTGGCGATCGCTTT TTTTTTTTTTTTTTTTX TTTTTTTTTTTTTTTTT TTGCGATCGCCTTTTT TTTTTTTTTTTTTTTTT TTTR2CTGCTTAT-3' (SEQ ID NO : 448) | 5'-YtacaagTCCGG ATAATAATTTTT TTTTTTTTTTTTT TTTTR2CTGGGC ACGCGTATAAGC AGR1-3' (SEQ ID NO : 445) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5’-GTGCCCAGR1TTTCT CGATCATTTTTTTTTT TTTTTGGCGATCGCTTT TTTTTTTTTTTTTTTTX TTTTTTTTTTTTTTTTT TTGCGATCGCCTTTTT TTTTTTTTTTTTTTTTT TTTR2CTGCTTAT-3' (SEQ ID NO : 448) | 5'-YtacaagTCCGG ATAATAATTTTT TTTTTTTTTTTTT TTTTR2CTGGGC ACGCGTATAAGC AGR1-3' (SEQ ID NO : 445) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GTGCCCAGR1TTTCT CGATCATTTTTTTTTT TTTTTGGCGATCGCTTT TTTTTTTTTTTTTTTTX TTTTTTTTTTTTTTTTT TTGCGATCGCCTTTTTT | 5’-tacaagYTAATA ATTTTTTTTTTT TTTTTTTTTTR2C TGGGCACGCGTA TAAGCAGR1-3' (SEQ ID NO : 446) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTTTTTTTTTTTTTTT TTTR2CTGCTTAT-3' (SEQ ID NO : 448) | | | |
| 5’-GTGCCCAGR1TTTCT CGATCATTTTTTTTTT TTTTTGGCGATCGCTTT TTTTTTTTTTTTTTTTX TTTTTTTTTTTTTTTTT TTGCGATCGCCTTTTTT TTTTTTTTTTTTTTTTT TTTR2CTGCTTAT-3' (SEQ ID NO : 448) | 5'-tacaagYTAATA ATTTTTTTTTTT TTTTTTTTTR2C TGGGCACGCGTA TAAGCAGR1-3' (SEQ ID NO : 446) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5’-GTGCCCAGR1TTTCT CGATCATTTTTTTTTT TTTTTGGCGATCGCTTT TTTTTTTTTTTTTTTTX TTTTTTTTTTTTTTTTT TTGCGATCGCCTTTTT TTTTTTTTTTTTTTTTT TTTR2CTGCTTAT-3' (SEQ ID NO : 448) | 5'-YtacaagTAATA ATTTTTTTTTTT TTTTTTTTTTR2C TGGGCACGCGTA TAAGCAGR1-3' (SEQ ID NO : 447) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5’-GTGCCCAGR1TTTCT CGATCATTTTTTTTTT TTTTTGGCGATCGCTTT TTTTTTTTTTTTTTTTX TTTTTTTTTTTTTTTTT TTGCGATCGCCTTTTT TTTTTTTTTTTTTTTTT TTTR2CTGCTTAT-3' (SEQ ID NO : 448) | 5'-YtacaagTAATA ATTTTTTTTTTT TTTTTTTTTTR2C TGGGCACGCGTA TAAGCAGR1-3' (SEQ ID NO : 447) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 3' - modèle 3 | | | |
|---|---|---|---|
| 5'-GTGCCCAGR1TTTCT CGATCATTTTTTTTTT TTTTTGCGGCGATCGG CTTTTTTTTTTTTTTTT TTTXTTTTTTTTTTTTT | 5'-YtacaagTCCGG ATAATAATTTTT TTTTTTTTTTTTT TTTTR2CTGGGC ACGCGTATAAGC | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTGCCGATCGCCG CTTTTTTTTTTTTTTT TTTTTTTTTTR2CTGCTT AT-3' (SEQ ID NO : 449) | AGR1-3' (SEQ ID NO : 445) | | |
| 5’-GTGCCCAGR1TTTCT CGATCATTTTTTTTTT TTTTTGCGGCGATCGG CTTTTTTTTTTTTTTT TTTXTTTTTTTTTTTT TTTTTTGCCGATCGCCG CTTTTTTTTTTTTTTT TTTTTTTTTTR2CTGCTT AT-3' (SEQ ID NO : 449) | 5'-YtacaagTCCGG ATAATAATTTTT TTTTTTTTTTTTT TTTTR2CTGGGC ACGCGTATAAGC AGR1-3' (SEQ ID NO : 445) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5’-GTGCCCAGR1TTTCT CGATCATTTTTTTTTT TTTTTGCGGCGATCGG CTTTTTTTTTTTTTTT TTTXTTTTTTTTTTTTT TTTTTTGCCGATCGCCG CTTTTTTTTTTTTTTT TTTTTTTTTTR2CTGCTT AT-3' (SEQ ID NO : 449) | 5’-tacaagYTAATA ATTTTTTTTTTT TTTTTTTTTTR2C TGGGCACGCGTA TAAGCAGR1-3' (SEQ ID NO : 446) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GTGCCCAGR1TTTCT CGATCATTTTTTTTTT TTTTTGCGGCGATCGG CTTTTTTTTTTTTTTT TTTXTTTTTTTTTTTT TTTTTTGCCGATCGCCG CTTTTTTTTTTTTTTT TTTTTTTTTR2CTGCTT AT-3' (SEQ ID NO : 449) | 5'-tacaagYTAATA ATTTTTTTTTTT TTTTTTTTTTR2C TGGGCACGCGTA TAAGCAGR1-3' (SEQ ID NO : 446) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GTGCCCAGR1TTTCT CGATCATTTTTTTTTTTT | 5'-YtacaagTAATA ATTTTTTTTTTTT | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTGCGGCGATCGG CTTTTTTTTTTTTTTT TTTXTTTTTTTTTTTT TTTTTTGCCGATCGCCG CTTTTTTTTTTTTTTT TTTTTTTTTTR2CTGCTT AT-3' (SEQ ID NO : 449) | TTTTTTTTTTR2C TGGGCACGCGTA TAAGCAGR1-3' (SEQ ID NO : 447) | | |
| 5'-GTGCCCAGR1TTTCT CGATCATTTTTTTTTT TTTTTGCGGCGATCGG CTTTTTTTTTTTTTTT TTTXTTTTTTTTTTTT TTTTTTGCCGATCGCCG CTTTTTTTTTTTTTTT TTTTTTTTTTR2CTGCTT AT-3' (SEQ ID NO : 449) | 5'-YtacaagTAATA ATTTTTTTTTTT TTTTTTTTTTR2C TGGGCACGCGTA TAAGCAGR1-3' (SEQ ID NO : 447) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 5' - modèle 1 | | | |
| 5'-ATCATCR1TTTTTTT TTTTTTTTTGATAGTA GR1TTTTTTTTTTTTTT TTTTTTTGGTCGCTTTT TTTTTTTTTTTTXTTTTT TTTTTTTTTTTGCGACC TTTTTTTTTTTTTGATA CATTTR2GATGAT-3' (SEQ ID NO : 450) | 5'-YacttggTTAATT AATTTTTTTTTTTT TTTTTTTTTTTTR2 CTACTATC-3' (SEQ ID NO : 451) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATCR1TTTTTTT TTTTTTTTTGATAGTA GR1TTTTTTTTTTTTTT TTTTTTTGGTCGCTTTT TTTTTTTTTTTTXTTTTT TTTTTTTTTTTGCGACC TTTTTTTTTTTTTGATA CATTTR2GATGAT-3' | 5'-YacttggTTAATT AATTTTTTTTTTT TTTTTTTTTTTTR2 CTACTATC-3' (SEQ ID NO : 451) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| (SEQ ID NO : 450) | | | |
| 5’-ATCATCR1TTTTTTT TTTTTTTTTTGATAGTA GR1TTTTTTTTTTTTTTT TTTTTTTGGTCGCTTTT TTTTTTTTTTTTTXTTTTT TTTTTTTTTTTGCGACC TTTTTTTTTTTTTGATA CATTTR2GATGAT-3’ (SEQ ID NO : 450) | 5’-acttggYTTAATT AATTTTTTTTTTT TTTTTTTTTTTTR2 CTACTATC-3' (SEQ ID NO : 452) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATCR1TTTTTTT TTTTTTTTTGATAGTA GR1TTTTTTTTTTTTTT TTTTTTTGGTCGCTTTT TTTTTTTTTTTTXTTTTT TTTTTTTTTTTGCGACC TTTTTTTTTTTTTGATA CATTTR2GATGAT-3' (SEQ ID NO : 450) | 5’-acttggYTTAATT AATTTTTTTTTT TTTTTTTTTTTTR2 CTACTATC-3' (SEQ ID NO : 452) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-ATCATCR1TTTTTTT TTTTTTTTTGATAGTA GR1TTTTTTTTTTTTTT TTTTTTTGGTCGCTTTT TTTTTTTTTTTTTXTTTTT TTTTTTTTTTTGCGACC TTTTTTTTTTTTTTGATA CATTTR2GATGAT-3' (SEQ ID NO : 450) | 5'-YacttggcatatgTT AATTAATTTTTTT TTTTTTTTTTTTT TTR2CTACTATC- 3' (SEQ ID NO : 453) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATC**R1**TTTTTTT TTTTTTTTTGATAGTA G**R1**TTTTTTTTTTTTTT TTTTTTTGGTCGCTTTT TTTTTTTTTTTTT**X**TTTTT TTTTTTTTTTTGCGACC TTTTTTTTTTTTTTGATA CATTT**R2**GATGAT-3' (**SEQ ID NO : 450**) | 5’-YacttggcatatgTT AATTAATTTTTTT TTTTTTTTTTTTT TTR2CTACTATC- 3' (SEQ ID NO : 453) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 5' - modèle 2 | | | |
|---|---|---|---|
| 5'-ATCATCR1TTTTTTT TTTTTTTTTGATAGTA GR1TTTTTTTTTTTTTT TTTTTTTGCGGCGATCG GCTTTTTTTTTTTTTTT XTTTTTTTTTTTTTTTG CCGATCGCCGCTTTTTT TTTTTTTGATACATTTR 2GATGAT-3' (SEQ ID NO : 454) | 5'-YacttggTTAATT AATTTTTTTTTTTT TTTTTTTTTTTTR2 CTACTATC-3' (SEQ ID NO : 451) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATCR1TTTTTTT TTTTTTTTTTGATAGTA GR1TTTTTTTTTTTTTT TTTTTTTGCGGCGATCG GCTTTTTTTTTTTTTTT XTTTTTTTTTTTTTTTG CCGATCGCCGCTTTTTT TTTTTTTGATACATTTR 2GATGAT-3' (SEQ ID NO : 454) | 5’-YacttggTTAATT AATTTTTTTTTTT TTTTTTTTTTTTR2 CTACTATC-3' (SEQ ID NO : 451) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-ATCATCR1TTTTTTT TTTTTTTTTGATAGTA GR1TTTTTTTTTTTTTT TTTTTTTGCGGCGATCG GCTTTTTTTTTTTTTTT XTTTTTTTTTTTTTTTTG CCGATCGCCGCTTTTTT TTTTTTTGATACATTTR 2GATGAT-3' (SEQ ID NO : 454) | 5’-acttggYTTAATT AATTTTTTTTTTT TTTTTTTTTTTTR2 CTACTATC-3' (SEQ ID NO : 452) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATCR1TTTTTTT TTTTTTTTTGATAGTA GR1TTTTTTTTTTTTTT TTTTTTTGCGGCGATCG GCTTTTTTTTTTTTTTT | 5’-acttggYTTAATT AATTTTTTTTTTTT TTTTTTTTTTTTR2 CTACTATC-3' (SEQ ID NO : 452) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| XTTTTTTTTTTTTTTTG CCGATCGCCGCTTTTTT TTTTTTTGATACATTTR 2GATGAT-3' (SEQ ID NO : 454) | | | |
| 5'-ATCATCR1TTTTTTT TTTTTTTTTGATAGTA GR1TTTTTTTTTTTTTT TTTTTTGCGGCGATCG GCTTTTTTTTTTTTTTT XTTTTTTTTTTTTTTTG CCGATCGCCGCTTTTTT TTTTTTTGATACATTTR 2GATGAT-3' (SEQ ID NO : 454) | 5’-Yacttggcatatg TT AATTAATTTTTTT TTTTTTTTTTTTTT TTR2CTACTATC- 3' (SEQ ID NO : 453) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-ATCATCR1TTTTTTT TTTTTTTTTGATAGTA GR1TTTTTTTTTTTTTT TTTTTTGCGGCGATCG GCTTTTTTTTTTTTTT XTTTTTTTTTTTTTTTG CCGATCGCCGCTTTTTT TTTTTTTGATACATTTR 2GATGAT-3' (SEQ ID NO : 454) | 5'-YacttggcatatgTT AATTAATTTTTTT TTTTTTTTTTTTT TTR2CTACTATC- 3' (SEQ ID NO : 453) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 5' - modèle 3 | | | |
| 5'-GATAGTAGR1TTTTT TTTTTTTTTTTTTTTTTG GCGATCGCTTTTTTTT TTTTTTTXTTTTTTTTT TTTTTTGCGATCGCCTT TTTTTTTTTTTGATACA TTTR2GATGATTTTTTT TTTTTTTTTTTATCATC R1-3' (SEQ ID NO : 455) | 5’-YacttggTTAATT AATTTTTTTTTTT TTTTTTTTTTTTR2 CTACTATC-3' (SEQ ID NO : 451) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GATAGTAGR1TTTTT TTTTTTTTTTTTTTTTTG GCGATCGCTTTTTTTT TTTTTTTXTTTTTTTTT TTTTTGCGATCGCCTT TTTTTTTTTTTGATACA TTTR2GATGATTTTTT TTTTTTTTTTTATCATC R1-3' (SEQ ID NO : 455) | 5'-YacttggTTAATT AATTTTTTTTTTT TTTTTTTTTTTTR2 CTACTATC-3' (SEQ ID NO : 451) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GATAGTAGR1TTTTT TTTTTTTTTTTTTTTTTTG GCGATCGCTTTTTTTTT TTTTTTTXTTTTTTTTTT TTTTTTGCGATCGCCTT TTTTTTTTTTTTGATACA TTTR2GATGATTTTTTT TTTTTTTTTTTTATCATC R1-3' (SEQ ID NO : 455) | 5'-acttggYTTAATT AATTTTTTTTTTT TTTTTTTTTTTTR2 CTACTATC-3' (SEQ ID NO : 452) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GATAGTAGR1TTTTT TTTTTTTTTTTTTTTTTG GCGATCGCTTTTTTTTT TTTTTTTXTTTTTTTTT TTTTTTGCGATCGCCTT TTTTTTTTTTTGATACA TTTR2GATGATTTTTTT TTTTTTTTTTTATCATC R1-3' (SEQ ID NO : 455) | 5’-acttggYTTAATT AATTTTTTTTTTT TTTTTTTTTTTTR2 CTACTATC-3' (SEQ ID NO : 452) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GATAGTAGR1TTTTT TTTTTTTTTTTTTTTTTG GCGATCGCTTTTTTTTT TTTTTTTXTTTTTTTTTT TTTTTTGCGATCGCCTT TTTTTTTTTTTGATACA TTTR2GATGATTTTTTT | 5'-YacttggcatatgTT AATTAATTTTTTT TTTTTTTTTTTTTT TTR2CTACTATC- 3' (SEQ ID NO : 453) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTTTTTTATCATC **R1**-3' (**SEQ ID NO : 455**) | | | |
| 5'-GATAGTAGR1TTTTT TTTTTTTTTTTTTTTTTTG GCGATCGCTTTTTTTTT TTTTTTTXTTTTTTTTTT TTTTTTGCGATCGCCTT TTTTTTTTTTTTGATACA TTTR2GATGATTTTTTT TTTTTTTTTTTATCATC R1-3' (SEQ ID NO : 455) | 5'-Yacttggcatatg TT AATTAATTTTTTT TTTTTTTTTTTTT TTR2CTACTATC- 3' (SEQ ID NO : 453) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 5' - modèle 4 | | | |
| 5’-GATAGTAGR1TTTTT TTTTTTTTTTTTTTTTTG GTCGCTTTTTTTTTTTT TTTTXTTTTTTTTTTTTT TTTGCGACCTTTTTTTT TTTTTGATACATTTR2G ATGATTTTTTTTTTTTT TTTTTATCATCR1-3’ (SEQ ID NO : 456) | 5'-YacttggTTAATT AATTTTTTTTTTT TTTTTTTTTTTTR2 CTACTATC-3' (SEQ ID NO : 451) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GATAGTAGR1TTTTT TTTTTTTTTTTTTTTTTG GTCGCTTTTTTTTTTTT TTTTXTTTTTTTTTTTT TTTGCGACCTTTTTTTT TTTTTGATACATTTR2G ATGATTTTTTTTTTTTT TTTTTATCATCR1-3' (SEQ ID NO : 456) | 5'-YacttggTTAATT AATTTTTTTTTTT TTTTTTTTTTTTR2 CTACTATC-3' (SEQ ID NO : 451) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5’-GATAGTAGR1TTTTT TTTTTTTTTTTTTTTTTTTG GTCGCTTTTTTTTTTTTTT | 5'-acttggYTTAATT AATTTTTTTTTTTT TTTTTTTTTTTTTR2 | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTXTTTTTTTTTTTTT TTTGCGACCTTTTTTTT TTTTTGATACATTTR2G ATGATTTTTTTTTTTTT TTTTTATCATCR1-3' (SEQ ID NO : 456) | CTACTATC-3' (SEQ ID NO : 452) | | |
| 5'-GATAGTAGR1TTTTT TTTTTTTTTTTTTTTTTTG GTCGCTTTTTTTTTTTT TTTTXTTTTTTTTTTTTT TTTGCGACCTTTTTTTT TTTTTGATACATTTR2G ATGATTTTTTTTTTTTT TTTTTATCATCR1-3' (SEQ ID NO : 456) | 5'-acttggYTTAATT AATTTTTTTTTTT TTTTTTTTTTTTR2 CTACTATC-3' (SEQ ID NO : 452) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GATAGTAGR1TTTTT TTTTTTTTTTTTTTTTTG GTCGCTTTTTTTTTTTT TTTTXTTTTTTTTTTTT TTTGCGACCTTTTTTTT TTTTTGATACATTTR2G ATGATTTTTTTTTTTTT TTTTTATCATCR1-3' (SEQ ID NO : 456) | 5’-YacttggcatatgTT AATTAATTTTTTT TTTTTTTTTTTTT TTR2CTACTATC- 3' (SEQ ID NO : 453) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GATAGTAGR1TTTTT TTTTTTTTTTTTTTTTTG GTCGCTTTTTTTTTTTT TTTTXTTTTTTTTTTTTT TTTGCGACCTTTTTTTT TTTTTGATACATTTR2G ATGATTTTTTTTTTTTT TTTTTATCATCR1-3' (SEQ ID NO : 456) | 5'-YacttggcatatgTT AATTAATTTTTTT TTTTTTTTTTTTTT TTR2CTACTATC- 3' (SEQ ID NO : 453) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 5' - modèle 5 | | | |
| 5'-GATAGTAGR1TTTTT | 5’-YacttggTTAATT | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTTTTTTTTTTTG CGGCGATCGGCTTTTT TTTTTTTTTTXTTTTTT TTTTTTTTTGCCGATC GCCGCTTTTTTTTTTT TGATACATTTR2GATG ATTTTTTTTTTTTTTTT TATCATCR1-3' (SEQ ID NO : 457) | AATTTTTTTTTTT TTTTTTTTTTTTR2 CTACTATC-3' (SEQ ID NO : 451) | | |
| 5'-GATAGTAGR1TTTTT TTTTTTTTTTTTTTTTTG CGGCGATCGGCTTTTT TTTTTTTTTTTXTTTTTT TTTTTTTTTGCCGATC GCCGCTTTTTTTTTTT TGATACATTTR2GATG ATTTTTTTTTTTTTTTT TATCATCR1-3' (SEQ ID NO : 457) | 5’-YacttggTTAATT AATTTTTTTTTT TTTTTTTTTTTTR2 CTACTATC-3' (SEQ ID NO : 451) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-GATAGTAGR1TTTTT TTTTTTTTTTTTTTTTTG CGGCGATCGGCTTTTT TTTTTTTTTTTXTTTTTT TTTTTTTTTGCCGATC GCCGCTTTTTTTTTTTT TGATACATTTR2GATG ATTTTTTTTTTTTTTTTT TATCATCR1-3' (SEQ ID NO : 457) | 5’-acttggYTTAATT AATTTTTTTTTTT TTTTTTTTTTTTR2 CTACTATC-3' (SEQ ID NO : 452) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GATAGTAGR1TTTTT TTTTTTTTTTTTTTTTTG CGGCGATCGGCTTTTT TTTTTTTTTTTXTTTTTT TTTTTTTTTTGCCGATC GCCGCTTTTTTTTTTTT TGATACATTTR2GATG ATTTTTTTTTTTTTTTTT | 5’-acttggYTTAATT AATTTTTTTTTTT TTTTTTTTTTTTR2 CTACTATC-3' (SEQ ID NO : 452) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| TATCATCR1-3' (SEQ ID NO : 457) | | | |
| 5'-GATAGTAGR1TTTTT TTTTTTTTTTTTTTTTTG CGGCGATCGGCTTTTT TTTTTTTTTTTXTTTTTT TTTTTTTTTGCCGATC GCCGCTTTTTTTTTTTT TGATACATTTR2GATG ATTTTTTTTTTTTTTTTT TATCATCR1-3' (SEQ ID NO : 457) | 5'-YacttggcatatgTT AATTAATTTTTTT TTTTTTTTTTTTTT TTR2CTACTATC- 3' (SEQ ID NO : 453) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-GATAGTAGR1TTTTT TTTTTTTTTTTTTTTTTG CGGCGATCGGCTTTTT TTTTTTTTTTTXTTTTTT TTTTTTTTTGCCGATC GCCGCTTTTTTTTTTTT TGATACATTTR2GATG ATTTTTTTTTTTTTTTT TATCATCR1-3' (SEQ ID NO : 457) | 5'-YacttggcatatgTT AATTAATTTTTTT TTTTTTTTTTTTT TTR2CTACTATC- 3' (SEQ ID NO : 453) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 3' - modèle 1 | | | |
| 5'-CACGTGR1TTTCTCG ATCATTATTATTTTTG GCGATCGCTTTTTTTT TTTTTTTXTTTTTTTTT TTTTTTGCGATCGCCTT TTTTTTTTTTTTTTTTTT TR2GACGAATATTTTTT TTTTTTTTTTTTTTR2CA CGTG-3' (SEQ ID NO : 458) | 5’-TATTCGTCR1T TTTTTTTTTTTTT TTTTATTAATTAT TActtgtaY-3' (SEQ ID NO : 459) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTTG | 5’-TATTCGTCR1T TTTTTTTTTTTTTT | SEQ ID NO : n n étant un nombre impair variant de 1 à 6 et de 269 à 424 | SEQ ID NO : n+1 n étant le même nombre impair que R1 variant de 1 à 6 et de 269 à 424 |
| GCGATCGCTTTTTTTTT TTTTTTTXTTTTTTTTTT TTTTTTGCGATCGCCTT TTTTTTTTTTTTTTTTTT TR2GACGAATATTTTTT TTTTTTTTTTTTTTR2CA CGTG-3’ (SEQ ID NO : 458) | TTTTATTAATTAT TActtgtaY-3' (SEQ ID NO : 459) | | |
| 5'-CACGTGR1TTTCTCG ATCATTATTATTTTTG GCGATCGCTTTTTTTT TTTTTTTXTTTTTTTTT TTTTTTGCGATCGCCTT TTTTTTTTTTTTTTTTTT TR2GACGAATATTTTTT TTTTTTTTTTTTTTR2CA CGTG-3' (SEQ ID NO : 458) | 5’-TATTCGTCR1T TTTTTTTTTTTTT TTTTATTAATTAT TAYcttgta-3' (SEQ ID NO : 460) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTG GCGATCGCTTTTTTTT TTTTTTTXTTTTTTTTT TTTTTTGCGATCGCCTT TTTTTTTTTTTTTTTTTT TR2GACGAATATTTTTT TTTTTTTTTTTTTTR2CA CGTG-3' (SEQ ID NO : 458) | 5'-TATTCGTCR1T TTTTTTTTTTTTT TTTTATTAATTAT TAYcttgta-3' (SEQ ID NO : 460) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-CACGTGR1TTTCTCG ATCATTATTATTTTTG GCGATCGCTTTTTTTT TTTTTTTXTTTTTTTTT TTTTTTGCGATCGCCTT TTTTTTTTTTTTTTTTTT TR2GACGAATATTTTTT TTTTTTTTTTTTTTTR2CA CGTG-3' | 5’-TATTCGTCR1T TTTTTTTTTTTTT TTTTATTAATTAT TATCCGGActtgtaY -3' (SEQ ID NO : 461) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| (SEQ ID NO : 458) | | | |
| 5'-CACGTGR1TTTCTCG ATCATTATTATTTTTG GCGATCGCTTTTTTTT TTTTTTTXTTTTTTTTTT TTTTTTGCGATCGCCTT TTTTTTTTTTTTTTTTTTT TR2GACGAATATTTTTT TTTTTTTTTTTTTTTR2CA CGTG-3' (SEQ ID NO : 458) | 5'-TATTCGTCR1T TTTTTTTTTTTTT TTTTATTAATTAT TATCCGGActtgtaY -3' (SEQ ID NO : 461) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 3' - modèle 2 | | | |
| 5'-CACGTGR1TTTCTCG ATCATTATTATTTTTG GTCGCTTTTTTTTTTT TTTTXTTTTTTTTTTTT TTTGCGACCTTTTTTT TTTTTTTTTTTTTR2GA CGAATATTTTTTTTTT TTTTTTTTTR2CACGTG- 3' (SEQ ID NO : 462) | 5'-TATTCGTCR1T TTTTTTTTTTTTT TTTTATTAATTAT TActtgtaY-3' (SEQ ID NO : 459) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTG GTCGCTTTTTTTTTTT TTTTXTTTTTTTTTTT TTTGCGACCTTTTTTT TTTTTTTTTTTTR2GA CGAATATTTTTTTTTT TTTTTTTTR2CACGTG- 3' (SEQ ID NO : 462) | 5'-TATTCGTCR1T TTTTTTTTTTTTT TTTTATTAATTAT TActtgtaY-3' (SEQ ID NO : 459) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-CACGTGR1TTTCTCG ATCATTATTATTTTTTG GTCGCTTTTTTTTTTTT | 5'-TATTCGTCR1T TTTTTTTTTTTTT TTTTATTAATTAT | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTXTTTTTTTTTTTTT TTTGCGACCTTTTTTTT TTTTTTTTTTTTTR2GA CGAATATTTTTTTTTTT TTTTTTTTTR2CACGTG- 3’ (SEQ ID NO : 462) | TAYcttgta-3' (SEQ ID NO : 460) | | |
| 5'-CACGTGR1TTTCTCG ATCATTATTATTTTTG GTCGCTTTTTTTTTTT TTTTXTTTTTTTTTTT TTTGCGACCTTTTTTT TTTTTTTTTTTTTR2GA CGAATATTTTTTTTTT TTTTTTTTTR2CACGTG- 3' (SEQ ID NO : 462) | 5'-TATTCGTCR1T TTTTTTTTTTTTT TTTTATTAATTAT TAYcttgta-3' (SEQ ID NO : 460) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-CACGTGR1TTTCTCG ATCATTATTATTTTTG GTCGCTTTTTTTTTTT TTTTXTTTTTTTTTTTT TTTGCGACCTTTTTTT TTTTTTTTTTTTTR2GA CGAATATTTTTTTTTT TTTTTTTTTR2CACGTG- 3' (SEQ ID NO : 462) | 5'-TATTCGTCR1T TTTTTTTTTTTTT TTTTATTAATTAT TATCCGGActtgtaY -3' (SEQ ID NO : 461) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTG GTCGCTTTTTTTTTTT TTTTXTTTTTTTTTTT TTTGCGACCTTTTTTT TTTTTTTTTTTTTR2GA CGAATATTTTTTTTTT TTTTTTTTR2CACGTG- 3' (SEQ ID NO : 462) | 5’-TATTCGTCR1T TTTTTTTTTTTTT TTTTATTAATTAT TATCCGGActtgtaY -3' (SEQ ID NO : 461) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Insertion 3' - modèle 3 | | | |
|---|---|---|---|
| 5'-CACGTGR1TTTCTCG ATCATTATTATTTTTG CGGCGATCGGCTTTTT TTTTTTTTTTXTTTTTT TTTTTTTTTGCCGATC GCCGCTTTTTTTTTTT TTTTTTTTR2GACGAA TATTTTTTTTTTTTTTT TTTTR2CACGTG-3' (SEQ ID NO : 463) | 5'-TATTCGTCR1T TTTTTTTTTTTTT TTTTATTAATTAT TActtgtaY-3' (SEQ ID NO : 459) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTG CGGCGATCGGCTTTTT TTTTTTTTTTXTTTTTT TTTTTTTTTGCCGATC GCCGCTTTTTTTTTTT TTTTTTTTTR2GACGAA TATTTTTTTTTTTTTTT TTTTR2CACGTG-3' (SEQ ID NO : 463) | 5'-TATTCGTCR1T TTTTTTTTTTTTT TTTTATTAATTAT TActtgtaY-3' (SEQ ID NO : 459) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-CACGTGR1TTTCTCG ATCATTATTATTTTTG CGGCGATCGGCTTTTT TTTTTTTTTTXTTTTTT TTTTTTTTTGCCGATC GCCGCTTTTTTTTTTT TTTTTTTTR2GACGAA TATTTTTTTTTTTTTTT TTTTR2CACGTG-3' (SEQ ID NO : 463) | 5'-TATTCGTCR1T TTTTTTTTTTTTT TTTTATTAATTAT TAYcttgta-3' (SEQ ID NO : 460) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTG CGGCGATCGGCTTTTT TTTTTTTTTTTXTTTTTT TTTTTTTTTTGCCGATC | 5'-TATTCGTCR1T TTTTTTTTTTTTT TTTTATTAATTAT TAYcttgta-3' (SEQ ID NO : 460) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| GCCGCTTTTTTTTTTTT TTTTTTTTTR2GACGAA TATTTTTTTTTTTTTTTT TTTTR2CACGTG-3’ (SEQ ID NO : 463) | | | |
| 5'-CACGTGR1TTTCTCG ATCATTATTATTTTTG CGGCGATCGGCTTTTT TTTTTTTTTTXTTTTTT TTTTTTTTTGCCGATC GCCGCTTTTTTTTTTT TTTTTTTTTR2GACGAA TATTTTTTTTTTTTTTT TTTTR2CACGTG-3' (SEQ ID NO : 463) | 5’-TATTCGTCR1T TTTTTTTTTTTTTT TTTTATTAATTAT TATCCGGActtgtaY -3' (SEQ ID NO : 461) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-CACGTGR1TTTCTCG ATCATTATTATTTTTG CGGCGATCGGCTTTTT TTTTTTTTTTXTTTTTT TTTTTTTTTGCCGATC GCCGCTTTTTTTTTTT TTTTTTTTR2GACGAA TATTTTTTTTTTTTTTT TTTTR2CACGTG-3' (SEQ ID NO : 463) | 5’-TATTCGTCR1T TTTTTTTTTTTTTT TTTTATTAATTAT TATCCGGActtgtaY -3' (SEQ ID NO : 461) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 3' - modèle 4 | | | |
| 5'-R2CACGTGTTTTTTT TTTTTTTTTTTTTCACG TGR1TTTCTCGATCATT ATTATTTTTGGCGATC GCTTTTTTTTTTTTTTT XTTTTTTTTTTTTTTTTG CGATCGCCTTTTTTTTT TTTTTTTTTTTTR2GAC GAATA-3' (SEQ ID NO : 464) | 5’-TATTCGTCR1T TTTTTTTTTTTTT TTTTATTAATTAT TActtgtaY-3' (SEQ ID NO : 459) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-R2CACGTGTTTTTTT TTTTTTTTTTTTTCACG TGR1TTTCTCGATCATT ATTATTTTTGGCGATC GCTTTTTTTTTTTTTT XTTTTTTTTTTTTTTTG CGATCGCCTTTTTTTT TTTTTTTTTTTR2GAC GAATA-3' (SEQ ID NO : 464) | 5'-TATTCGTCR1T TTTTTTTTTTTTT TTTTATTAATTAT TActtgtaY-3' (SEQ ID NO : 459) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-R2CACGTGTTTTTTT TTTTTTTTTTTTTCACG TGR1TTTCTCGATCATT ATTATTTTTGGCGATC GCTTTTTTTTTTTTTTT XTTTTTTTTTTTTTTTTG CGATCGCCTTTTTTTTT TTTTTTTTTTTTR2GAC GAATA-3' (SEQ ID NO : 464) | 5’-TATTCGTCR1T TTTTTTTTTTTTTT TTTTATTAATTAT TAYcttgta-3' (SEQ ID NO : 460) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5’-**R2**CACGTGTTTTTTT TTTTTTTTTTTTTCACG TG**R1**TTTCTCGATCATT ATTATTTTTTGGCGATC GCTTTTTTTTTTTTTTT **X**TTTTTTTTTTTTTTTG CGATCGCCTTTTTTTTT TTTTTTTTTTTTT**R2**GAC GAATA-3’ (**SEQ ID NO : 464**) | 5’-TATTCGTCR1T TTTTTTTTTTTTT TTTTATTAATTAT TAYcttgta-3' (SEQ ID NO : 460) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-R2CACGTGTTTTTTT TTTTTTTTTTTTTCACG TGR1TTTCTCGATCATT ATTATTTTTGGCGATC GCTTTTTTTTTTTTTTT XTTTTTTTTTTTTTTTG CGATCGCCTTTTTTTTT | 5’-TATTCGTCR1T TTTTTTTTTTTTT TTTTATTAATTAT TATCCGGActtgtaY -3' (SEQ ID NO : 461) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTTTTTTTR2GAC GAATA-3’ (SEQ ID NO : 464) | | | |
| 5'-R2CACGTGTTTTTTT TTTTTTTTTTTTTCACG TGR1TTTCTCGATCATT ATTATTTTTGGCGATC GCTTTTTTTTTTTTTTT XTTTTTTTTTTTTTTTTG CGATCGCCTTTTTTTTT TTTTTTTTTTTTR2GAC GAATA-3' (SEQ ID NO : 464) | 5’-TATTCGTCR1T TTTTTTTTTTTTT TTTTATTAATTAT TATCCGGActtgtaY -3' (SEQ ID NO : 461) | SEQ ID NO : n n étant un nombre impair variant de 1 à 6 et de 269 à 424 | SEQ ID NO : n+1 n étant le même nombre impair que R1 variant de 1 à 6 et de 269 à 424 |

| Séquences avec deux séquences de transposase | | | |
|---|---|---|---|
| Insertion 3' - modèle 5 | | | |
| 5'-R2CACGTGTTTTTTT TTTTTTTTTTTTTCACG TGR1TTTCTCGATCATT ATTATTTTTGGTCGCT TTTTTTTTTTTTTTXTT TTTTTTTTTTTTTTGCG ACCTTTTTTTTTTTTTT TTTTTTR2GACGAATA- 3' (SEQ ID NO : 465) | 5’-TATTCGTCR1T TTTTTTTTTTTTT TTTTATTAATTAT TActtgtaY-3' (SEQ ID NO : 459) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-R2CACGTGTTTTTTT TTTTTTTTTTTTTCACG TGR1TTTCTCGATCATT ATTATTTTTGGTCGCT TTTTTTTTTTTTTTXTT TTTTTTTTTTTTTTGCG ACCTTTTTTTTTTTTTT TTTTTTR2GACGAATA- 3' (SEQ ID NO : 465) | 5'-TATTCGTCR1T TTTTTTTTTTTTT TTTTATTAATTAT TActtgtaY-3' (SEQ ID NO : 459) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-R2CACGTGTTTTTTT | 5'-TATTCGTCR1T | SEQ ID NO : n n étant un nombre pair variant de 1 à 6 et de 269 à 424 | SEQ ID NO : n-1 n étant le même nombre pair que R1 variant de 1 à 6 et de 269 à 424 |
| TTTTTTTTTTTTTCACG TGR1TTTCTCGATCATT ATTATTTTTGGTCGCT TTTTTTTTTTTTTTXTT TTTTTTTTTTTTTGCG ACCTTTTTTTTTTTTTT TTTTTTR2GACGAATA- 3' (SEQ ID NO : 465) | TTTTTTTTTTTTT TTTTATTAATTAT TAYcttgta-3' (SEQ ID NO : 460) | | |
| 5'-R2CACGTGTTTTTTT TTTTTTTTTTTTTCACG TGR1TTTCTCGATCATT ATTATTTTTGGTCGCT TTTTTTTTTTTTTTXTT TTTTTTTTTTTTTGCG ACCTTTTTTTTTTTTTT TTTTTTR2GACGAATA- 3' (SEQ ID NO : 465) | 5'-TATTCGTCR1T TTTTTTTTTTTTT TTTTATTAATTAT TAYcttgta-3' (SEQ ID NO : 460) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-R2CACGTGTTTTTTT TTTTTTTTTTTTTCACG TGR1TTTCTCGATCATT ATTATTTTTGGTCGCT TTTTTTTTTTTTTTTXTT TTTTTTTTTTTTTTGCG ACCTTTTTTTTTTTTTT TTTTTTR2GACGAATA- 3' (SEQ ID NO : 465) | 5’-TATTCGTCR1T TTTTTTTTTTTTTT TTTTATTAATTAT TATCCGGActtgtaY -3' (SEQ ID NO : 461) 1 | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-R2CACGTGTTTTTTT TTTTTTTTTTTTTCACG TGR1TTTCTCGATCATT ATTATTTTTGGTCGCT TTTTTTTTTTTTTTXTT TTTTTTTTTTTTTTGCG ACCTTTTTTTTTTTTTT TTTTTTR2GACGAATA- | 5’-TATTCGTCR1T TTTTTTTTTTTTTT TTTTATTAATTAT TATCCGGActtgtaY -3' (SEQ ID NO : 461) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 3’ (SEQ ID NO : 465) | | | |

| Séquences avec deux séquences de transposase | | | |
|---|---|---|---|
| Insertion 3' - modèle 6 | | | |
| 5'-R2CACGTGTTTTTTT TTTTTTTTTTTTTCACG TGR1TTTCTCGATCATT ATTATTTTTGCGGCG ATCGGCTTTTTTTTTT TTTTTXTTTTTTTTTTT TTTTGCCGATCGCCGC TTTTTTTTTTTTTTTTT TTTR2GACGAATA-3' (SEQ ID NO : 466) | 5'-TATTCGTCR1T TTTTTTTTTTTTT TTTTATTAATTAT TActtgtaY-3' (SEQ ID NO : 459) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-R2CACGTGTTTTTTT TTTTTTTTTTTTTCACG TGR1TTTCTCGATCATT ATTATTTTTGCGGCG ATCGGCTTTTTTTTTT TTTTTXTTTTTTTTTTT TTTTGCCGATCGCCGC TTTTTTTTTTTTTTTTT TTTR2GACGAATA-3' (SEQ ID NO : 466) | 5'-TATTCGTCR1T TTTTTTTTTTTTT TTTTATTAATTAT TActtgtaY-3' (SEQ ID NO : 459) | SEQ ID NO : n n étant un nombre impair variant de 1 à 6 et de 269 à 424 | SEQ ID NO : n+1 n étant le même nombre impair que R1 variant de 1 à 6 et de 269 à 424 |
| 5'-R2CACGTGTTTTTTT TTTTTTTTTTTTTCACG TGR1TTTCTCGATCATT ATTATTTTTGCGGCG ATCGGCTTTTTTTTTT TTTTTXTTTTTTTTTTT TTTTGCCGATCGCCGC TTTTTTTTTTTTTTTTT TTTR2GACGAATA-3' (SEQ ID NO : 466) | 5'-TATTCGTCR1T TTTTTTTTTTTTT TTTTATTAATTAT TAYcttgta-3' (SEQ ID NO : 460) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-**R2**CACGTGTTTTTTT TTTTTTTTTTTTTTTCACG | 5'-TATTCGTCR1T TTTTTTTTTTTTTT | SEQ ID NO : n n étant un nombre impair variant de 1 à 6 et de 269 à 424 | SEQ ID NO : n+1 n étant le même nombre impair que R1 variant de 1 à 6 et de 269 à 424 |
| TGR1TTTCTCGATCATT ATTATTTTTGCGGCG ATCGGCTTTTTTTTTT TTTTTXTTTTTTTTTTT TTTTGCCGATCGCCGC TTTTTTTTTTTTTTTTT TTTR2GACGAATA-3’ (SEQ ID NO : 466) | TTTTATTAATTAT TAYcttgta-3' (SEQ ID NO : 460) | | |
| 5'-R2CACGTGTTTTTTT TTTTTTTTTTTTTCACG TGR1TTTCTCGATCATT ATTATTTTTGCGGCG ATCGGCTTTTTTTTTT TTTTTXTTTTTTTTTTT TTTTGCCGATCGCCGC TTTTTTTTTTTTTTTTT TTTR2GACGAATA-3' (SEQ ID NO : 466) | 5’-TATTCGTCR1T TTTTTTTTTTTTTT TTTTATTAATTAT TATCCGGActtgtaY -3' (SEQ ID NO : 461) | SEQ ID NO : n n étant un nombre pair variant de 1 à 6 et de 269 à 424 | SEQ ID NO : n-1 n étant le même nombre pair que R1 variant de 1 à 6 et de 269 à 424 |
| 5'-R2CACGTGTTTTTTT TTTTTTTTTTTTTCACG TGR1TTTCTCGATCATT ATTATTTTTGCGGCG ATCGGCTTTTTTTTTT TTTTTXTTTTTTTTTTT TTTTGCCGATCGCCGC TTTTTTTTTTTTTTTTT TTTR2GACGAATA-3' (SEQ ID NO : 466) | 5’-TATTCGTCR1T TTTTTTTTTTTTT TTTTATTAATTAT TATCCGGActtgtaY -3' (SEQ ID NO : 461) | SEQ ID NO : n n étant un nombre impair variant de 1 à 6 et de 269 à 424 | SEQ ID NO : n+1 n étant le même nombre impair que R1 variant de 1 à 6 et de 269 à 424 |

En d'autres termes l'invention concerne avantageusement un complexe susmentionné, le complexe comprenant les couples de première et de deuxième molécules, lesdites première et de deuxième molécules comprenant les séquences respectives suivantes :
- SEQ ID NO : 440 et SEQ ID NO : 441, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 440 et SEQ ID NO : 441, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 440 et SEQ ID NO : 442, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 440 et SEQ ID NO : 442, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 440 et SEQ ID NO : 443, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 440 et SEQ ID NO : 443, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 444 et SEQ ID NO : 445, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 444 et SEQ ID NO : 445, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 444 et SEQ ID NO : 446, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 444 et SEQ ID NO : 446, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 444 et SEQ ID NO : 447, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 444 et SEQ ID NO : 447, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 448 et SEQ ID NO : 445, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 448 et SEQ ID NO : 445, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 448 et SEQ ID NO : 446, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 448 et SEQ ID NO : 446, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 448 et SEQ ID NO : 447, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 448 et SEQ ID NO : 447, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 449 et SEQ ID NO : 445, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 449 et SEQ ID NO : 445, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 449 et SEQ ID NO : 446, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 449 et SEQ ID NO : 446, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 449 et SEQ ID NO : 447, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 449 et SEQ ID NO : 447, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 450 et SEQ ID NO : 451, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 450 et SEQ ID NO : 451, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 450 et SEQ ID NO : 452, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 450 et SEQ ID NO : 452, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 450 et SEQ ID NO : 453, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 450 et SEQ ID NO : 453, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 454 et SEQ ID NO : 451, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 454 et SEQ ID NO : 451, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 454 et SEQ ID NO : 452, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 454 et SEQ ID NO : 452, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 454 et SEQ ID NO : 453, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 454 et SEQ ID NO : 453, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 455 et SEQ ID NO : 451, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 455 et SEQ ID NO : 451, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 455 et SEQ ID NO : 452, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 455 et SEQ ID NO : 452, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 455 et SEQ ID NO : 453, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 455 et SEQ ID NO : 453, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 456 et SEQ ID NO : 451, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 456 et SEQ ID NO : 451, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 456 et SEQ ID NO : 452, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 456 et SEQ ID NO : 452, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 456 et SEQ ID NO : 453, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 456 et SEQ ID NO : 453, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 457 et SEQ ID NO : 451, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 457 et SEQ ID NO : 451, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 457 et SEQ ID NO : 452, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 457 et SEQ ID NO : 452, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 457 et SEQ ID NO : 453, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 457 et SEQ ID NO : 453, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 458 et SEQ ID NO : 459, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 458 et SEQ ID NO : 459, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 458 et SEQ ID NO : 460, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 458 et SEQ ID NO : 460, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 458 et SEQ ID NO : 461, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 458 et SEQ ID NO : 461, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 462 et SEQ ID NO : 459, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 462 et SEQ ID NO : 459, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 462 et SEQ ID NO : 460, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 462 et SEQ ID NO : 460, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 462 et SEQ ID NO : 461, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 462 et SEQ ID NO : 461, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 463 et SEQ ID NO : 459, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 463 et SEQ ID NO : 459, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 463 et SEQ ID NO : 460, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 463 et SEQ ID NO : 460, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 463 et SEQ ID NO : 461, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 463 et SEQ ID NO : 461, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 464 et SEQ ID NO : 459, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 464 et SEQ ID NO : 459, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 464 et SEQ ID NO : 460, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 464 et SEQ ID NO : 460, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 464 et SEQ ID NO : 461, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 464 et SEQ ID NO : 461, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 465 et SEQ ID NO : 459, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 465 et SEQ ID NO : 459, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 465 et SEQ ID NO : 460, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 465 et SEQ ID NO : 460, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 465 et SEQ ID NO : 461, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 465 et SEQ ID NO : 461, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 466 et SEQ ID NO : 459, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 466 et SEQ ID NO : 459, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 466 et SEQ ID NO : 460, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424,
- SEQ ID NO : 466 et SEQ ID NO : 460, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424,
- SEQ ID NO : 466 et SEQ ID NO : 461, où R1 est l'une quelconque des séquences SEQ ID NO : n, n étant un chiffre pair variant de 269 à 424, et R2 est l'une quelconque des séquences SEQ ID NO : n+1, n étant le même chiffre pair que celui de R1 variant de 269 à 424, et
- SEQ ID NO : 466 et SEQ ID NO : 461, où R1 est l'une quelconque des séquences SEQ ID NO : n, n étant un chiffre impaire variant de 269 à 424, et R2 est l'une quelconque des séquences SEQ ID NO : n-1, n étant le même chiffre impaire que celui de R1 variant de 269 à 424.

De manière avantageuse, l'invention concerne le complexe susmentionné, ledit complexe comprenant l'un des 300 couples de première et deuxième molécule du tableau 4 suivant :

**[Tableaux4]**

| # | la première molécule comprenant la séquence | la deuxième molécule comprenant la séquence |
|---|---|---|
| 1 | SEQ ID NO: 467 | SEQ ID NO :503, |
| 2 | SEQ ID NO : 468 | SEQ ID NO :504, |
| 3 | SEQ ID NO : 469 | SEQ ID NO :503, |
| 4 | SEQ ID NO : 470 | SEQ ID NO :504, |
| 5 | SEQ ID NO : 469 | SEQ ID NO :505, |
| 6 | SEQ ID NO : 470 | SEQ ID NO :506, |
| 7 | SEQ ID NO : 471 | SEQ ID NO :503, |
| 8 | SEQ ID NO : 472 | SEQ ID NO :504, |
| 9 | SEQ ID NO : 471 | SEQ ID NO :505, |
| 10 | SEQ ID NO : 472 | SEQ ID NO :506, |
| 11 | SEQ ID NO : 473 | SEQ ID NO :507, |
| 12 | SEQ ID NO : 474 | SEQ ID NO :508, |
| 13 | SEQ ID NO : 473 | SEQ ID NO :509, |
| 14 | SEQ ID NO : 474 | SEQ ID NO :510, |
| 15 | SEQ ID NO : 473 | SEQ ID NO :511, |
| 16 | SEQ ID NO : 474 | SEQ ID NO :512, |
| 17 | SEQ ID NO : 475 | SEQ ID NO :507, |
| 18 | SEQ ID NO : 476 | SEQ ID NO :508, |
| 19 | SEQ ID NO : 475 | SEQ ID NO :509, |
| 20 | SEQ ID NO : 476 | SEQ ID NO :510, |
| 21 | SEQ ID NO : 475 | SEQ ID NO :511, |
| 22 | SEQ ID NO : 476 | SEQ ID NO :512, |
| 23 | SEQ ID NO : 477 | SEQ ID NO :507, |
| 24 | SEQ ID NO : 478 | SEQ ID NO :508, |
| 25 | SEQ ID NO : 477 | SEQ ID NO :509, |
| 26 | SEQ ID NO : 478 | SEQ ID NO :510, |
| 27 | SEQ ID NO : 477 | SEQ ID NO :511, |
| 28 | SEQ ID NO : 478 | SEQ ID NO :512, |
| 29 | SEQ ID NO : 479 | SEQ ID NO :513, |
| 30 | SEQ ID NO : 480 | SEQ ID NO :514, |
| 31 | SEQ ID NO : 479 | SEQ ID NO :515, |
| 32 | SEQ ID NO : 480 | SEQ ID NO :516, |
| 33 | SEQ ID NO : 479 | SEQ ID NO :517, |
| 34 | SEQ ID NO : 480 | SEQ ID NO :518, |
| 35 | SEQ ID NO : 481 | SEQ ID NO :513, |
| 36 | SEQ ID NO : 482 | SEQ ID NO :514, |
| 37 | SEQ ID NO : 481 | SEQ ID NO :515, |
| 38 | SEQ ID NO : 482 | SEQ ID NO :516, |
| 39 | SEQ ID NO : 481 | SEQ ID NO :517, |
| 40 | SEQ ID NO : 482 | SEQ ID NO :518, |
| 41 | SEQ ID NO : 483 | SEQ ID NO :513, |
| 42 | SEQ ID NO : 484 | SEQ ID NO :514, |
| 43 | SEQ ID NO : 483 | SEQ ID NO :515, |
| 44 | SEQ ID NO : 484 | SEQ ID NO :516, |
| 45 | SEQ ID NO : 483 | SEQ ID NO :517, |
| 46 | SEQ ID NO : 484 | SEQ ID NO :518, |
| 47 | SEQ ID NO : 485 | SEQ ID NO :513, |
| 48 | SEQ ID NO : 486 | SEQ ID NO :514, |
| 49 | SEQ ID NO : 485 | SEQ ID NO :515, |
| 50 | SEQ ID NO : 486 | SEQ ID NO :516, |
| 51 | SEQ ID NO : 485 | SEQ ID NO :517, |
| 52 | SEQ ID NO : 486 | SEQ ID NO :518, |
| 53 | SEQ ID NO : 487 | SEQ ID NO :513, |
| 54 | SEQ ID NO : 488 | SEQ ID NO :514, |
| 55 | SEQ ID NO : 487 | SEQ ID NO :515, |
| 56 | SEQ ID NO : 488 | SEQ ID NO :516, |
| 57 | SEQ ID NO : 487 | SEQ ID NO :517, |
| 58 | SEQ ID NO : 488 | SEQ ID NO :518, |
| 59 | SEQ ID NO : 489 | SEQ ID NO :513, |
| 60 | SEQ ID NO : 490 | SEQ ID NO :514, |
| 61 | SEQ ID NO : 489 | SEQ ID NO :515, |
| 62 | SEQ ID NO : 490 | SEQ ID NO :516, |
| 63 | SEQ ID NO : 489 | SEQ ID NO :517, |
| 64 | SEQ ID NO : 490 | SEQ ID NO :518, |
| 65 | SEQ ID NO : 491 | SEQ ID NO :519, |
| 66 | SEQ ID NO : 492 | SEQ ID NO :520, |
| 67 | SEQ ID NO : 491 | SEQ ID NO :521, |
| 68 | SEQ ID NO : 492 | SEQ ID NO :522, |
| 69 | SEQ ID NO : 491 | SEQ ID NO :523, |
| 70 | SEQ ID NO : 492 | SEQ ID NO :524, |
| 71 | SEQ ID NO : 493 | SEQ ID NO :519, |
| 72 | SEQ ID NO : 494 | SEQ ID NO :520, |
| 73 | SEQ ID NO : 493 | SEQ ID NO :521, |
| 74 | SEQ ID NO : 494 | SEQ ID NO :522, |
| 75 | SEQ ID NO : 493 | SEQ ID NO :523, |
| 76 | SEQ ID NO : 494 | SEQ ID NO :524, |
| 77 | SEQ ID NO : 495 | SEQ ID NO :519, |
| 78 | SEQ ID NO : 496 | SEQ ID NO :520, |
| 79 | SEQ ID NO : 495 | SEQ ID NO :521, |
| 80 | SEQ ID NO : 496 | SEQ ID NO :522, |
| 81 | SEQ ID NO : 495 | SEQ ID NO :523, |
| 82 | SEQ ID NO : 496 | SEQ ID NO :524, |
| 83 | SEQ ID NO : 497 | SEQ ID NO :519, |
| 84 | SEQ ID NO : 498 | SEQ ID NO :520, |
| 85 | SEQ ID NO : 497 | SEQ ID NO :521, |
| 86 | SEQ ID NO : 498 | SEQ ID NO :522, |
| 87 | SEQ ID NO : 497 | SEQ ID NO :523, |
| 88 | SEQ ID NO : 498 | SEQ ID NO :524, |
| 89 | SEQ ID NO : 499 | SEQ ID NO :519, |
| 90 | SEQ ID NO : 500 | SEQ ID NO :520, |
| 91 | SEQ ID NO : 499 | SEQ ID NO :521, |
| 92 | SEQ ID NO : 500 | SEQ ID NO :522, |
| 93 | SEQ ID NO : 499 | SEQ ID NO :523, |
| 94 | SEQ ID NO : 500 | SEQ ID NO :524, |
| 95 | SEQ ID NO : 501 | SEQ ID NO :519, |
| 96 | SEQ ID NO : 502 | SEQ ID NO :520, |
| 97 | SEQ ID NO : 501 | SEQ ID NO :521, |
| 98 | SEQ ID NO : 502 | SEQ ID NO :522, |
| 99 | SEQ ID NO : 501 | SEQ ID NO :523, |
| 100 | SEQ ID NO : 502 | SEQ ID NO :524, |
| 101 | SEQ ID NO : 525 | SEQ ID NO :561, |
| 102 | SEQ ID NO : 526 | SEQ ID NO :562, |
| 103 | SEQ ID NO : 527 | SEQ ID NO :561, |
| 104 | SEQ ID NO : 528 | SEQ ID NO :562, |
| 105 | SEQ ID NO : 527 | SEQ ID NO :563, |
| 106 | SEQ ID NO : 528 | SEQ ID NO :564, |
| 107 | SEQ ID NO : 529 | SEQ ID NO :561, |
| 108 | SEQ ID NO : 530 | SEQ ID NO :562, |
| 109 | SEQ ID NO : 529 | SEQ ID NO :563, |
| 110 | SEQ ID NO : 530 | SEQ ID NO :564, |
| 111 | SEQ ID NO : 531 | SEQ ID NO :565, |
| 112 | SEQ ID NO : 532 | SEQ ID NO :566, |
| 113 | SEQ ID NO : 531 | SEQ ID NO :567, |
| 114 | SEQ ID NO : 532 | SEQ ID NO :568, |
| 115 | SEQ ID NO : 531 | SEQ ID NO :569, |
| 116 | SEQ ID NO : 532 | SEQ ID NO :570, |
| 117 | SEQ ID NO : 533 | SEQ ID NO :565, |
| 118 | SEQ ID NO : 534 | SEQ ID NO :566, |
| 119 | SEQ ID NO : 533 | SEQ ID NO :567, |
| 120 | SEQ ID NO : 534 | SEQ ID NO :568, |
| 121 | SEQ ID NO : 533 | SEQ ID NO :569, |
| 122 | SEQ ID NO : 534 | SEQ ID NO :570, |
| 123 | SEQ ID NO : 535 | SEQ ID NO :565, |
| 124 | SEQ ID NO : 536 | SEQ ID NO :566, |
| 125 | SEQ ID NO : 535 | SEQ ID NO :567, |
| 126 | SEQ ID NO : 536 | SEQ ID NO :568, |
| 127 | SEQ ID NO : 535 | SEQ ID NO :569, |
| 128 | SEQ ID NO : 536 | SEQ ID NO :570, |
| 129 | SEQ ID NO : 537 | SEQ ID NO :571, |
| 130 | SEQ ID NO : 538 | SEQ ID NO :572, |
| 131 | SEQ ID NO : 537 | SEQ ID NO :573, |
| 132 | SEQ ID NO : 538 | SEQ ID NO :574, |
| 133 | SEQ ID NO : 537 | SEQ ID NO :575, |
| 134 | SEQ ID NO : 538 | SEQ ID NO :576, |
| 135 | SEQ ID NO : 539 | SEQ ID NO :571, |
| 136 | SEQ ID NO : 540 | SEQ ID NO :572, |
| 137 | SEQ ID NO : 539 | SEQ ID NO :573, |
| 138 | SEQ ID NO : 540 | SEQ ID NO :574, |
| 139 | SEQ ID NO : 539 | SEQ ID NO :575, |
| 140 | SEQ ID NO : 540 | SEQ ID NO :576, |
| 141 | SEQ ID NO : 541 | SEQ ID NO :571, |
| 142 | SEQ ID NO : 542 | SEQ ID NO :572, |
| 143 | SEQ ID NO : 541 | SEQ ID NO :573, |
| 144 | SEQ ID NO : 542 | SEQ ID NO :574, |
| 145 | SEQ ID NO : 541 | SEQ ID NO :575, |
| 146 | SEQ ID NO : 542 | SEQ ID NO :576, |
| 147 | SEQ ID NO : 543 | SEQ ID NO :571, |
| 148 | SEQ ID NO : 544 | SEQ ID NO :572, |
| 149 | SEQ ID NO : 543 | SEQ ID NO :573, |
| 150 | SEQ ID NO : 544 | SEQ ID NO :574, |
| 151 | SEQ ID NO : 543 | SEQ ID NO :575, |
| 152 | SEQ ID NO : 544 | SEQ ID NO :576, |
| 153 | SEQ ID NO : 545 | SEQ ID NO :571, |
| 154 | SEQ ID NO : 546 | SEQ ID NO :572, |
| 155 | SEQ ID NO : 545 | SEQ ID NO :573, |
| 156 | SEQ ID NO : 546 | SEQ ID NO :574, |
| 157 | SEQ ID NO : 545 | SEQ ID NO :575, |
| 158 | SEQ ID NO : 546 | SEQ ID NO :576, |
| 159 | SEQ ID NO : 547 | SEQ ID NO :571, |
| 160 | SEQ ID NO : 548 | SEQ ID NO :572, |
| 161 | SEQ ID NO : 547 | SEQ ID NO :573, |
| 162 | SEQ ID NO : 548 | SEQ ID NO :574, |
| 163 | SEQ ID NO : 547 | SEQ ID NO :575, |
| 164 | SEQ ID NO : 548 | SEQ ID NO :576, |
| 165 | SEQ ID NO : 549 | SEQ ID NO :577, |
| 166 | SEQ ID NO : 550 | SEQ ID NO :578, |
| 167 | SEQ ID NO : 549 | SEQ ID NO :579, |
| 168 | SEQ ID NO : 550 | SEQ ID NO :580, |
| 169 | SEQ ID NO : 549 | SEQ ID NO :581, |
| 170 | SEQ ID NO : 550 | SEQ ID NO :582, |
| 171 | SEQ ID NO : 551 | SEQ ID NO :577, |
| 172 | SEQ ID NO : 552 | SEQ ID NO :578, |
| 173 | SEQ ID NO : 551 | SEQ ID NO :579, |
| 174 | SEQ ID NO : 552 | SEQ ID NO :580, |
| 175 | SEQ ID NO : 551 | SEQ ID NO :581, |
| 176 | SEQ ID NO : 552 | SEQ ID NO :582, |
| 177 | SEQ ID NO : 553 | SEQ ID NO :577, |
| 178 | SEQ ID NO : 554 | SEQ ID NO :578, |
| 179 | SEQ ID NO : 553 | SEQ ID NO :579, |
| 180 | SEQ ID NO : 554 | SEQ ID NO :580, |
| 181 | SEQ ID NO : 553 | SEQ ID NO :581, |
| 182 | SEQ ID NO : 554 | SEQ ID NO :582, |
| 183 | SEQ ID NO : 555 | SEQ ID NO :577, |
| 184 | SEQ ID NO : 556 | SEQ ID NO :578, |
| 185 | SEQ ID NO : 555 | SEQ ID NO :579, |
| 186 | SEQ ID NO : 556 | SEQ ID NO :580, |
| 187 | SEQ ID NO : 555 | SEQ ID NO :581, |
| 188 | SEQ ID NO : 556 | SEQ ID NO :582, |
| 189 | SEQ ID NO : 557 | SEQ ID NO :577, |
| 190 | SEQ ID NO : 558 | SEQ ID NO :578, |
| 191 | SEQ ID NO : 557 | SEQ ID NO :579, |
| 192 | SEQ ID NO : 558 | SEQ ID NO :580, |
| 193 | SEQ ID NO : 557 | SEQ ID NO :581, |
| 194 | SEQ ID NO : 558 | SEQ ID NO :582, |
| 195 | SEQ ID NO : 559 | SEQ ID NO :577, |
| 196 | SEQ ID NO : 560 | SEQ ID NO :578, |
| 197 | SEQ ID NO : 559 | SEQ ID NO :579, |
| 198 | SEQ ID NO : 560 | SEQ ID NO :580, |
| 199 | SEQ ID NO : 559 | SEQ ID NO :581, |
| 200 | SEQ ID NO : 560 | SEQ ID NO :582, |
| 201 | SEQ ID NO : 583 | SEQ ID NO :619, |
| 202 | SEQ ID NO : 584 | SEQ ID NO :620, |
| 203 | SEQ ID NO : 585 | SEQ ID NO :619, |
| 204 | SEQ ID NO : 586 | SEQ ID NO :620, |
| 205 | SEQ ID NO : 585 | SEQ ID NO :621, |
| 206 | SEQ ID NO : 586 | SEQ ID NO :622, |
| 207 | SEQ ID NO : 587 | SEQ ID NO :619, |
| 208 | SEQ ID NO : 588 | SEQ ID NO :620, |
| 209 | SEQ ID NO : 587 | SEQ ID NO :621, |
| 210 | SEQ ID NO : 588 | SEQ ID NO :622, |
| 211 | SEQ ID NO : 589 | SEQ ID NO :623, |
| 212 | SEQ ID NO : 590 | SEQ ID NO :624, |
| 213 | SEQ ID NO : 589 | SEQ ID NO :625, |
| 214 | SEQ ID NO : 590 | SEQ ID NO :626, |
| 215 | SEQ ID NO : 589 | SEQ ID NO :627, |
| 216 | SEQ ID NO : 590 | SEQ ID NO :628, |
| 217 | SEQ ID NO : 591 | SEQ ID NO :623, |
| 218 | SEQ ID NO : 592 | SEQ ID NO :624, |
| 219 | SEQ ID NO : 591 | SEQ ID NO :625, |
| 220 | SEQ ID NO : 592 | SEQ ID NO :626, |
| 221 | SEQ ID NO : 591 | SEQ ID NO :627, |
| 222 | SEQ ID NO : 592 | SEQ ID NO :628, |
| 223 | SEQ ID NO : 593 | SEQ ID NO :623, |
| 224 | SEQ ID NO : 594 | SEQ ID NO :624, |
| 225 | SEQ ID NO : 593 | SEQ ID NO :625, |
| 226 | SEQ ID NO : 594 | SEQ ID NO :626, |
| 227 | SEQ ID NO : 593 | SEQ ID NO :627, |
| 228 | SEQ ID NO : 594 | SEQ ID NO :628, |
| 229 | SEQ ID NO : 595 | SEQ ID NO :629, |
| 230 | SEQ ID NO : 596 | SEQ ID NO :630, |
| 231 | SEQ ID NO : 595 | SEQ ID NO :631, |
| 232 | SEQ ID NO : 596 | SEQ ID NO :632, |
| 233 | SEQ ID NO : 595 | SEQ ID NO :633, |
| 234 | SEQ ID NO : 596 | SEQ ID NO :634, |
| 235 | SEQ ID NO : 597 | SEQ ID NO :629, |
| 236 | SEQ ID NO : 598 | SEQ ID NO :630, |
| 237 | SEQ ID NO : 597 | SEQ ID NO :631, |
| 238 | SEQ ID NO : 598 | SEQ ID NO :632, |
| 239 | SEQ ID NO : 597 | SEQ ID NO :633, |
| 240 | SEQ ID NO : 598 | SEQ ID NO :634, |
| 241 | SEQ ID NO : 599 | SEQ ID NO :629, |
| 242 | SEQ ID NO : 600 | SEQ ID NO :630, |
| 243 | SEQ ID NO : 599 | SEQ ID NO :631, |
| 244 | SEQ ID NO : 600 | SEQ ID NO :632, |
| 245 | SEQ ID NO : 599 | SEQ ID NO :633, |
| 246 | SEQ ID NO : 600 | SEQ ID NO :634, |
| 247 | SEQ ID NO : 601 | SEQ ID NO :629, |
| 248 | SEQ ID NO : 602 | SEQ ID NO :630, |
| 249 | SEQ ID NO : 601 | SEQ ID NO :631, |
| 250 | SEQ ID NO : 602 | SEQ ID NO :632, |
| 251 | SEQ ID NO : 601 | SEQ ID NO :633, |
| 252 | SEQ ID NO : 602 | SEQ ID NO :634, |
| 253 | SEQ ID NO : 603 | SEQ ID NO :629, |
| 254 | SEQ ID NO : 604 | SEQ ID NO :630, |
| 255 | SEQ ID NO : 603 | SEQ ID NO :631, |
| 256 | SEQ ID NO : 604 | SEQ ID NO :632, |
| 257 | SEQ ID NO : 603 | SEQ ID NO :633, |
| 258 | SEQ ID NO : 604 | SEQ ID NO :634, |
| 259 | SEQ ID NO : 605 | SEQ ID NO :629, |
| 260 | SEQ ID NO : 606 | SEQ ID NO :630, |
| 261 | SEQ ID NO : 605 | SEQ ID NO :631, |
| 262 | SEQ ID NO : 606 | SEQ ID NO :632, |
| 263 | SEQ ID NO : 605 | SEQ ID NO :633, |
| 264 | SEQ ID NO : 606 | SEQ ID NO :634, |
| 265 | SEQ ID NO : 607 | SEQ ID NO :635, |
| 266 | SEQ ID NO : 608 | SEQ ID NO :636, |
| 267 | SEQ ID NO : 607 | SEQ ID NO :637, |
| 268 | SEQ ID NO : 608 | SEQ ID NO :638, |
| 269 | SEQ ID NO : 607 | SEQ ID NO :639, |
| 270 | SEQ ID NO : 608 | SEQ ID NO :640, |
| 271 | SEQ ID NO : 609 | SEQ ID NO :635, |
| 272 | SEQ ID NO : 610 | SEQ ID NO :636, |
| 273 | SEQ ID NO : 609 | SEQ ID NO :637, |
| 274 | SEQ ID NO : 610 | SEQ ID NO :638, |
| 275 | SEQ ID NO : 609 | SEQ ID NO :639, |
| 276 | SEQ ID NO : 610 | SEQ ID NO :640, |
| 277 | SEQ ID NO : 611 | SEQ ID NO :635, |
| 278 | SEQ ID NO : 612 | SEQ ID NO :636, |
| 279 | SEQ ID NO : 611 | SEQ ID NO :637, |
| 280 | SEQ ID NO : 612 | SEQ ID NO :638, |
| 281 | SEQ ID NO : 611 | SEQ ID NO :639, |
| 282 | SEQ ID NO : 612 | SEQ ID NO :640, |
| 283 | SEQ ID NO : 613 | SEQ ID NO :635, |
| 284 | SEQ ID NO : 614 | SEQ ID NO :636, |
| 285 | SEQ ID NO : 613 | SEQ ID NO :637, |
| 286 | SEQ ID NO : 614 | SEQ ID NO :638, |
| 287 | SEQ ID NO : 613 | SEQ ID NO :639, |
| 288 | SEQ ID NO : 614 | SEQ ID NO :640, |
| 289 | SEQ ID NO : 615 | SEQ ID NO :635, |
| 290 | SEQ ID NO : 616 | SEQ ID NO :636, |
| 291 | SEQ ID NO : 615 | SEQ ID NO :637, |
| 292 | SEQ ID NO : 616 | SEQ ID NO :638, |
| 293 | SEQ ID NO : 615 | SEQ ID NO :639, |
| 294 | SEQ ID NO : 616 | SEQ ID NO :640, |
| 295 | SEQ ID NO : 617 | SEQ ID NO :635, |
| 296 | SEQ ID NO : 618 | SEQ ID NO :636, |
| 297 | SEQ ID NO : 617 | SEQ ID NO :637, |
| 298 | SEQ ID NO : 618 | SEQ ID NO :638, |
| 299 | SEQ ID NO : 617 | SEQ ID NO :639, |
| 300 | SEQ ID NO : 618 | SEQ ID NO :640, |

Dans un autre aspect, l'invention concerne un ensemble comprenant
- une première molécule d'acides nucléiques simple brin comprenant ou constituée essentiellement d'une séquence A permettant l'insertion d'une séquence complémentaire d'un acide nucléique d'intérêt, ou comprenant une séquence complémentaire d'un acide nucléique d'intérêt, ladite séquence complémentaire étant liée en 5' à une première séquence riches en A/T, notamment riches en T, de 40 à 60 nucléotides de long et en 3' à une deuxième séquence riches en A/T, notamment riches en T, de 40 à 60 nucléotides de long, lesdites première et deuxième séquences riches en A/T, notamment riches en T, comprenant respectivement un premier et un deuxième domaine de 6 à 12 nucléotides riches en G/C, la séquence du premier domaine étant complémentaire de la séquence du deuxième domaine, ledit premier et deuxième domaine étant positionnés de 15 à 52 nucléotides de ladite séquence A, ladite première molécule comprenant en son extrémité 5' au moins une première séquence orientés 5'-3' de reconnaissance d'une transposase et en son extrémité 3' une deuxième séquence de reconnaissance de ladite transposase,
- une deuxième molécule d'acides nucléiques simple brin comprenant ou constituée essentiellement d'une séquence B permettant l'insertion d'une séquence complémentaire d'un acide nucléique d'intérêt, ou comprenant une séquence complémentaire d'un acide nucléique d'intérêt, ladite séquence B complémentaire étant liée en 5' à une troisième séquence riche en T de 40 à 60 nucléotides de long et en 3' à une quatrième séquence riche en T de 40 à 60 nucléotides de long, ladite troisième et quatrième séquence riche en T comprenant respectivement un troisième et un quatrième domaine de 6 à 12 nucléotides riches en G/C, la séquence du troisième domaine étant complémentaire de la séquence du quatrième domaine, ledit troisième et quatrième domaine étant positionnés de 15 à 52 nucléotides de ladite séquence B, ladite deuxième molécule comprenant en son extrémité 5' au moins la première séquence orientés 5'-3' de reconnaissance de ladite transposase et en son extrémité 3' la deuxième séquence de reconnaissance de ladite transposase,
   ladite séquence B étant une séquence complémentaire dudit acide nucléique d'intérêt, la séquence A étant positionnée en 5' d'une région d'intérêt dudit acide nucléique d'intérêt et la séquence B positionnée en 3' de la région d'intérêt dudit acide nucléique d'intérêt, et
- une troisième molécule simple brin comprenant
   dans sa partie 5', au moins une séquence complémentaire de ladite deuxième séquence de reconnaissance de ladite transposase (de la première molécule),
   dans sa partie 3' au moins une séquence complémentaire de ladite première séquence de reconnaissance de ladite transposase (de la deuxième molécule), et
- une région située entre séquence complémentaire de ladite deuxième séquence de reconnaissance de ladite transposase (de la première molécule) et la séquence complémentaire de ladite première séquence de reconnaissance de ladite transposase (de la deuxième molécule) permettant l'insertion d'une molécule d'acide nucléique de remplacement (simple brin),
   les première et troisième molécules d'acides nucléiques simple brin étant appariées selon la complémentarité des bases définie par Watson et Crick de sorte à définir deux sites double brin de liaison de ladite transposase et les deuxième et troisième molécules d'acides nucléiques simple brin étant appariées selon la complémentarité des bases définie par Watson et Crick de sorte à définir deux sites double brin de liaison de ladite transposase.

L'ensemble susmentionné comprend donc le complexe moléculaire décrit ci-dessus. Aussi tous les modes de réalisation et détails techniques décrits ci-dessus pour le complexe moléculaire s'appliquent *mutatis mutandis* à l'ensemble susmentionné.

Dans cet aspect de l'invention il est décrit un ensemble de trois molécules permettant de réaliser un remplacement précis d'une séquence contenue dans un acide nucléique d'intérêt par une séquence choisie.

L'ensemble selon l'invention est basé sur le complexe sus-décrit, celui-ci étant complété d'une troisième molécule similaire à la première molécule. La [Fig.8] illustre schématiquement l'ensemble selon l'invention

La première molécule du complexe correspond à la première molécule de l'ensemble, la deuxième molécule du complexe correspond à la troisième molécule de l'ensemble et la troisième molécule de l'ensemble est ajoutée et est structurellement similaire à la première molécule de l'ensemble ou du complexe.

L'ensemble selon l'invention, lorsque les trois molécules sont correctement appariées, comprend deux paires de sites de liaison double brin de reconnaissance d'une transposase :
- la première paire étant obtenue par l'hybridation de la première molécule avec la troisième molécule, et
- la seconde paire étant obtenue par l'hybridation de la deuxième molécule avec la troisième molécule.

La séquence A contenue dans la première molécule de l'ensemble est complémentaire du même brin de l'acide nucléique qui est complémentaire de la séquence B contenu dans la deuxième molécule. En d'autres termes, la séquence A contenu dans la première molécule et la séquence B contenue dans la deuxième molécule sont capable de s'hybrider au même acide nucléique simultanément, car les deux séquences A et B ne reconnaissent pas la même séquence.

Pour clarifier encore plus le propos, l'intérêt de l'ensemble défini dans la présente invention est de proposer une première molécule et une deuxième molécule, toutes deux telles que définies ci-dessus, les séquences A et B respectives étant telles qu'elles sont capables de reconnaître pour l'une, une séquence localisée en 5' de la séquence cible de l'acide nucléique d'intérêt et pour l'autre, une séquence localisée en 3' de la même séquence cible de l'acide nucléique d'intérêt. Les séquences A et B sont donc complémentaires de régions bordant la séquence d'intérêt, que l'on souhaite remplacer, de la molécule d'acide nucléique d'intérêt.

Les première et deuxièmes molécules d'intérêt sont donc essentielles pour cibler spécifiquement la molécule d'intérêt, afin d'encadrer la séquence à remplacer.

La troisième molécule de l'ensemble, quant à elle, est celle qui apporte la molécule d'acide nucléique qui contient la séquence de remplacement.

D'un point de vue mécanistique, l'ensemble selon l'invention est tel qu'il est constitué de ses trois molécules, la première et la deuxième molécule étant structurellement organisées dans l'espace de sorte que leurs régions riches en G/C soient appariées.

De part et d'autre de la troisième molécule, c'est à dire en 5' et en 3', du faite de l'hybridation avec les première et deuxième molécules, deux paires de site de liaison à une transposase permettent, lorsqu'elle est présente, à des dimères de transposases de lier à l'ensemble.

On notera que les sites de liaison à la transposase de la première molécule peuvent être les mêmes que ceux de la deuxième molécule, ou être différents. Dans le cas où les séquences de liaison sont les mêmes, les dimères de transposases en 5' de la troisième molécule (par hybridation de la partie 5' de la troisième molécule avec la première molécule), et en 3' de la troisième molécule (par hybridation de la partie 3' de la troisième molécule avec la deuxième molécule) seront les mêmes. Aussi, à titre d'exemple, si les sites de liaison sont tous des sites de liaison de la transposase de Tn5, l'ensemble sera associé à 2 dimères de transposases de Tn5.

Il est également possible que les sites de liaison de la première molécule et de la deuxième molécule ne reconnaissent pas la même transposase. Dans ce cas, et selon la définition de l'ensemble donnée ci-dessus, la partie 5' de la troisième molécule formera par hybridation avec la première molécule deux sites double brin de liaison à une première transposase, et partie 3' de la troisième molécule formera par hybridation avec la deuxième molécule deux sites double brin de liaison à une deuxième transposase.

Si maintenant l'ensemble susmentionné, lié à deux dimères de transposase est mis en présence d'une molécule d'acide nucléiques d'intérêt dont la partie 5' est complémentaire de la séquence A de la première molécule de l'ensemble et dont la partie 3' est complémentaire de la séquence B de la deuxième molécule de l'ensemble, alors il y aura appariement entre la molécule d'acide nucléiques d'intérêt et l'ensemble au niveau des régions A et B sus-décrites. Cette interaction aura pour conséquence de rompre l'interaction des deux séquences riches en G/C de chacune des première et deuxième molécules de l'ensemble. Dès lors, la troisième molécule et la molécule d'acide nucléiques d'intérêt seront rapprochée spatialement et les transposases pourront exercer leur activité de tagmentation, dont le résultat sera le remplacement de la séquence de la molécule d'intérêt, bordée par les séquences complémentaires des séquences A et B, par la séquence de la troisième molécule de de l'ensemble, qui se trouve entre les demi-sites de liaison à la /aux transposases en 5' et en 3'.

Dans l'invention, les premières molécules, deuxièmes molécules et troisième molécule de l'ensemble sont avantageusement des molécules constituées de désoxyri-bonucléotides, afin de former des molécules d'ADN simple brin.

De manière encore plus avantageux, les premières molécules, deuxièmes molécules et troisième molécule de l'ensemble sont des molécules hybrides ADN/ARN, où, le « squelette » des molécules est de l'ADN, et les séquences de reconnaissance A et B de la molécule d'acide nucléique d'intérêt, et la région centrale de la troisième molécule sont de l'ARN. Ceci est particulièrement avantageux lors que le remplacement de séquence que permet l'invention doit se faire directement sur une molécule d'ARN.

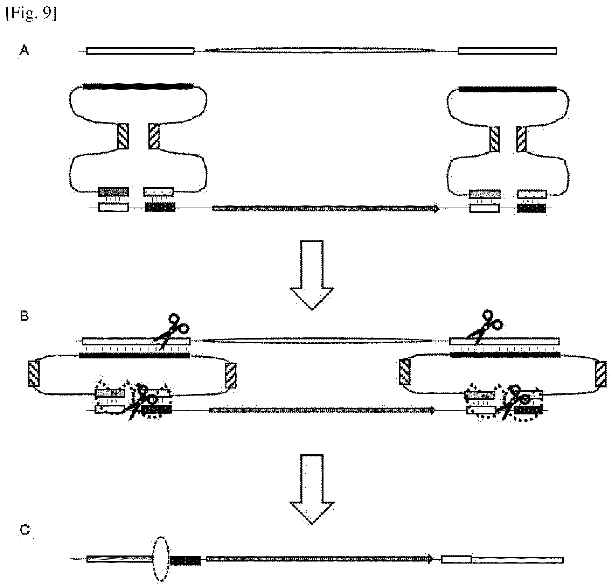

La [Fig.9]-A illustre l'interaction entre la molécule d'intérêt et l'ensemble selon l'invention.

De manière avantageuse, l'invention concerne le complexe susmentionné, où ladite première molécule ou ladite deuxième molécule, ou les deux est/sont couplée(s) à une enzyme, notamment par le biais d'un nucléotide modifié. Cette enzyme a pour but de favoriser le remplacement de la molécule d'intérêt. Il peut s'agir
- d'une hélicase, enzyme capable d'ouvrir une molécule double brin qui serait super-enroulée ou encore associée à des protéines telles que des histones,
- une topoisomérase, enzyme agissant sur la structure topologique de l'ADN en générant des coupures transitoires,
- une ligase qui permet de former une liaison phosphodiester entre une extrémité 5' phosphate d'un nucléotide et l'extrémité 3' OH d'un autre nucléotide, - une polymérase, qui synthétisera une molécule d'acides nucléiques à partir d'un site d'initiation, 3'OH libre, dans le sens 5'-> 3', notamment en recopiant un brin complémentaire antiparallèle selon le modèle de Watson et Crick.

Il est également possible de combiner deux ou plusieurs desdites enzymes pour disposer de tout le matériel enzymatique nécessaire pour permettre le remplacement de séquence prévu dans le cadre de l'invention.

Dans un aspect particulier, de l'invention, la première molécule de l'ensemble peut contenir dans sa partie 5', plus précisément entre le premier site de liaison à la transposase et la région A, un ou plusieurs nucléotides modifiés. De la même manière la troisième molécule de l'ensemble peut contenir dans sa partie 3', plus précisément entre le premier site de liaison à la transposase et la région 3, un ou plusieurs nucléotides modifiés.

Ce nucléotide modifié est notamment modifié par greffage d'une chaine carbonée substituée avec une étiquette protéique, ou *tag* en anglais, ou encore une molécule permettant une interaction spécifique telle que la streptavidine ou la biotine.

De telles modifications permettent alors de lier spécifiquement, à la première molécule de l'ensemble, des enzymes qui peuvent servir à favoriser la tagmentation, et le remplacement de séquence. Il sera particulièrement avantageux d'avoir par exemple un greffage steptatvidine, qui permettra de greffer une hélicase biotinylée (inversement hélicase greffée à la streprepatvidine et nucléotide biotinylé) servant à ouvrir une molécule double brin. Il est également possible d'envisager le greffage avec une ligase biotinylée (ou couplé à la steptavidine), afin de relier le brin recombiné coté 3'.

Il est également possible d'associer à la première molécule ou à la deuxième molécule de l'ensemble un oligonucléotide, de sorte que ledit oligonucléotide s'appariera sur une région prédéterminée de la dite première ou deuxième molécule. Cet oligonucléotide est alors avantageusement couplé à une molécule de greffage comme expliqué ci-dessus.

L'ensemble sus-décrit est approprié pour un remplacement de séquence simple brin, comme par exemple le remplacement d'une séquence sur une molécule d'ADN simple brin ou d'un ARN.

Pour remplacer les deux brins d'une molécule double brin, il sera alors utile d'utiliser deux en ensemble susmentionnés, ces deux ensembles étant tels que
- le premier ensemble comprend la première la deuxième et la troisième molécule telles que définis ci-dessus,
- le deuxième ensemble comprend une quatrième, une cinquième et une sixième séquence,
   de sorte que :
- la troisième et la sixième séquence comprennent chacune un brin de la séquence de remplacement, ces séquences étant complémentaires selon l'appariement de Watson et Crick ;
- la première et la quatrième séquence comprennent, dans la région A et la région A' (la région A' étant l'équivalent de la quatrième molécule de la région A de la première molécule) une séquence reconnaissant la partie 5' de la région à remplacer et la partie 3' du brin complémentaire de la région à remplacer, respectivement, et
- la deuxième et la cinquième séquence comprennent, dans la région B et la région B' (la région B' étant l'équivalent de la cinquième molécule de la région B de la deuxième molécule) une séquence reconnaissant la partie 3' de la région à remplacer et la partie 5' du brin complémentaire de la région à remplacer, respectivement,

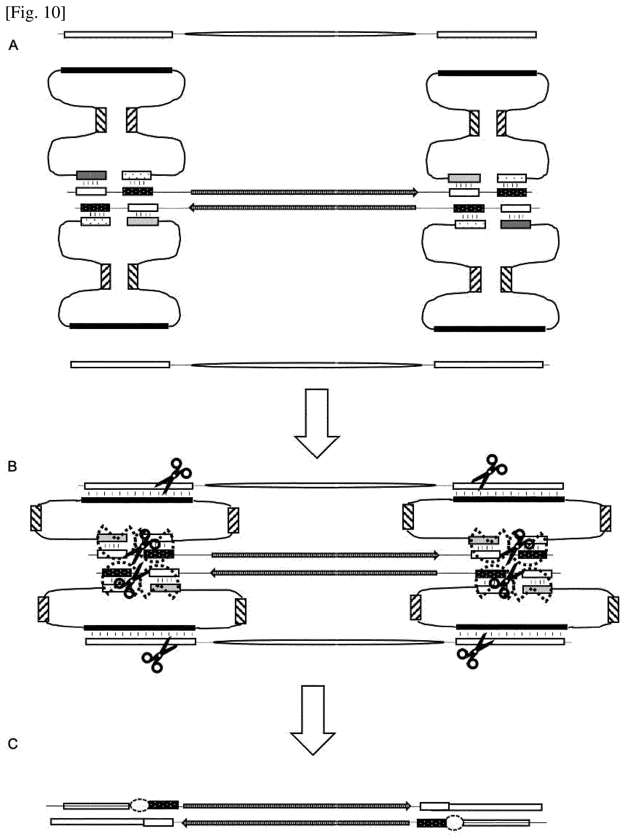

Ce double complexe ou ce double ensemble est alors représenté en [Fig.10]-A.

De manière avantageuse, l'invention concerne l'ensemble susmentionné, ledit ensemble comprenant l'un des 300 couples de première et troisième molécule du tableau 5 suivant :

**[Tableaux5]**

| # | la première molécule comprenant la séquence | la troisième molécule comprenant la séquence |
|---|---|---|
| 1 | SEQ ID NO: 467 | SEQ ID NO :503, |
| 2 | SEQ ID NO : 468 | SEQ ID NO :504, |
| 3 | SEQ ID NO : 469 | SEQ ID NO :503, |
| 4 | SEQ ID NO : 470 | SEQ ID NO :504, |
| 5 | SEQ ID NO : 469 | SEQ ID NO :505, |
| 6 | SEQ ID NO : 470 | SEQ ID NO :506, |
| 7 | SEQ ID NO : 471 | SEQ ID NO :503, |
| 8 | SEQ ID NO : 472 | SEQ ID NO :504, |
| 9 | SEQ ID NO : 471 | SEQ ID NO :505, |
| 10 | SEQ ID NO : 472 | SEQ ID NO :506, |
| 11 | SEQ ID NO : 473 | SEQ ID NO :507, |
| 12 | SEQ ID NO : 474 | SEQ ID NO :508, |
| 13 | SEQ ID NO : 473 | SEQ ID NO :509, |
| 14 | SEQ ID NO : 474 | SEQ ID NO :510, |
| 15 | SEQ ID NO : 473 | SEQ ID NO :511, |
| 16 | SEQ ID NO : 474 | SEQ ID NO :512, |
| 17 | SEQ ID NO : 475 | SEQ ID NO :507, |
| 18 | SEQ ID NO : 476 | SEQ ID NO :508, |
| 19 | SEQ ID NO : 475 | SEQ ID NO :509, |
| 20 | SEQ ID NO : 476 | SEQ ID NO :510, |
| 21 | SEQ ID NO : 475 | SEQ ID NO :511, |
| 22 | SEQ ID NO : 476 | SEQ ID NO :512, |
| 23 | SEQ ID NO : 477 | SEQ ID NO :507, |
| 24 | SEQ ID NO : 478 | SEQ ID NO :508, |
| 25 | SEQ ID NO : 477 | SEQ ID NO :509, |
| 26 | SEQ ID NO : 478 | SEQ ID NO :510, |
| 27 | SEQ ID NO : 477 | SEQ ID NO :511, |
| 28 | SEQ ID NO : 478 | SEQ ID NO :512, |
| 29 | SEQ ID NO : 479 | SEQ ID NO :513, |
| 30 | SEQ ID NO : 480 | SEQ ID NO :514, |
| 31 | SEQ ID NO : 479 | SEQ ID NO :515, |
| 32 | SEQ ID NO : 480 | SEQ ID NO :516, |
| 33 | SEQ ID NO : 479 | SEQ ID NO :517, |
| 34 | SEQ ID NO : 480 | SEQ ID NO :518, |
| 35 | SEQ ID NO : 481 | SEQ ID NO :513, |
| 36 | SEQ ID NO : 482 | SEQ ID NO :514, |
| 37 | SEQ ID NO : 481 | SEQ ID NO :515, |
| 38 | SEQ ID NO : 482 | SEQ ID NO :516, |
| 39 | SEQ ID NO : 481 | SEQ ID NO :517, |
| 40 | SEQ ID NO : 482 | SEQ ID NO :518, |
| 41 | SEQ ID NO : 483 | SEQ ID NO :513, |
| 42 | SEQ ID NO : 484 | SEQ ID NO :514, |
| 43 | SEQ ID NO : 483 | SEQ ID NO :515, |
| 44 | SEQ ID NO : 484 | SEQ ID NO :516, |
| 45 | SEQ ID NO : 483 | SEQ ID NO :517, |
| 46 | SEQ ID NO : 484 | SEQ ID NO :518, |
| 47 | SEQ ID NO : 485 | SEQ ID NO :513, |
| 48 | SEQ ID NO : 486 | SEQ ID NO :514, |
| 49 | SEQ ID NO : 485 | SEQ ID NO :515, |
| 50 | SEQ ID NO : 486 | SEQ ID NO :516, |
| 51 | SEQ ID NO : 485 | SEQ ID NO :517, |
| 52 | SEQ ID NO : 486 | SEQ ID NO :518, |
| 53 | SEQ ID NO : 487 | SEQ ID NO :513, |
| 54 | SEQ ID NO : 488 | SEQ ID NO :514, |
| 55 | SEQ ID NO : 487 | SEQ ID NO :515, |
| 56 | SEQ ID NO : 488 | SEQ ID NO :516, |
| 57 | SEQ ID NO : 487 | SEQ ID NO :517, |
| 58 | SEQ ID NO : 488 | SEQ ID NO :518, |
| 59 | SEQ ID NO : 489 | SEQ ID NO :513, |
| 60 | SEQ ID NO : 490 | SEQ ID NO :514, |
| 61 | SEQ ID NO : 489 | SEQ ID NO :515, |
| 62 | SEQ ID NO : 490 | SEQ ID NO :516, |
| 63 | SEQ ID NO : 489 | SEQ ID NO :517, |
| 64 | SEQ ID NO : 490 | SEQ ID NO :518, |
| 65 | SEQ ID NO : 491 | SEQ ID NO :519, |
| 66 | SEQ ID NO : 492 | SEQ ID NO :520, |
| 67 | SEQ ID NO : 491 | SEQ ID NO :521, |
| 68 | SEQ ID NO : 492 | SEQ ID NO :522, |
| 69 | SEQ ID NO : 491 | SEQ ID NO :523, |
| 70 | SEQ ID NO : 492 | SEQ ID NO :524, |
| 71 | SEQ ID NO : 493 | SEQ ID NO :519, |
| 72 | SEQ ID NO : 494 | SEQ ID NO :520, |
| 73 | SEQ ID NO : 493 | SEQ ID NO :521, |
| 74 | SEQ ID NO : 494 | SEQ ID NO :522, |
| 75 | SEQ ID NO : 493 | SEQ ID NO :523, |
| 76 | SEQ ID NO : 494 | SEQ ID NO :524, |
| 77 | SEQ ID NO : 495 | SEQ ID NO :519, |
| 78 | SEQ ID NO : 496 | SEQ ID NO :520, |
| 79 | SEQ ID NO : 495 | SEQ ID NO :521, |
| 80 | SEQ ID NO : 496 | SEQ ID NO :522, |
| 81 | SEQ ID NO : 495 | SEQ ID NO :523, |
| 82 | SEQ ID NO : 496 | SEQ ID NO :524, |
| 83 | SEQ ID NO : 497 | SEQ ID NO :519, |
| 84 | SEQ ID NO : 498 | SEQ ID NO :520, |
| 85 | SEQ ID NO : 497 | SEQ ID NO :521, |
| 86 | SEQ ID NO : 498 | SEQ ID NO :522, |
| 87 | SEQ ID NO : 497 | SEQ ID NO :523, |
| 88 | SEQ ID NO : 498 | SEQ ID NO :524, |
| 89 | SEQ ID NO : 499 | SEQ ID NO :519, |
| 90 | SEQ ID NO : 500 | SEQ ID NO :520, |
| 91 | SEQ ID NO : 499 | SEQ ID NO :521, |
| 92 | SEQ ID NO : 500 | SEQ ID NO :522, |
| 93 | SEQ ID NO : 499 | SEQ ID NO :523, |
| 94 | SEQ ID NO : 500 | SEQ ID NO :524, |
| 95 | SEQ ID NO : 501 | SEQ ID NO :519, |
| 96 | SEQ ID NO : 502 | SEQ ID NO :520, |
| 97 | SEQ ID NO : 501 | SEQ ID NO :521, |
| 98 | SEQ ID NO : 502 | SEQ ID NO :522, |
| 99 | SEQ ID NO : 501 | SEQ ID NO :523, |
| 100 | SEQ ID NO : 502 | SEQ ID NO :524, |
| 101 | SEQ ID NO : 525 | SEQ ID NO :561, |
| 102 | SEQ ID NO : 526 | SEQ ID NO :562, |
| 103 | SEQ ID NO : 527 | SEQ ID NO :561, |
| 104 | SEQ ID NO : 528 | SEQ ID NO :562, |
| 105 | SEQ ID NO : 527 | SEQ ID NO :563, |
| 106 | SEQ ID NO : 528 | SEQ ID NO :564, |
| 107 | SEQ ID NO : 529 | SEQ ID NO :561, |
| 108 | SEQ ID NO : 530 | SEQ ID NO :562, |
| 109 | SEQ ID NO : 529 | SEQ ID NO :563, |
| 110 | SEQ ID NO : 530 | SEQ ID NO :564, |
| 111 | SEQ ID NO : 531 | SEQ ID NO :565, |
| 112 | SEQ ID NO : 532 | SEQ ID NO :566, |
| 113 | SEQ ID NO : 531 | SEQ ID NO :567, |
| 114 | SEQ ID NO : 532 | SEQ ID NO :568, |
| 115 | SEQ ID NO : 531 | SEQ ID NO :569, |
| 116 | SEQ ID NO : 532 | SEQ ID NO :570, |
| 117 | SEQ ID NO : 533 | SEQ ID NO :565, |
| 118 | SEQ ID NO : 534 | SEQ ID NO :566, |
| 119 | SEQ ID NO : 533 | SEQ ID NO :567, |
| 120 | SEQ ID NO : 534 | SEQ ID NO :568, |
| 121 | SEQ ID NO : 533 | SEQ ID NO :569, |
| 122 | SEQ ID NO : 534 | SEQ ID NO :570, |
| 123 | SEQ ID NO : 535 | SEQ ID NO :565, |
| 124 | SEQ ID NO : 536 | SEQ ID NO :566, |
| 125 | SEQ ID NO : 535 | SEQ ID NO :567, |
| 126 | SEQ ID NO : 536 | SEQ ID NO :568, |
| 127 | SEQ ID NO : 535 | SEQ ID NO :569, |
| 128 | SEQ ID NO : 536 | SEQ ID NO :570, |
| 129 | SEQ ID NO : 537 | SEQ ID NO :571, |
| 130 | SEQ ID NO : 538 | SEQ ID NO :572, |
| 131 | SEQ ID NO : 537 | SEQ ID NO :573, |
| 132 | SEQ ID NO : 538 | SEQ ID NO :574, |
| 133 | SEQ ID NO : 537 | SEQ ID NO :575, |
| 134 | SEQ ID NO : 538 | SEQ ID NO :576, |
| 135 | SEQ ID NO : 539 | SEQ ID NO :571, |
| 136 | SEQ ID NO : 540 | SEQ ID NO :572, |
| 137 | SEQ ID NO : 539 | SEQ ID NO :573, |
| 138 | SEQ ID NO : 540 | SEQ ID NO :574, |
| 139 | SEQ ID NO : 539 | SEQ ID NO :575, |
| 140 | SEQ ID NO : 540 | SEQ ID NO :576, |
| 141 | SEQ ID NO : 541 | SEQ ID NO :571, |
| 142 | SEQ ID NO : 542 | SEQ ID NO :572, |
| 143 | SEQ ID NO : 541 | SEQ ID NO :573, |
| 144 | SEQ ID NO : 542 | SEQ ID NO :574, |
| 145 | SEQ ID NO : 541 | SEQ ID NO :575, |
| 146 | SEQ ID NO : 542 | SEQ ID NO :576, |
| 147 | SEQ ID NO : 543 | SEQ ID NO :571, |
| 148 | SEQ ID NO : 544 | SEQ ID NO :572, |
| 149 | SEQ ID NO : 543 | SEQ ID NO :573, |
| 150 | SEQ ID NO : 544 | SEQ ID NO :574, |
| 151 | SEQ ID NO : 543 | SEQ ID NO :575, |
| 152 | SEQ ID NO : 544 | SEQ ID NO :576, |
| 153 | SEQ ID NO : 545 | SEQ ID NO :571, |
| 154 | SEQ ID NO : 546 | SEQ ID NO :572, |
| 155 | SEQ ID NO : 545 | SEQ ID NO :573, |
| 156 | SEQ ID NO : 546 | SEQ ID NO :574, |
| 157 | SEQ ID NO : 545 | SEQ ID NO :575, |
| 158 | SEQ ID NO : 546 | SEQ ID NO :576, |
| 159 | SEQ ID NO : 547 | SEQ ID NO :571, |
| 160 | SEQ ID NO : 548 | SEQ ID NO :572, |
| 161 | SEQ ID NO : 547 | SEQ ID NO :573, |
| 162 | SEQ ID NO : 548 | SEQ ID NO :574, |
| 163 | SEQ ID NO : 547 | SEQ ID NO :575, |
| 164 | SEQ ID NO : 548 | SEQ ID NO :576, |
| 165 | SEQ ID NO : 549 | SEQ ID NO :577, |
| 166 | SEQ ID NO : 550 | SEQ ID NO :578, |
| 167 | SEQ ID NO : 549 | SEQ ID NO :579, |
| 168 | SEQ ID NO : 550 | SEQ ID NO :580, |
| 169 | SEQ ID NO : 549 | SEQ ID NO :581, |
| 170 | SEQ ID NO : 550 | SEQ ID NO :582, |
| 171 | SEQ ID NO : 551 | SEQ ID NO :577, |
| 172 | SEQ ID NO : 552 | SEQ ID NO :578, |
| 173 | SEQ ID NO : 551 | SEQ ID NO :579, |
| 174 | SEQ ID NO : 552 | SEQ ID NO :580, |
| 175 | SEQ ID NO : 551 | SEQ ID NO :581, |
| 176 | SEQ ID NO : 552 | SEQ ID NO :582, |
| 177 | SEQ ID NO : 553 | SEQ ID NO :577, |
| 178 | SEQ ID NO : 554 | SEQ ID NO :578, |
| 179 | SEQ ID NO : 553 | SEQ ID NO :579, |
| 180 | SEQ ID NO : 554 | SEQ ID NO :580, |
| 181 | SEQ ID NO : 553 | SEQ ID NO :581, |
| 182 | SEQ ID NO : 554 | SEQ ID NO :582, |
| 183 | SEQ ID NO : 555 | SEQ ID NO :577, |
| 184 | SEQ ID NO : 556 | SEQ ID NO :578, |
| 185 | SEQ ID NO : 555 | SEQ ID NO :579, |
| 186 | SEQ ID NO : 556 | SEQ ID NO :580, |
| 187 | SEQ ID NO : 555 | SEQ ID NO :581, |
| 188 | SEQ ID NO : 556 | SEQ ID NO :582, |
| 189 | SEQ ID NO : 557 | SEQ ID NO :577, |
| 190 | SEQ ID NO : 558 | SEQ ID NO :578, |
| 191 | SEQ ID NO : 557 | SEQ ID NO :579, |
| 192 | SEQ ID NO : 558 | SEQ ID NO :580, |
| 193 | SEQ ID NO : 557 | SEQ ID NO :581, |
| 194 | SEQ ID NO : 558 | SEQ ID NO :582, |
| 195 | SEQ ID NO : 559 | SEQ ID NO :577, |
| 196 | SEQ ID NO : 560 | SEQ ID NO :578, |
| 197 | SEQ ID NO : 559 | SEQ ID NO :579, |
| 198 | SEQ ID NO : 560 | SEQ ID NO :580, |
| 199 | SEQ ID NO : 559 | SEQ ID NO :581, |
| 200 | SEQ ID NO : 560 | SEQ ID NO :582, |
| 201 | SEQ ID NO : 583 | SEQ ID NO :619, |
| 202 | SEQ ID NO : 584 | SEQ ID NO :620, |
| 203 | SEQ ID NO : 585 | SEQ ID NO :619, |
| 204 | SEQ ID NO : 586 | SEQ ID NO :620, |
| 205 | SEQ ID NO : 585 | SEQ ID NO :621, |
| 206 | SEQ ID NO : 586 | SEQ ID NO :622, |
| 207 | SEQ ID NO : 587 | SEQ ID NO :619, |
| 208 | SEQ ID NO : 588 | SEQ ID NO :620, |
| 209 | SEQ ID NO : 587 | SEQ ID NO :621, |
| 210 | SEQ ID NO : 588 | SEQ ID NO :622, |
| 211 | SEQ ID NO : 589 | SEQ ID NO :623, |
| 212 | SEQ ID NO : 590 | SEQ ID NO :624, |
| 213 | SEQ ID NO : 589 | SEQ ID NO :625, |
| 214 | SEQ ID NO : 590 | SEQ ID NO :626, |
| 215 | SEQ ID NO : 589 | SEQ ID NO :627, |
| 216 | SEQ ID NO : 590 | SEQ ID NO :628, |
| 217 | SEQ ID NO : 591 | SEQ ID NO :623, |
| 218 | SEQ ID NO : 592 | SEQ ID NO :624, |
| 219 | SEQ ID NO : 591 | SEQ ID NO :625, |
| 220 | SEQ ID NO : 592 | SEQ ID NO :626, |
| 221 | SEQ ID NO : 591 | SEQ ID NO :627, |
| 222 | SEQ ID NO : 592 | SEQ ID NO :628, |
| 223 | SEQ ID NO : 593 | SEQ ID NO :623, |
| 224 | SEQ ID NO : 594 | SEQ ID NO :624, |
| 225 | SEQ ID NO : 593 | SEQ ID NO :625, |
| 226 | SEQ ID NO : 594 | SEQ ID NO :626, |
| 227 | SEQ ID NO : 593 | SEQ ID NO :627, |
| 228 | SEQ ID NO : 594 | SEQ ID NO :628, |
| 229 | SEQ ID NO : 595 | SEQ ID NO :629, |
| 230 | SEQ ID NO : 596 | SEQ ID NO :630, |
| 231 | SEQ ID NO : 595 | SEQ ID NO :631, |
| 232 | SEQ ID NO : 596 | SEQ ID NO :632, |
| 233 | SEQ ID NO : 595 | SEQ ID NO :633, |
| 234 | SEQ ID NO : 596 | SEQ ID NO :634, |
| 235 | SEQ ID NO : 597 | SEQ ID NO :629, |
| 236 | SEQ ID NO : 598 | SEQ ID NO :630, |
| 237 | SEQ ID NO : 597 | SEQ ID NO :631, |
| 238 | SEQ ID NO : 598 | SEQ ID NO :632, |
| 239 | SEQ ID NO : 597 | SEQ ID NO :633, |
| 240 | SEQ ID NO : 598 | SEQ ID NO :634, |
| 241 | SEQ ID NO : 599 | SEQ ID NO :629, |
| 242 | SEQ ID NO : 600 | SEQ ID NO :630, |
| 243 | SEQ ID NO : 599 | SEQ ID NO :631, |
| 244 | SEQ ID NO : 600 | SEQ ID NO :632, |
| 245 | SEQ ID NO : 599 | SEQ ID NO :633, |
| 246 | SEQ ID NO : 600 | SEQ ID NO :634, |
| 247 | SEQ ID NO : 601 | SEQ ID NO :629, |
| 248 | SEQ ID NO : 602 | SEQ ID NO :630, |
| 249 | SEQ ID NO : 601 | SEQ ID NO :631, |
| 250 | SEQ ID NO : 602 | SEQ ID NO :632, |
| 251 | SEQ ID NO : 601 | SEQ ID NO :633, |
| 252 | SEQ ID NO : 602 | SEQ ID NO :634, |
| 253 | SEQ ID NO : 603 | SEQ ID NO :629, |
| 254 | SEQ ID NO : 604 | SEQ ID NO :630, |
| 255 | SEQ ID NO : 603 | SEQ ID NO :631, |
| 256 | SEQ ID NO : 604 | SEQ ID NO :632, |
| 257 | SEQ ID NO : 603 | SEQ ID NO :633, |
| 258 | SEQ ID NO : 604 | SEQ ID NO :634, |
| 259 | SEQ ID NO : 605 | SEQ ID NO :629, |
| 260 | SEQ ID NO : 606 | SEQ ID NO :630, |
| 261 | SEQ ID NO : 605 | SEQ ID NO :631, |
| 262 | SEQ ID NO : 606 | SEQ ID NO :632, |
| 263 | SEQ ID NO : 605 | SEQ ID NO :633, |
| 264 | SEQ ID NO : 606 | SEQ ID NO :634, |
| 265 | SEQ ID NO : 607 | SEQ ID NO :635, |
| 266 | SEQ ID NO : 608 | SEQ ID NO :636, |
| 267 | SEQ ID NO : 607 | SEQ ID NO :637, |
| 268 | SEQ ID NO : 608 | SEQ ID NO :638, |
| 269 | SEQ ID NO : 607 | SEQ ID NO :639, |
| 270 | SEQ ID NO : 608 | SEQ ID NO :640, |
| 271 | SEQ ID NO : 609 | SEQ ID NO :635, |
| 272 | SEQ ID NO : 610 | SEQ ID NO :636, |
| 273 | SEQ ID NO : 609 | SEQ ID NO :637, |
| 274 | SEQ ID NO : 610 | SEQ ID NO :638, |
| 275 | SEQ ID NO : 609 | SEQ ID NO :639, |
| 276 | SEQ ID NO : 610 | SEQ ID NO :640, |
| 277 | SEQ ID NO : 611 | SEQ ID NO :635, |
| 278 | SEQ ID NO : 612 | SEQ ID NO :636, |
| 279 | SEQ ID NO : 611 | SEQ ID NO :637, |
| 280 | SEQ ID NO : 612 | SEQ ID NO :638, |
| 281 | SEQ ID NO : 611 | SEQ ID NO :639, |
| 282 | SEQ ID NO : 612 | SEQ ID NO :640, |
| 283 | SEQ ID NO : 613 | SEQ ID NO :635, |
| 284 | SEQ ID NO : 614 | SEQ ID NO :636, |
| 285 | SEQ ID NO : 613 | SEQ ID NO :637, |
| 286 | SEQ ID NO : 614 | SEQ ID NO :638, |
| 287 | SEQ ID NO : 613 | SEQ ID NO :639, |
| 288 | SEQ ID NO : 614 | SEQ ID NO :640, |
| 289 | SEQ ID NO : 615 | SEQ ID NO :635, |
| 290 | SEQ ID NO : 616 | SEQ ID NO :636, |
| 291 | SEQ ID NO : 615 | SEQ ID NO :637, |
| 292 | SEQ ID NO : 616 | SEQ ID NO :638, |
| 293 | SEQ ID NO : 615 | SEQ ID NO :639, |
| 294 | SEQ ID NO : 616 | SEQ ID NO :640, |
| 295 | SEQ ID NO : 617 | SEQ ID NO :635, |
| 296 | SEQ ID NO : 618 | SEQ ID NO :636, |
| 297 | SEQ ID NO : 617 | SEQ ID NO :637, |
| 298 | SEQ ID NO : 618 | SEQ ID NO :638, |
| 299 | SEQ ID NO : 617 | SEQ ID NO :639, |
| 300 | SEQ ID NO : 618 | SEQ ID NO :640, |

Dans encore un autre aspect, l'invention concerne un kit, ou une trousse, comprenant au moins un vecteur permettant l'expression d'une recombinase et les première, deuxième et troisième molécules de l'ensemble tel que défini ci-dessus.

Le kit est composé soit d'un contenant comprenant les 3 molécules de l'ensemble, ou plusieurs contenant séparés.

La transposase contenu dans le kit susmentionné est une transposase capable de reconnaître les sites de liaison formés par l'interaction entre la première molécule et la troisième molécule du complexe, et/ou la deuxième molécule et la troisième molécule du complexe.

Dans l'hypothèse ou le complexe est tel qu'il contient deux sites de liaison pour deux transposases différentes, le kit comprendra alors deux vecteurs différents chacun codant une des transposases.

Le kit peut également contenu en outre, comme discuté plus haut, d'autres enzymes, comme par exemple une hélicase, ou une ligase.

Le kit selon l'invention avantageusement comprend de quoi former deux ensembles tels que défini ci-dessus.

L'invention concerne également l'utilisation de l'ensemble tel que défini ci-dessus, pour l'ingénierie des acides nucléiques, notamment pour la substitution d'une séquence cible par une séquence d'intérêt. L'utilisation susmentionnée est notamment sous réserve qu'elle ne comprenne pas un procédé pour la modification de l'identité génétique germinale d'êtres humains et/ou que ladite utilisation ne soit pas une méthode pour le traitement du corps humain ou animal par une chirurgie ou une thérapie.

Comme expliqué ci-dessus l'ensemble susmentionné permet de cibler spécifiquement une région cible et de la remplacer par une séquence d'intérêt, en utilisant les propriétés de tagmentation des transposases.

L'ensemble selon l'invention est notamment avantageux dans le pour réaliser des modifications géniques ciblées (et en particulier des remplacements de gène, ou de séquences non codantes) chez différents organismes vivants, par exemple chez la plante pour obtenir des plantes transgéniques, ou chez l'animal pour réaliser des modèles de maladies humaines qui ne font pas partie de l'invention, ou des outils thérapeutiques vivants qui ne font pas partie de l'invention, permettant de tester des médicaments.

En outre l'invention concerne l'ensemble susmentionné, pour son utilisation en tant que médicament, plus particulièrement en tant que médicament de thérapie génique, notamment pour traiter ou prévenir une maladie liée à une modification d'un acide nucléique

Puisque l'ensemble susmentionné permet la substitution de séquence de manière spécifique, il est possible de concevoir des premières et deuxièmes molécules reconnaissant spécifiquement un gène muté, par une modification telle qu'une substitution, une délétion ou une insertion, et de concevoir une troisième molécule comprenant la séquence sauvage de référence dudit gène muté.

Aussi, en présence de la transposase appropriée, il sera possible de remplacer la séquence du gène muté par la séquence sauvage, et ainsi traiter un individu atteint d'une maladie, dont la cause est la mutation dudit gène.

La divulgation concerne également l'utilisation du complexe susmentionné, pour la fabrication d'un médicament pour traiter ou prévenir une maladie liée à une modification d'un acide nucléique.

Dans certains aspects ces médicaments ne sont pas destinés à modifier l'identité germinale des êtres humains, et ne sont pas non plus destinés à créer de la souffrance injustifiée chez les animaux.

L'invention concerne par ailleurs une méthode, notamment in vitro, de remplacement d'une région cible d'une molécule d'acide nucléique par une région d'intérêt d'une autre molécule d'acide nucléique, de sorte à obtenir une molécule d'acide nucléique hybride, ladite méthode comprenant :
- la mise en contact d'un ensemble tel que défini ci-dessus, avec l'acide nucléique comprenant la région cible,
   ledit ensemble étant tel que la séquence A de ladite première molécule comprend une séquence complémentaire de la région immédiatement en 5' de la région cible,
   la séquence B de ladite première molécule comprend une séquence complémentaire de la région immédiatement en 3' de la région cible, et
   la troisième molécule comprend ladite région d'intérêt dans la région située entre séquence complémentaire de ladite deuxième séquence de reconnaissance de ladite transposase de la première molécule et la séquence complémentaire de ladite première séquence de reconnaissance de ladite transposase de la deuxième molécule, afin d'obtenir un complexe de remplacement,
- la mise en présence du complexe de remplacement avec une transposase reconnaissant les sites double brin de liaison de ladite transposase contenus dans ledit ensemble, pour obtenir un complexe de recombinaison, et
- la recombinaison du complexe de combinaison pour obtenir la molécule d'acide nucléique hybride comprenant la région d'intérêt à la place de la région cible.

Il est à noter que lors du remplacement, c'est à dire la tagmentation par la transposase, l'extrémité 3' du fragment de remplacement sera lié par liaison phosphodiester à l'extrémité 5' de la séquence qui suit immédiatement la séquence qui a été remplacée. Cette ligation est opérée lors de la tagmentation.

A l'inverse, l'extrémité 3' de la séquence localisée juste avant la séquence remplacée et l'extrémité 5' du brin de remplacement ne seront pas liées entre elles. Aussi, pour finaliser le remplacement, il sera nécessaire, à l'aide d'une ligase, de relier ces deux extrémités.

Il est à noter par ailleurs qu'au niveau du site de remplacement la molécule de remplacement est bordée en 3' et en 5' par une séquence de reconnaissance de la transposase.

Le mécanisme de remplacement pour une cible simple brin est représenté à la [Fig.9].

Dans un premier temps, l'ensemble constitué e la première, de la deuxième et de la troisième molécule, comprenant respectivement la région complémentaire de la partie 5' de la séquence cible, la respectivement la région complémentaire de la partie 3' de la séquence cible, et la séquence d'intérêt, sont organisées dans l'espace de sorte que les régions complémentaires riches en G/C de la première molécule sont appariées entre elles et celles de la deuxième molécule sont appariées entre elles ([Fig.9]-A).

En présence de la séquence cible, les régions complémentaires de la partie 5' et de la partie 3' de la séquence cible, contenues respectivement dans les première et deuxième molécules vont s'apparier avec leur région complémentaire respective de sorte à former un complexe quatrimoléculaire contenant la molécule cible, et les trois molécules de l'ensemble. L'interaction (ou l'appariement) entre les premières et deuxièmes molécules et leurs cibles a pour effet de rompre l'interaction des régions riches en G/C, de sorte que les sites de liaison à la transposase, sur lesquelles sont positionnés les dimères de transposases se retrouvent spatialement proche de la molécule cible. Les transposases vont alors pouvoir exercer leur activité et couper à la fois la molécule cible et la molécule d'intérêt (entre les deux sites de liaison) comme le montre la [Fig.9]**-B**.

Dès lors la tagmentation va s'opérer de sorte que
- l'extrémité 3' de la troisième molécule va être liée à l'extrémité 5' libre générée par la transposase dans la région en 3' de la séquence cible. Cette jonction sera covalente par ligation des deux extrémités,
- l'extrémité 5' de la troisième molécule va être mise en regard de l'extrémité 3' libre générée par la transposase dans la région en 5' de la séquence cible. Une délétion de 9 paires de bases est générée par la transposase région en 5' de la séquence cible (représentée par le cercle en pointillés sur la [Fig.9]**-C**). Afin de recouvrer une molécule entière avec une la séquence d'intérêt située entre les régions en 5' et en 3' de la séquence cible initiale, une ligation sera nécessaire.

La molécule finale, issue de la tagmentation, sera constituée, dans le sens 5'->3' d'une partie de la région 5' de la séquence cible initiale, en partie délétée de 9 paires de bases, suivie de la deuxième séquence de liaison à la transposase, suivi de la séquence d'intérêt elle-même suivie de la première séquence de liaison à la transposase et enfin une partie de la région 5' de la séquence cible initiale.

Dans encore un autre aspect, l'invention concerne une méthode, notamment in vitro ou ex vivo, d'édition du génome d'une cellule, permettant de remplacer un fragment spécifique de l'ADN double brin dudit génome de ladite cellule par un autre fragment d'ADN double brin d'intérêt, pour obtenir un génome hybride recombiné comprenant l'autre fragment d'ADN double brin d'intérêt à la place du fragment spécifique de l'ADN double brin, ladite méthode comprenant :
- la préparation d'un premier ensemble tel que défini ci-dessus,
   où la séquence A de la première molécule comprend une séquence complémentaire de la région adjacente en 5' du fragment spécifique ;
   où la séquence B de la deuxième molécule comprend une séquence complémentaire de la région adjacente en 3' du fragment spécifique ; et
   où la troisième molécule comprend, entre la région située entre séquence complémentaire de ladite deuxième séquence de reconnaissance de ladite transposase de la première molécule et la séquence complémentaire de ladite première séquence de reconnaissance de ladite transposase de la deuxième molécule, une séquence d'un des brins dudit fragment spécifique ;
- et éventuellement la préparation d'un deuxième ensemble tel que défini ci-dessus,
   où la séquence A de ladite région complémentaire de la première molécule du deuxième ensemble comprend une séquence complémentaire de la région adjacente en 5' du fragment spécifique ;
   où la séquence B de ladite région complémentaire de la deuxième molécule du deuxième ensemble comprend une séquence complémentaire de la région adjacente en 3' du fragment spécifique ; et
   où la troisième molécule du deuxième ensemble comprend, entre la région située entre séquence complémentaire de ladite deuxième séquence de reconnaissance de ladite transposase de la première molécule du deuxième ensemble et la séquence complémentaire de ladite première séquence de reconnaissance de ladite transposase de la deuxième molécule du deuxième ensemble, la séquence du brin complémentaire dudit fragment spécifique contenu dans le troisième séquence du premier ensemble ;
   où la séquence complémentaire de la région adjacente en 5' du fragment spécifique contenue dans la région A de la première molécule du premier ensemble est au plus complémentaire à 95% de la séquence complémentaire de la région adjacente en 5' du fragment spécifique contenue dans la région A de la première molécule du deuxième ensemble ; et
   où la séquence complémentaire de la région adjacente en 3' du fragment spécifique contenue dans la région B de la première molécule du premier ensemble est au plus complémentaire à 95% de la séquence complémentaire de la région adjacente en 3' du fragment spécifique contenue dans la région B de la première molécule du deuxième ensemble ;
   pour obtenir un complexe de recombinaison ;
- la mise en contact de ladite cellule avec ledit complexe de recombinaison, pour obtenir une cellule prête à être éditée,
- l'expression dans ladite cellule prête à être éditée de ladite transposase, pour obtenir une cellule éditée,
- la sélection de la cellule éditée, où le génome de ladite cellule éditée comprend à la place du fragment spécifique de l'ADN double brin l'autre fragment d'ADN double brin d'intérêt,
   sous réserve que ladite méthode ne soit pas un procédé pour la modification de l'identité génétique germinale d'êtres humains et que ladite méthode ne soit pas une méthode pour le traitement du corps humain ou animal par une chirurgie ou une thérapie.

De manière avantageuse, l'invention concerne une méthode d'édition du génome d'une cellule, permettant de remplacer un fragment spécifique de l'ADN double brin dudit génome de ladite cellule par un autre fragment d'ADN double brin d'intérêt, pour obtenir un génome hybride recombiné comprenant l'autre fragment d'ADN double brin d'intérêt à la place du fragment spécifique de l'ADN double brin, ladite méthode comprenant :
- la préparation d'un premier ensemble tel que défini ci-dessus,
   où la séquence A de la première molécule comprend une séquence complémentaire de la région adjacente en 5' du fragment spécifique ;
   où la séquence B de la deuxième molécule comprend une séquence complémentaire de la région adjacente en 3' du fragment spécifique ; et
   où la troisième molécule comprend, entre la région située entre séquence complémentaire de ladite deuxième séquence de reconnaissance de ladite transposase de la première molécule et la séquence complémentaire de ladite première séquence de reconnaissance de ladite transposase de la deuxième molécule, une séquence d'un des brins dudit fragment spécifique ;
- et la préparation d'un deuxième ensemble tel que défini ci-dessus,
   où la séquence A de ladite région complémentaire de la première molécule du deuxième ensemble comprend une séquence complémentaire de la région adjacente en 5' du fragment spécifique ;
   où la séquence B de ladite région complémentaire de la deuxième molécule du deuxième ensemble comprend une séquence complémentaire de la région adjacente en 3' du fragment spécifique ; et
   où la troisième molécule du deuxième ensemble comprend, entre la région située entre séquence complémentaire de ladite deuxième séquence de reconnaissance de ladite transposase de la première molécule du deuxième ensemble et la séquence complémentaire de ladite première séquence de reconnaissance de ladite transposase de la deuxième molécule du deuxième ensemble, la séquence du brin complémentaire dudit fragment spécifique contenu dans le troisième séquence du premier ensemble ;
   où la séquence complémentaire de la région adjacente en 5' du fragment spécifique contenue dans la région A de la première molécule du premier ensemble est au plus complémentaire à 95% de la séquence complémentaire de la région adjacente en 5' du fragment spécifique contenue dans la région A de la première molécule du deuxième ensemble ; et
   où la séquence complémentaire de la région adjacente en 3' du fragment spécifique contenue dans la région B de la première molécule du premier ensemble est au plus complémentaire à 95% de la séquence complémentaire de la région adjacente en 3' du fragment spécifique contenue dans la région B de la première molécule du deuxième ensemble ;
   pour obtenir un complexe de recombinaison ;
- la mise en contact de ladite cellule avec ledit complexe de recombinaison, pour obtenir une cellule prête à être éditée,
- l'expression dans ladite cellule prête à être éditée de ladite transposase, pour obtenir une cellule éditée,
- la sélection de la cellule éditée, où le génome de ladite cellule éditée comprend à la place du fragment spécifique de l'ADN double brin l'autre fragment d'ADN double brin d'intérêt,
   sous réserve que ladite méthode ne soit pas un procédé pour la modification de l'identité génétique germinale d'êtres humains et que ladite méthode ne soit pas une méthode pour le traitement du corps humain ou animal par une chirurgie ou une thérapie.

Dans la mesure où cette méthode d'édition du génome repose sur la méthode de replacement d'une molécule simple ci-dessus décrite, et utilisant un ensemble sus-décrit, toutes les caractéristiques détaillées précédemment et toutes les variantes s'appliquent ici *mutatis mutandis.*

Dans cet aspect de l'invention, il est à noter que la première molécule de l'ensemble considéré (premier ou deuxième) est toujours positionnée en 5' de la troisième molécule dudit ensemble considéré.

Aussi, la première molécule du premier ensemble se retrouvera « au-dessus » de la deuxième molécule du deuxième ensemble, et réciproquement. Aussi, les orientation 5'->3' susmentionnées permettent de positionner correctement la séquence d'intérêt par rapport à la séquence cible.

L'édition du génome (de l'anglais *genome editing*) consiste à modifier le génome d'une cellule avec une grande précision. Il est possible d'inactiver un gène, d'introduire une mutation ciblée, de corriger une mutation particulière ou d'insérer un nouveau gène. Cette technique de génie génétique fait appel à des nucléases, ici la transposase, capable de couper des acides nucléiques au niveau des liaisons phospho-diesters.

Dans le cadre de l'invention, et comme expliqué précédemment, les première et deuxième molécules de l'ensemble selon l'invention permettent de positionner l'ensemble au niveau de la région cible de sorte à encadrer la région cible pour qu'elle soit remplacée par la séquence d'intérêt contenue dans la troisième séquence.

Dans le cadre de l'édition d'une molécule double brin, il est nécessaire de disposer, pour remplacer simultanément les deux brins de la molécule cible de disposer de deux ensembles selon l'invention, chaque ensemble ciblant spécifiquement l'un des deux brins de la molécule cible.

Il est à noter que la séquence complémentaire contenu dans la séquence A de la première molécule du premier ensemble n'est complémentaire de la séquence complémentaire contenu dans la séquence A de la première molécule du deuxième ensemble. De même, la séquence B de la deuxième molécule du premier ensemble n'est complémentaire de la séquence complémentaire contenu dans la séquence B de la deuxième molécule du deuxième ensemble.

En outre, il est avantageux que la séquence complémentaire contenue dans la séquence A de la première molécule du premier ensemble soit décalée par rapport à la séquence complémentaire contenue dans la séquence B de la deuxième molécule du premier ensemble, ces deux molécules étant l'une « au-dessus » de l'autre. Cela signifie que la région de reconnaissance de ces séquences complémentaires est au maximum de 95%. Autrement dit, si la séquence complémentaire contenue dans ladite séquence A et ladite séquence B fait vingt nucléotides, les séquences complémentaires ne seront complémentaires entre elles qu'au plus de 19 nucléotides.

Il est toutefois préférable que ces régions complémentaires soient le moins possible complémentaires entre elles, voire pas du tout complémentaires entre elles.

Afin de bien illustrer ce propos, si l'on considère la partie 5' de la séquence cible comme comprenant 40 nucléotides, alors il serait approprié que la région complémentaire contenue dans la séquence A de la première molécule du premier ensemble soit complémentaire des 20 premiers nucléotides, alors que la région complémentaire contenue dans la séquence B de la deuxième molécule du deuxième ensemble soit complémentaire des 20 derniers nucléotides de la séquence complémentaire la partie 5' de la séquence cible.

Ce décalage, même si les orientations de séquences ne le permettent pas, évitent toute mauvaise orientation de la séquence d'intérêt.

La séquence des étapes de tagmentation et la résultante sont décrites en [Fig.10].

Afin d'obtenir la molécule finale, et du fait de la délétion de 9 paires de base en 5' suite à la tagmentation, il sera nécessaire que la cellule mobilise le système de réparation, afin de combler le trou, notamment en utilisant une ADN polymérase qui recopiera le brin complémentaire dont l'extrémité 3' a été liée lors de la tagmentation.

### Brève description des figures

L'invention sera mieux comprise à la lecture des exemples et des figures suivantes :
[Fig.1] la [Fig.1] est une représentation schématique de la première molécule d'acide nucléique sous forme linéaire. Les rectangles avec des tête de flèche représentent des sites de liaison à une transposase, et les rectangles avec des barres obliques représentent les régions riches en G/C.
[Fig.2] la [Fig.2] est une représentation schématique de la première molécule d'acide nucléique sous forme structurée. Les légendes sont les mêmes que pour la [Fig.1].
[Fig.3] la [Fig.3] est une représentation schématique du complexe selon l'invention. Les légendes sont les mêmes que pour la [Fig.1].
[Fig.4] la [Fig.4] est une représentation schématique de deux versions du complexe selon l'invention. Les légendes sont les mêmes que pour la [Fig.1]. En pointillés sont représentés les différentes options.
[Fig.5] la [Fig.5] est une représentation schématique d'un premier mode de réalisation du complexe selon l'invention. Les légendes sont les mêmes que pour la [Fig.1].
[Fig.6] la [Fig.6] est une représentation schématique d'un deuxième (6A) et d'un troisième (6B) mode de réalisation du complexe selon l'invention, ou la région 3' de la première séquence comprend deux demi sites de la transposase. Les légendes sont les mêmes que pour la [Fig.1].
[Fig.7] la [Fig.7] est une représentation schématique d'un troisième mode de réalisation du complexe selon l'invention. Les légendes sont les mêmes que pour la [Fig.1].
[Fig.8] la [Fig.8] est une représentation schématique d'un ensemble selon l'invention. Les légendes sont les mêmes que pour la [Fig.1].
[Fig.9] la [Fig.9] est une représentation schématique de la séquence de certaines étapes de remplacement d'une séquence cible d'une molécule simple brin par une séquence d'intérêt.
   A : représente la molécule cible et l'ensemble non appariés.
   B : représente la molécule cible et l'ensemble appariés. Les transposases sont représentées en pointillés, et les coupures sont représentées par des ciseaux. Notons que la coupure sur la troisième molécule de l'ensemble se fait entre les deux sites de liaison à la transposase, de part et d'autre de la molécule (deux coupures).
   C : représente la molécule résultante de la tagmentation. En pointillés est représentée la délétion de 9 bases en 5' de la région remplacée.
[Fig.10] la [Fig.10] est une représentation schématique de la séquence de certaines étapes de remplacement d'une séquence cible d'une molécule double brin par une séquence d'intérêt, elle-même double brin.
   A : représente la molécule cible et l'ensemble non appariés.
   B : représente la molécule cible et l'ensemble appariés. Les transposases sont représentées en pointillés, et les coupures sont représentées par des ciseaux. Notons que la coupure sur la troisième molécule de l'ensemble se fait entre les deux sites de liaison à la transposase, de part et d'autre de la molécule (deux coupures).
   C : représente la molécule résultante de la tagmentation. En pointillés est représentée la délétion de 9 bases en 5' de la région remplacée.

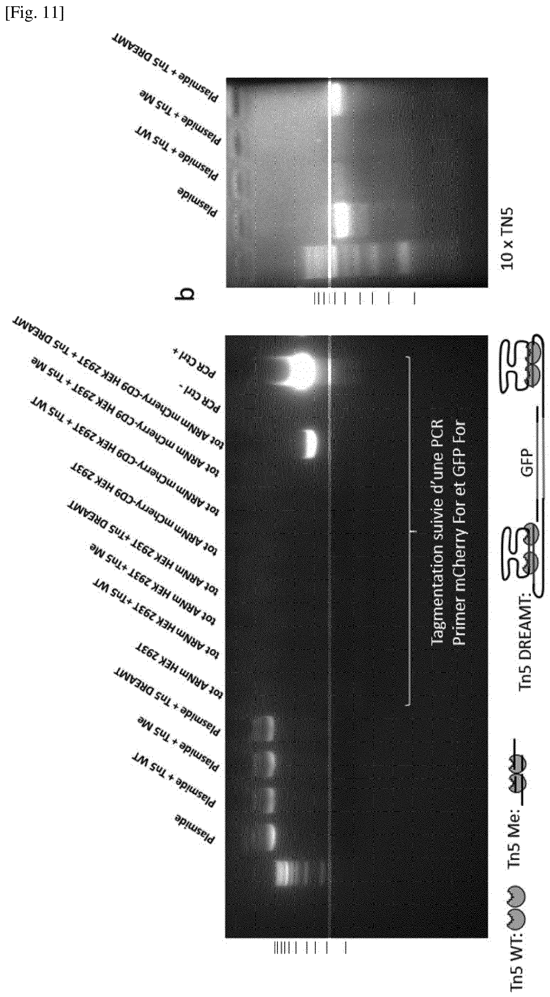

[Fig.11] la [Fig.11] représente des gels d'agaroses montrant la tagmentation selon l'invention. a. Gel d'agarose avec 3 groupes d'échantillons ; test des différents complexes de transposases (contrôle négatif, Tn5 WT, Tn5 Me et Tn5 DREAMT, i.e. selon l'invention) avec plasmide mCherry-CD9, PCR amplification des ARNm totaux de HEK293T, et PCR amplification des ARNm totaux de HEK 293T transfectées avec le plasmide mCherry-CD9 (ajout de contrôle PCR +/-). b Gel d'agarose des différents mix de transposases (voir a.) dix fois plus concentrés sur plasmides mCherry-CD9.

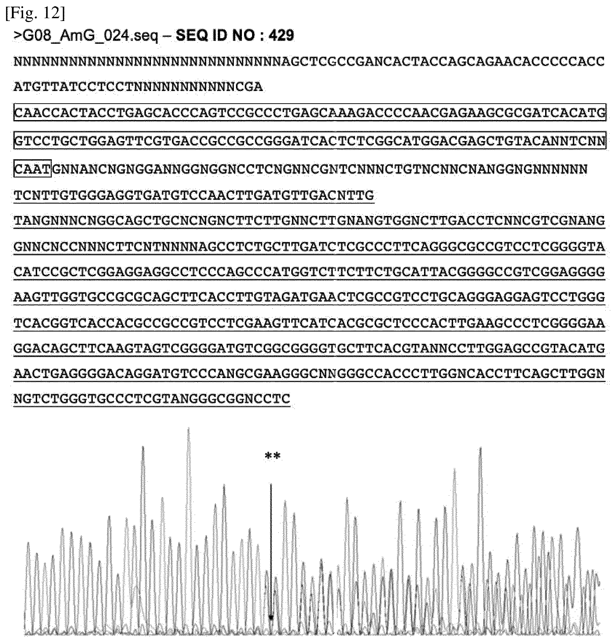

[Fig.12] la [Fig.12] représente le résultat de séquençage selon la méthode de Sanger de la bande amplifiée positive (Gel [Fig.10] a) pour le mix Tn5 DREAMT. La séquence (SEQ ID NO : 429) obtenue et le chromatogramme correspondant sont présentés. ** représente la zone d'insertion GFP. La séquence de la GFP est encadrée, et la séquence de la mCherry est soulignée.

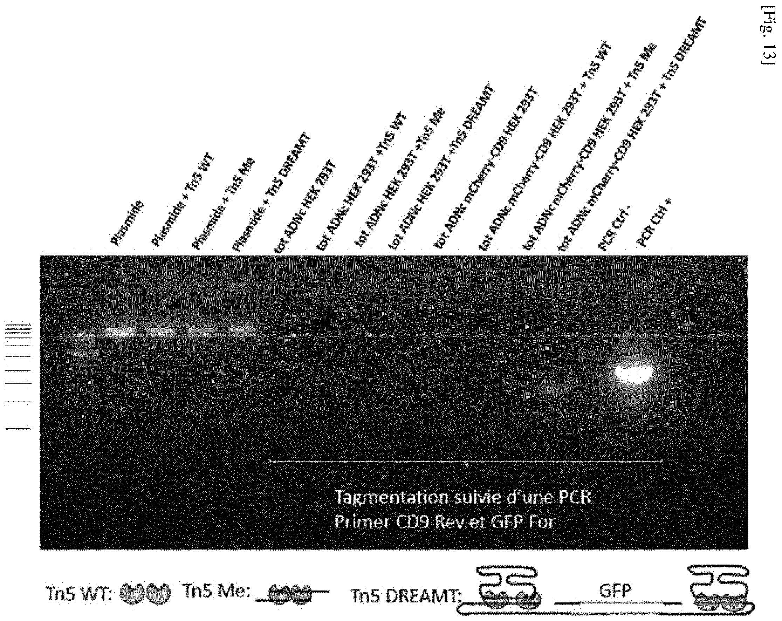

[Fig.13] la [Fig.13] représente un gel d'agarose avec 3 groupes d'échantillons ; test des différents complexes de transposases (contrôle négatif, Tn5 WT, Tn5 Me et Tn5 DREAMT) avec plasmide mCherry-CD9, PCR amplification d'ADNc total de HEK293T, et PCR amplification d'ADNc total de HEK 293T transfectées avec plasmide mCherry-CD9 (ajout de contrôle PCR +/-).

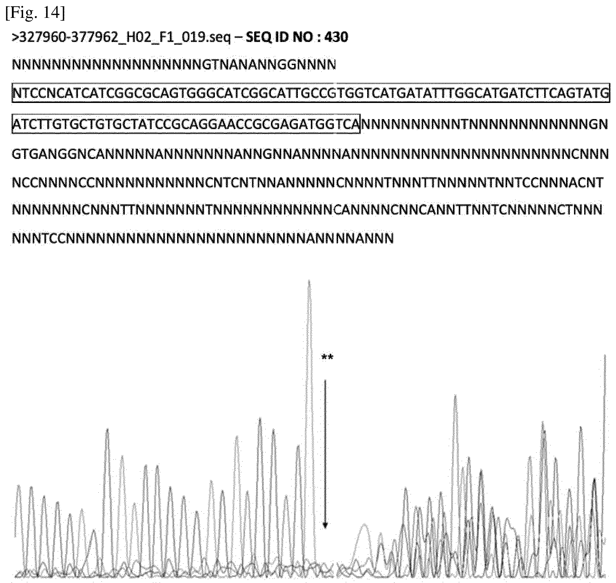

[Fig.14] la [Fig.14] montre le résultat de séquençage selon la méthode de Sanger de la bande amplifiée positive (Gel [Fig.13]) pour le mix Tn5 DREAMT. La séquence obtenue (SEQ ID NO : 430) et le chromatogramme correspondant sont présentés. ** représente la zone d'insertion GFP. La séquence CD9 est encadrée.

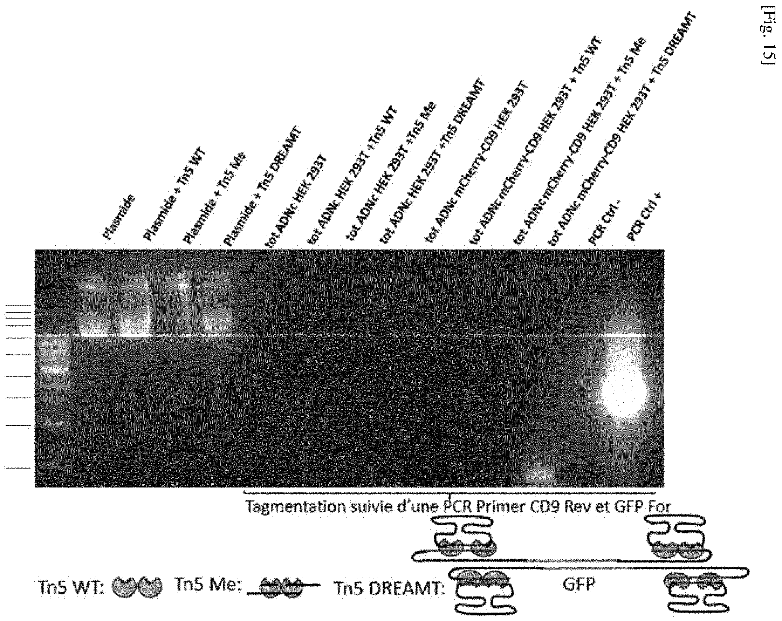

[Fig.15] la [Fig.15] représente un gel d'agarose avec 3 groupes d'échantillons ; test des différents complexes de transposases (contrôle négatif, Tn5 WT, Tn5 Me et Tn5 DREAMT) avec plasmide mCherry-CD9, PCR amplification d'ADNc total de HEK293T, et PCR amplification d'ADNc total de HEK 293T transfectées avec plasmide mCherry-CD9 (ajout de contrôle PCR +/-).

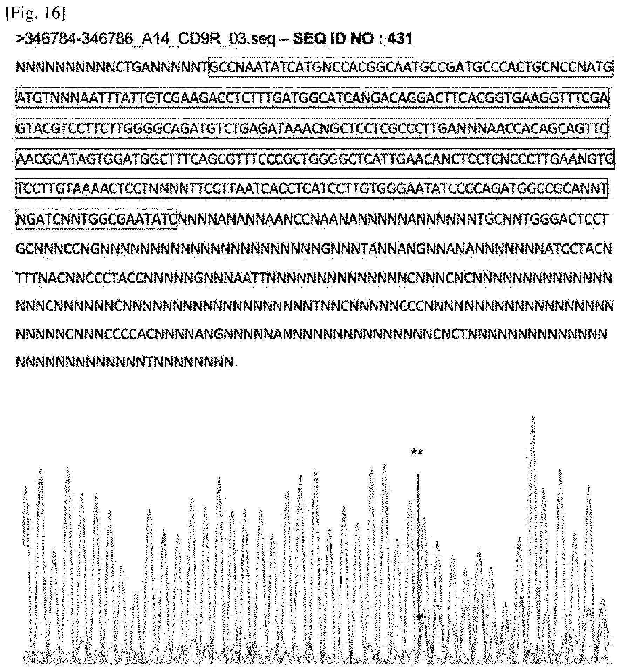

[Fig.16] la [Fig.16] représente le résultat de séquençage selon la méthode de Sanger de la bande amplifiée positive (Gel [Fig.15]) pour le mix Tn5 DREAMT. La séquence obtenue (SEQ ID NO : 431) et le chromatogramme correspondant sont présentés. ** représente la zone d'insertion GFP. La séquence CD9 est encadrée.

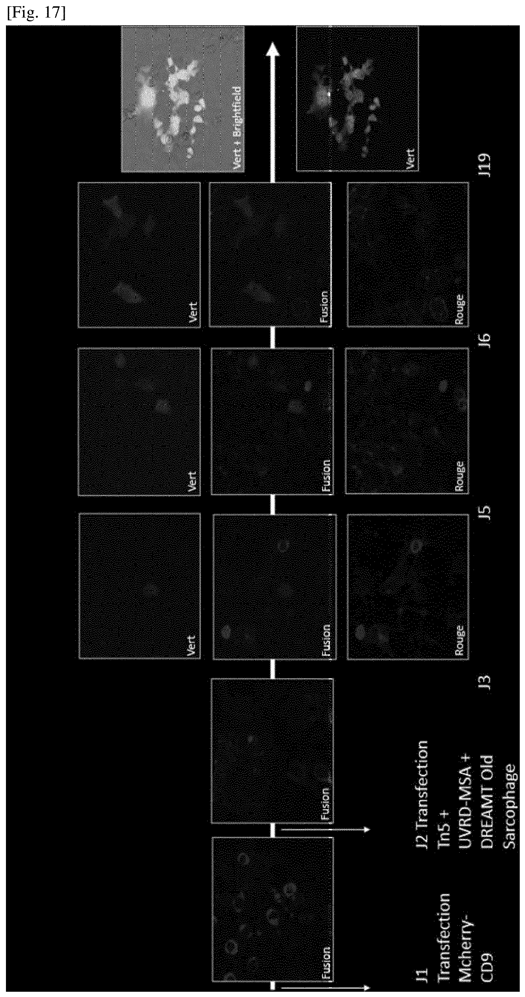

[Fig.17] la [Fig.17] représente le test du complexe UVRD-mSA/Tn5/DREAMT « nouveau design » sur le plasmide mcherry-CD9. Injection (transfection) du plasmide mCherry-CD9 aux cellules HEK 293T puis de la technologie DREAMT, recherche de changement de couleur visible (Rouge->vert comme mentionné sur la figure)

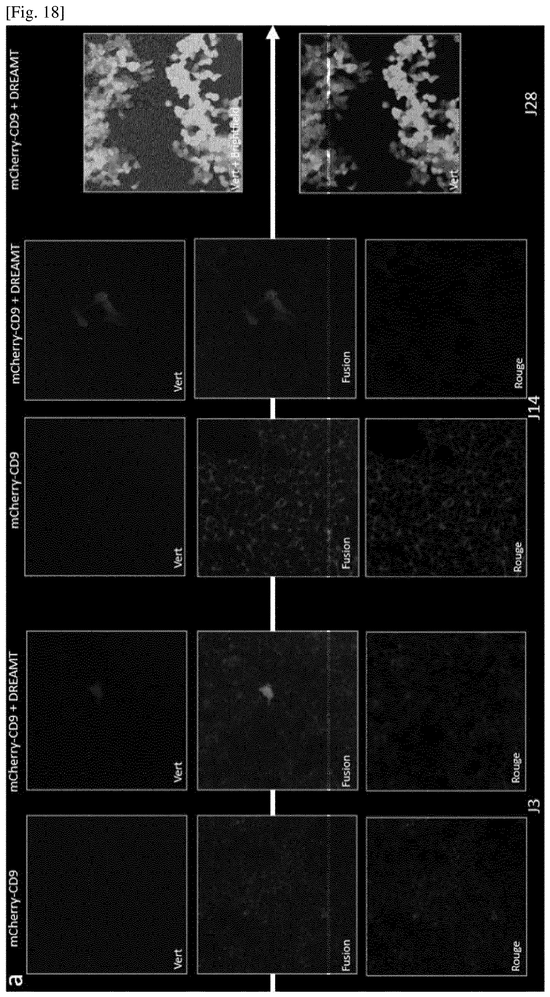

[Fig.18] la [Fig.18] représente la transfection de la technologie selon l'invention dans la lignée cellulaire HEK 293T exprimant de manière stable mCherry-CD9, puis recherche de changement de couleur visible (Rouge->vert comme mentionné sur la figure).

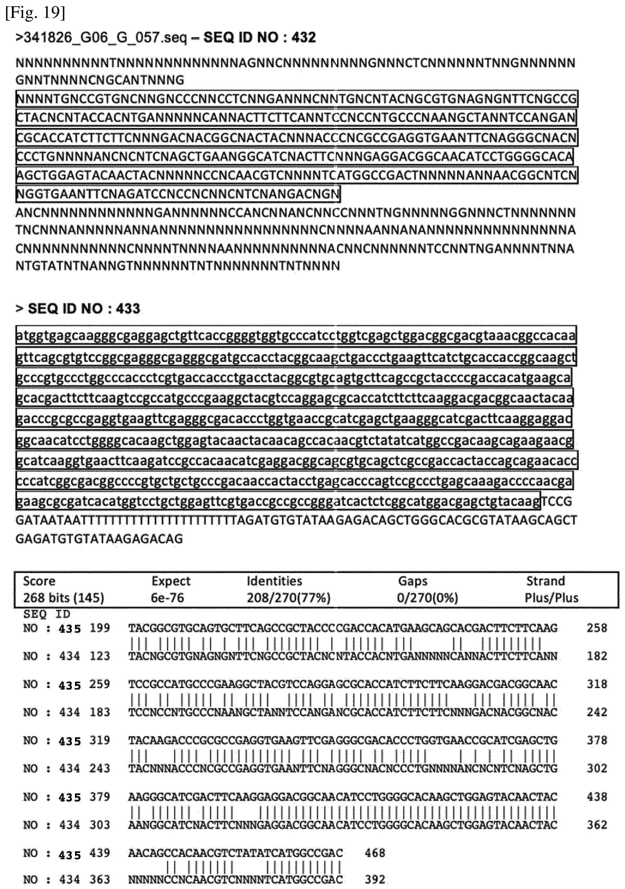

[Fig.19] la [Fig.19] représente les résultats de séquençage selon la méthode de Sanger du fragment GFP amplifié de la banque d'ADNc provenant de la lignée cellulaire mCherry-CD9+ de cellules HEK 293T transfectée avec la technologie DREAMT « nouveau design » (Cellules utilisées à J18) - SEQ ID NO : 432 - le remplacement GFP est indiqué en encadré. La séquence SEQ ID NO : 433 représente la séquence de la GFP Théorique (partie encadrée)

### Exemples

### Exemple 1 - Mise en œuvre de l'invention in vitro.

Le but de cet exemple est de montrer que l'ensemble selon l'invention permet facilement et spécifiquement de remplacer une séquence d'une molécule d'acide nucléique simple brin (ARN ou ADNc) par une séquence d'intérêt.

Dans cet exemple le but est de remplacer la séquence de mCherry-CD9 par la séquence de la GFP.
- Préparation de l'ensemble de recombinaison ciblant mCherry-CD9.

Préparation de deux tubes séparés contenant :
- pour le Tube 1 (10µL) :
   + 10µM l'oligonucléotide loop A (première molécule) comprenant dans la région A une séquence de reconnaissance de la séquence de mCherry ;
   + 10µM l'oligonucléotide A (troisième molécule) comprenant en 3' un demi site de restriction
   + l'oligo reverse BSPEi Frag (10µM)
- pour le Tube 2 (10µL),
   + 10µM l'oligonucléotide loop B (deuxième molécule) comprenant dans la région B une séquence de reconnaissance de la séquence de CD9 ;
   + 10µM l'oligonucléotide A (troisième molécule) comprenant en 3' un demi site de restriction
   + l'oligo Nde I Frag (10µM).

Les deux tubes sont alors chauffés à 95°C pendant 5 min puis laissés 1h à température ambiante.

Une fois à température ambiante, le contenu des tubes est mis en présence soit de l'enzyme NdeI soit de l'enzyme BSPEi afin de permettre la digestion des sites de restriction.

Les produits de digestion sont ensuite purifiés avec un kit PCR purification (élution 20µL).

En parallèle, une séquence codant la GFP est amplifiée afin de disposer en 5' et en 3' des sites de restriction Nde I et BSPEi. Le produit de PCR est ensuite soumis à une digestion avec les eux enzymes de restriction et le produit de digestion est purifié avec un kit PCR purification (élution 20µL).

Les contenus des Tubes 1 et 2 sont ensuite combinés avec le fragment GFP (amplifié et digéré) en présence de ligase du phage T4 (4µL, soit 100U) avec 5µL de T4 Buffer (10X) et 1µL ddH20, pour un volume total de réaction de 50µL.

La solution est alors laissée 1h à température ambiante, puis une purification sur gel est alors réalisée pour isoler le fragment GFP le plus important, supérieur à 1kilo bases (kb), et comportant les deux molécules simple brin (première et deuxième molécules de l'ensemble) à ses extrémités 5' et 3', pour former un complexe de recombinaison.

Le complexe de recombinaison est alors prêt à être incubé avec des dimères de transposases.

En contrôle, des oligonucléotides MeA et MeB à 10µM chacun ont été préparés avec le fragment MERev (préalablement décrit par S. Picelli et al Genome Res 2014) :
Tn5MErev,
   5'-[phos]CTGTCTCTTATACACATCT-3' (SEQ ID NO : 11)
Tn5ME-A (Illumina FC-121-1030),
   5'-TCGTCGGCAGCGTCAGATGTGTATAAGAGACAG-3'; (SEQ ID NO : 12)
et Tn5ME-B (Illumina FC-121-1031),
   5'-GTCTCGTGGGCTCGGAGATGTGTATAAGAGACAG-3' (SEQ ID NO : 13)
par appariement à 95°C pendant 5 min puis laissés 1h à température ambiante. Ces oligos servent de contrôle positif pour la réaction de tagmentation.

Concernant la production de la transposase Tn5, elle est produite via les recommandations de la publication suivante : S. Picelli et al Genome res 2014.

Les deux précédentes préparations, complexe de recombinaison et les oligos de contrôle positif ont ensuite été mélangé avec la production de transposase Tn5 via le protocole suivant :
0,125 Vol de la solution à 10µM de complexe de recombinaison ou des oligos de contrôle (ou juste de la Dialysis buffer (cf S. Picelli et al Genome res 2014) pour le Tn5 sauvage dit WT) + 0,4 Vol de solution 100% glycérol + 0,24 Vol de Dialysis buffer (cf S. Picelli et al Genome res 2014) + 0,36 Vol de solution Tn5.

La réaction est alors mise 30 min de 45°C à 37°C en diminuant de 1°C toutes les 4 minutes dans un thermocycleur.

Dans le même temps, plusieurs cibles ont été produites pour tester la technologie selon l'invention.

Un plasmide mCherry-CD9 a été utilisé comme contrôle négatif ainsi qu'une production d'une banque d'ARNm et d'ADNc provenant de cellules HEK293T préalablement transfectées par lipofectamine 3000 avec le vecteur mCherry-CD9.

Quarante-huit heures après transfection et contrôle visuel (membrane rouge du fait de l'expression de la mCherry) des cellules HEK, la population totale des ARNm est alors extraite et reverse transcrite en ADNc.

Ces banques d'ARNm ou d'ADNc sont alors utilisées pour notre test « en tube » de la technologie DREAMT.

Afin de caractériser et tester la technologie selon l'invention, différentes cibles d'ARNm/ADNc et plasmide ont été mises en contact avec
- les dimères Tn5 WT : sans oligo,
- Tn5 Me : avec oligo Me (contrôle positif) et
- Tn5 Complexe : avec l'ensemble selon l'invention.

Trois types de cibles ont été testées, le plasmide mCherry-CD9 et la banque d'ARNm ou d'ADNc avec ou sans transfection de mCherry-CD9.

Les solutions d'ADN/ARN double ou simple brin ont été mises en contact avec les différentes solutions de Tn5 activées via la réaction suivante :
- concentration X ou 10X de solution Tn5 WT, Tn5 Me, Tn5 Complexe ou même volume de ddH20 + 500ng de plasmide mCherry-CD9 ou 2µL de cDNA ou ARNm
   + 5X tagmentation buffer (100mM HEPES, 50 mM MgCl2, 40% PEG 3500)
   + ddH20 QSP 20µL.

Chaque réaction de tagmentation est réalisée dans un thermocycleur à 55°C durant 7 min, puis 0.5µL de solution de protéinase k (20µg/µL) est ajoutée avant un second temp d'incubation à 55°C durant 7 min afin d'inactiver la transposase.

Ainsi comme présenté sur la [Fig.11], une sédimentation (ligation) du bout 3' du GFP a été effectuée vers la séquence cible mCherry.

Sur le gel d'agarose [Fig.11], le plasmide mCherry-CD9 (Davidson Lab) a été mis en contact avec deux concentrations (X et 10X) de solution de Tn5. Comme attendu, à forte concentration (10X), le mix WT Tn5 et le contrôle positif Me Tn5 ont réalisés leur tagmentation, illustré par une dégradation du plasmide (*smear* ou trainée de dégradation, disparition de la bande plasmidique).

De plus, comme attendu, l'ensemble seul n'a pas pu réaliser de tagmentation (dégradation du plasmide), illustré par la préservation de la bande d'ADN sur le gel d'agarose ([Fig.11]) [car elle ne comporte pas de molécule pour ouvrir le double brin d'ADN plasmidique (Hélicases) afin de réaliser un appariment sur la cible mCherry-CD9 ce qui libèrerait les molécules de Tn5 et permettrait la tagmentation]. Cette donnée permet de conclure en l'emprisonnement des dimères de Tn5 Complexe qui ne peuvent réaliser leur activité de tagmentation que par l'appariement du brin à recombiner avec le simple brin cible, ici le fragment d'ARNm mCherry-CD9.

La même concentration de solution de Tn5 (X) a été utilisée avec 2µL de la banque d'ARNm (provenant des cellules HEK293T normales ou transfectées avec du mCherry CD9). Puis une PCR avec les primers Forward mCherry et Forward GFP a été réalisée (cf Tableau 3).

Table 3 : Oligos utilisés pour les réactions de PCR et les contructions plasmidiques

**[Tableaux3]**

| Noms | Séquences 5' to 3' |
|---|---|
| GFP For | CTGGTCGAGCTGGACGGCGACG (SEQ ID NO : 14) |
| GFP Rev | CACGAACTCCAGCAGGACCATG (SEQ ID NO : 15) |
| mCherry For | AAGGGCGAGGAGGATAACATG (SEQ ID NO : 16) |
| CD9 Rev | GACCATCTCGCGGTTCCT (SEQ ID NO : 17) |
| GFP Nde1 For | ACTTGGCATATGATGGTGAGCAAGGGCGAGGA (SEQ ID NO : 18) |
| GFP Nde1 Rev | ACTTGGCATATGCTTGTACAGCTCGTCCAT (SEQ ID NO : 19) |
| GFP bspei For | TACAAGTCCGGAATGGTGAGCAAGGGCGAGGA (SEQ ID NO : 20) |
| GFP bspei Rev | TACAAGTCCGGACTTGTACAGCTCGTCCAT (SEQ ID NO : 21) |
| CMV Nde1 For | ACTTGGCATATGCCAAGTACGCCCCCTATTGA (SEQ ID NO : 22) |
| UVRD For | CTCTTCGCTAGCTGCCACCATGACGCGTGGCCCCAAGA AAAAGCGGAAAGTGGGACCGGCCACCATGGACGTTTC TTACCTGCTCG (SEQ ID NO : 23) |
| UVRD Rev | AACAACACGTGCCGGTGATACCCGCTTCCGCGCCTGAT CCACCACCACCTGACCCACCACCAGCCACCGACTCCAG CCGGG (SEQ ID NO : 24) |
| mSA For | CTCTTCGCTAGCGCGGAAGCGGGTATCAC (SEQ ID NO : 25) |
| mSA Rev | AACAAGAATTCTTATTATTTAACTTTGGTGAAGGT (SEQ ID NO : 26) |
| Tn5 For | CTCTTCGCTAGCATGATTACCAGTGCACTGCAT (SEQ ID NO : 27) |
| Tn5 Rev | AACAAGAATTCTTATTAGATTTTAATGCCCTGCGCCA (SEQ ID NO : 28) |

Comme attendu, aucun signal n'a été détecté pour la banque d'ARNm obtenue à partir de cellules sans transfection du vecteur mCherry-CD9. De même, aucune amplification n'a également pas été détectée pour les contrôles négatifs (fragments ARNm seuls, ARNm + Tn5 WT, ARNm + Tn5 Me) et les échantillons de banque d'ARNm avec transfection du mCherry-CD9. Néanmoins, toujours sur ce même lot d'échantillons, une bande franche a été détectée au bon poids moléculaire ≈1kb ( [Fig.11]) pour l'échantillon correspondant à un remplacement du mCherry-CD9 par la séquence GFP (Tn5 Complexe). Cette bande souligne une coupure du bout 3' mCherry (ARNm anti-sens) avec insertion du bout 3' GFP (brin sens) ou autrement dit, une ligation du brin antisens mCherry ARNm au brin sens GFP dans un sens 3' vers 5'.

Le produit d'amplification a été séquencé et la séquence obtenue est présentée en [Fig.12]. Comme attendu, nous avons détecté une amplification de mCherry vers le coté 3' GFP avec une détection des deux séquences reliées par ligation. Le chromatogramme de ce séquençage montre clairement la zone de sédimentation (tagmentation) à 10 pb de la séquence cible ([Fig.12]).

La même analyse a été répétée mais cette fois-ci en changeant le design pour une ligation vers le cote 3' : CD9 du brin sens d'DNAc ([Fig.12]). Ainsi, les mêmes résultats ont été obtenus avec un gel d'amplification montrant une bande attendue vers ≈1kb ([Fig.13]) pour la banque d'ADNc avec transfection mCherry-CD9 et mise en contact avec l'ensemble selon l'invention (Tn5 Complexe), et un séquençage du fragment obtenu a été réalisé ([Fig.14]). Comme attendu, ce séquençage nous a permis d'identifier la séquence du brin sens CD9 d'ADNc sédimenté a de multiples séquences du brin sens GFP coté 5' (ligation en 3' de la séquence sens du GFP ; [Fig.14]), à 5pb de la séquence cible.

### Exemple 2 - Mise en œuvre de l'invention in cellulo

Fort de ces résultats encourageants, le design a été modifié afin d'obtenir une version compatible avec une transfection directe de l'ensemble, du plasmide Tn5 et la protéine d'ouverture de brin, le plasmide de l'hélicase bactérienne UVRD qui fusionne avec la streptavidine monomérique (plasmide UVRD-mSA) afin que cette dernière s'attache in situ au à l'une des molécules de l'ensemble via des oligos préalablement biotinylés. Pour ce faire, une nouvelle version de l'ensemble a été développée pour une mise en place plus facile et permettant un contrôle du design proche des 100% (réduction des dimères de Tn5 non emprisonnés par le système de sarcophage Beacon et de ce fait des possibles « off target » associées). Ainsi, pour ce « Ensemble New design », le même type de protocole que le précèdent a été réalisé avec certains changements majeurs. En termes de design lui-même, les oligos Loop nDREAMT A, B, C et D (un par dimère de Tn5 et par cible pour une tagmentation des quatre zones cibles - remplacement double brin complet-) sont plus longs avec trois séquences d'attachement à la transposase. Cela permet un appariement rapide et contrôlé avec juste un ajout de la dernière séquence d'attache à la transposase reliée aux fragments GFP de remplacement du fragment cible mCherry-CD9 (Oligos nDREAMT A, B, C et D). De plus, d'autres oligos nDREAMT Bio A et B (oligos biotinylés) sont ajoutés pour permettre un assemblage *in cellulo* du de l'ensemble avec la protéine de fusion UVRD-mSA.

Dans deux tube séparés 1 et 2 ont été mélangés les oligos suivants ; pour le tube 1 (10µL), loop nDREAMT A et C (10µM) + oligos nDREAMT A et C (10µM) + oligo nDREAMT Bio A (10µM), pour le tube 2 10µL), loop nDREAMT B et D (10µM) + oligos nDREAMT B et D (10µM) + oligo nDREAMT Bio B (10µM). Les deux tubes sont alors chauffés à 95°C pendant 5 min puis laissés 1h à température ambiante puis chaque tube est alors digéré avec les enzymes de restriction correspondantes et purifié par PCR purification kit (élution 20µL).

Dans le même temps, une séquence amplifiée du GFP comprenant comme bouts flanquant les séquences d'enzymes de restriction Nde I en 5' et BSPEi en 3' (oligos avec architecture de site de restriction digérés après PCR) fut amplifiée puis digérée avec les enzymes de restriction correspondantes et purifiée par kit PCR purification élué à un volume de 20µL.

Les contenus des Tubes 1 et 2 sont ensuite mixés avec le Fragment GFP néo amplifié et digéré, une solution de T4 ligase (4µL soit 100U), 5µL de T4 Buffer (10X) et 1µL ddH20, pour un volume total de réaction de 50µL. La solution est laissée 1h à température ambiante, puis une purification sur gel est réalisée pour isoler le fragment GFP le plus important, supérieur à 1Kb, et comportant les quatre sarcophages A, B, C et D à ses extrémités 5' et 3'. Le fragment de remplacement GFP avec ses quatre sarcophages positionnés aux extrémités 5' et 3' est alors prêt à recevoir les dimères de transposase ([Fig.15]).

Avant de tester ce système au niveau cellulaire, le nouveau design a été testé via la même série d'expériences que précédemment avec un ligation du coté 3' GFP (Brin sens) vers le coté CD9 d'ADNc cible mCherry-CD9 (Brin sens). Ainsi, le même type de résultat que précédemment fut obtenu (tous les contrôles négatifs restants sont non détectables) avec après amplification via les primers PCR Forward GFP et Reverse CD9, une bande attendue vers les 0.5 kb ([Fig.15]). Apres séquençage de Sanger de cette bande, comme attendu, une détection du fragment CD9 dans le sens 5' vers 3' fut faite avec une ligation de séquence multiple de GFP à son extrémité 3' ([Fig.16]), à 2 bases près de la séquence cible originelle ([Fig.16]).

Après cette vérification positive de ce nouveau design, test in situ dans des cellules HEK293T exprimant du mCherry-CD9 a été réalisé. Pour ce faire, deux plasmides d'expression protéique intracellulaire pour les protéines de transposase Tn5 et UVRD-mSA ont été créés. Pour le plasmide Tn5, la séquence Tn5 a été amplifiée par PCR via le plasmide PTXB1-Tn5 (S. Picelli et al Genome Res 2014). A noté que l'étiquette queue intéine permettant la purification de la transposase Tn5 via une liaison avec des billes de chitine et un auto-clivage ne laissant aucun élément du tag sur la protéine Tn5 (S. Picelli et al Genome Res 2014), a été remplacée par un codon stop. Le tout a été cloné à la place du fragment mCherry-CD9 du plasmide mCherry-CD9 (Davidson Lab) via les enzymes de restriction BMT1 et EcoR1, pour obtenir, finalement, un plasmide promoteur CMV-Tn5. L'amplification par PCR du fragment mSA a été réalisée (Table 1) via le plasmide pRSET-mSA (Sheldon Park Lab) avec un ajout des sites de restriction BMT1 en 5' et EcoR1 en 3' (Table 1). Une première étape de clonage a été réalisée dans le plasmide mCherry-CD9 via les enzymes BMT1 et EcoR1 avec un remplacement du fragment mCherry-CD9 par le fragment mSA obtenant un plasmide promoteur CMV-mSA. Pour le fragment UVRD, une banque d'ADNc de bactéries E-coli a été générée (DH5α). Une amplification de l'ADNc UVRD par PCR a été réalisée avec des oligonucléotides précis (Table 1). En coté 5', on retrouve les éléments habituels comme la séquence BMT1 pour le clonage et la séquence Kozac mais également la séquence d'importation nucléaire NLS pour permettre d'avoir un complexe complet Complexe + Tn5 + UVRD-mSA importé dans le noyau cellulaire. En 3', une partie flexible a été ajoutée (queue poly glycine type GGGSx3), un bout de la séquence 5' mSA jusqu'à une enzyme de restriction précise contenue dans la séquence 5'mSA PMiI. Une deuxième étape de clonage a alors été réalisée utilisant les enzymes de restriction BMT1 et PMiI permettant un ajout en 5' de la séquence UVRD et obtenant un plasmide final de type promoteur CMV-UVRD-mSA.

La transfection des cellules HEK 293T a été réalisée par lipofectamine 3000 avec le plasmide mCherry-CD9. Après une attente de 24h et une vérification par microscope confocal du signal rouge sur la membrane cytoplasmique, les cellules ont été transfectées avec les trois composés : les plasmides CMV-Tn5 et CMV-UVRD-mSA et le complexe New design. Ce complexe complet permet un remplacement double brin de la séquence située entre les quatre cibles (quadruplet de cibles ; deux cotés mCherry et deux cotés CD9) par une séquence CMV-GFP. Ainsi, par cette expérience, une diminution voire un remplacement du signal membranaire rouge par un signal cytoplasmique vert était attendu.

Comme attendu et présenté à la [Fig.17], à 5 jours de la deuxième transfection avec la technologie selon l'invention, des cellules vertes ont été détectées.

Ces cellules ont été isolées par tri cellulaire par cytométrie et sachant que le fragment CMV-GFP a remplacé la partie mCherry-CD9 du plasmide CMV-mCherry-CD9, ce nouveau plasmide CMV-GFP devrait contenir une séquence de sélection antibiotique de création de lignée cellulaire NeoR/KanR. Après plus de 6 jours de transfection, ces cellules encore vertes ont été placées dans un milieu comprenant 2mg/ml de G418 changée tous les jours pendant 14 jours puis maintenue à concentration 0.5mg/ml pendant 3 mois.

Comme attendu, après 19 jours post transfection, une nouvelle lignée cellulaire verte GFP+ fut obtenue, très stable malgré les congélations et décongélations répétées (cf [Fig.17]).

Afin de reconfirmer ce résultat in situ avec plus de certitudes, une lignée cellulaire stable HEK293T rouge membranaire a été créée par une transfection avec le plasmide CMV-mCherry-CD9 et traitement G418 2mg/ml comme réalisé précédemment. Après deux semaines de traitement, une lignée cellulaire HEK 293T rouge membranaire a été obtenue.

Cette lignée cellulaire a été transfectée comme précédemment avec la technologie susmentionnée. Après trois jours de transfection, les premières cellules vertes ont commencé à apparaitre (cf [Fig.18]). Après 14 jours post transfection, certains groupes de cellules vertes ont été identifiés et isolés pour une analyse plus approfondie (cf [Fig.18]).

Après isolement de ces cellules, une reverse transcription a été réalisée à partir de ces cellules 18 jours post transfection, afin d'obtenir une banque d'ADNc.

Une amplification de la séquence GFP a alors été faite par PCR et le fragment amplifié a alors été séquencé en selon la méthode de Sanger.

Comme attendu, après analyse par confrontation avec les bases de données de séquence (NCBI blast), on retrouve une similitude très proche avec le fragment GFP théorique de remplacement de la séquence mCherry-CD9, [Fig.19]. De plus, après presque un mois, les cellules vertes obtenues restent stables avec un fort signal vert ( [Fig.18]). Ces résultats confirment l'efficacité in cellulo de la technologie selon l'invention, permettant un remplacement de la séquence cible mCherry-CD9 par la séquence de remplacement GFP illustrée par le changement de couleur du rouge membranaire vers le vert cytoplasmique. Ce changement de couleur permet de valider l'importation nucléaire du complexe new design + Tn5 + UVRD-mSA (avec le NLS sur la séquence N-Term de l'UVRD) l'ouverture des brins d'ADN réalisée par les deux hélicases coté 5' et 3', l'appariement aux quatre endroits cibles voulus (deux coté mCherry et deux coté CD9) et le remplacement de la séquence mCherry-CD9 par le fragment CMV-GFP par la technologie selon l'invention.

## Revendications

1. Complexe comprenant
- une première molécule d'acides nucléiques simple brin comprenant ou constituée essentiellement d'une séquence A permettant l'insertion d'une séquence complémentaire d'un acide nucléique d'intérêt,
ladite séquence A étant liée en 5' à une première séquence riches en A/T, notamment riches en T, de 40 à 60 nucléotides de long et en 3' à une deuxième séquence riches en A/T, notamment riches en T, de 40 à 60 nucléotides de long, lesdites première et deuxième séquences riches en A/T, notamment riches en T, comprenant respectivement un premier et un deuxième domaine de 6 à 12 nucléotides riches en G/C, la séquence du premier domaine étant complémentaire de la séquence du deuxième domaine, ledit premier et deuxième domaine étant positionnés de 15 à 52 nucléotides de ladite séquence A, ladite première molécule comprenant en son extrémité 5' une première séquence orientés 5'-3' de reconnaissance d'une transposase et en son extrémité 3' au moins une deuxième séquence de reconnaissance de ladite transposase ; et
- une deuxième molécule d'acides nucléiques simple brin comprenant ou constituée essentiellement en son extrémité 5' d'au moins une séquence complémentaire de ladite deuxième séquence de reconnaissance de ladite transposase,
ledit complexe étant tel que les première et deuxième molécules d'acides nucléiques simple brin sont appariées selon la complémentarité des bases définie par Watson et Crick de sorte à définir deux sites double brin de liaison de ladite transposase.

2. Complexe selon la revendication 1, où ladite séquence A comprend une séquence complémentaire d'un acide nucléique d'intérêt.

3. Complexe selon l'une quelconque des revendications 1 à 2, où ladite première molécule comprend en son extrémité 5' une première séquence orientée 5'-3' de reconnaissance d'une transposase et en son extrémité 3' une deuxième séquence orientée 5'-3' de reconnaissance de ladite transposase et
où la deuxième molécule comprend son extrémité 5' une première séquence complémentaire de ladite première séquence de reconnaissance de ladite transposase suivi d'une une deuxième séquence complémentaire de ladite deuxième séquence de reconnaissance de ladite transposase.

4. Complexe selon l'une quelconque des revendications 1 à 3, où ladite première molécule comprend en son extrémité 5' une première séquence orientée 5'-3' de reconnaissance d'une transposase et en son extrémité 3' une deuxième séquence de reconnaissance de ladite transposase, suivie d'une première séquence complémentaire de ladite première séquence de reconnaissance de ladite transposase et
où la deuxième molécule comprend son extrémité 5' une séquence complémentaire de ladite deuxième séquence de reconnaissance de ladite transposase.

5. Complexe selon l'une quelconque des revendications 1 à 4, où ladite transposase est une transposase bactérienne, notamment une transposase choisie parmi Tn5, Tn9, Tn10 ou Tc1/mariner.

6. Complexe selon l'une quelconque des revendications 1 à 5, où ladite première molécule est couplée à une enzyme, notamment par le biais d'un nucléotide modifié.

7. Complexe selon l'une quelconque des revendications 1 à 6, où ladite première molécule comprend l'une des séquences suivantes SEQ ID NO : 436, SEQ ID NO : 440, SEQ ID NO : 443, SEQ ID NO : 444, SEQ ID NO : 448, SEQ ID NO : 449, SEQ ID NO : 450, SEQ ID NO : 454, SEQ ID NO : 455, SEQ ID NO : 456, SEQ ID NO : 457, SEQ IDNO : 458, SEQ ID NO : 462, SEQ ID NO : 463, SEQ ID NO : 464, SEQ ID NO : 465, SEQ ID NO : 466 et SEQ ID NO : 641.

8. Complexe selon l'une quelconque des revendications 1 à 7, ledit complexe comprenant un couple de première et de deuxième molécules, lesdites première et de deuxième molécules comprenant les séquences telles que définies dans la table 2.

9. Complexe selon l'une quelconque des revendications 1 à 6, ledit complexe comprenant l'un des 300 couples de première et deuxième molécule tels que définis dans la table 4.

10. Ensemble comprenant
- une première molécule d'acides nucléiques simple brin comprenant ou constituée essentiellement d'une séquence A permettant l'insertion d'une séquence complémentaire d'un acide nucléique d'intérêt, ou comprenant une séquence complémentaire d'un acide nucléique d'intérêt, ladite séquence complémentaire étant liée en 5' à une première séquence riches en T de 40 à 60 nucléotides de long et en 3' à une deuxième séquence riches en T de 40 à 60 nucléotides de long, lesdites première et deuxième séquences riches en T comprenant respectivement un premier et un deuxième domaine de 6 à 12 nucléotides riches en G/C, la séquence du premier domaine étant complémentaire de la séquence du deuxième domaine, ledit premier et deuxième domaine étant positionnés de 15 à 52 nucléotides de ladite séquence A, ladite première molécule comprenant en son extrémité 5' au moins une première séquence orientés 5'-3' de reconnaissance d'une transposase et en son extrémité 3' une deuxième séquence de reconnaissance de ladite transposase,
- une deuxième molécule d'acides nucléiques simple brin comprenant ou constituée essentiellement d'une séquence B permettant l'insertion d'une séquence complémentaire d'un acide nucléique d'intérêt, ou comprenant une séquence complémentaire d'un acide nucléique d'intérêt, ladite séquence B complémentaire étant liée en 5' à une troisième séquence riche en T de 40 à 60 nucléotides de long et en 3' à une quatrième séquence riche en T de 40 à 60 nucléotides de long, ladite troisième et quatrième séquence riche en T comprenant respectivement un troisième et un quatrième domaine de 6 à 12 nucléotides riches en G/C, la séquence du troisième domaine étant complémentaire de la séquence du quatrième domaine, ledit troisième et quatrième domaine étant positionnés de 15 à 52 nucléotides de ladite séquence B, ladite deuxième molécule comprenant en son extrémité 5' au moins la première séquence orientés 5'-3' de reconnaissance de ladite transposase et en son extrémité 3' la deuxième séquence de reconnaissance de ladite transposase,
ladite séquence B étant une séquence complémentaire dudit acide nucléique d'intérêt, la séquence A étant positionnée en 5' d'une région d'intérêt dudit acide nucléique d'intérêt et la séquence B positionnée en 3' de la région d'intérêt dudit acide nucléique d'intérêt, et
- une troisième molécule simple brin comprenant
dans sa partie 5', au moins une séquence complémentaire de ladite deuxième séquence de reconnaissance de ladite transposase de la première molécule,
dans sa partie 3' au moins une séquence complémentaire de ladite première séquence de reconnaissance de ladite transposase de la deuxième molécule, et une région située entre séquence complémentaire de ladite deuxième séquence de reconnaissance de ladite transposase de la première molécule et la séquence complémentaire de ladite première séquence de reconnaissance de ladite transposase de la deuxième molécule permettant l'insertion d'une molécule d'acide nucléique de remplacement simple brin,
les première et troisième molécules d'acides nucléiques simple brin étant appariées selon la complémentarité des bases définie par Watson et Crick de sorte à définir deux sites double brin de liaison de ladite transposase et les deuxième et troisième molécules d'acides nucléiques simple brin étant appariées selon la complémentarité des bases définie par Watson et Crick de sorte à définir deux sites double brin de liaison de ladite transposase.

11. Ensemble selon la revendication 10, ledit ensemble comprenant l'un des 300 couples de première et troisième molécule tels que définis dans la table 5.

12. Kit comprenant au moins un vecteur permettant l'expression d'une recombinase et les première, deuxième et troisième molécules de l'ensemble tel que défini à la revendication 10.

13. Utilisation de l'ensemble tel que défini à la revendication 10, pour l'ingénierie des acides nucléiques, notamment pour la substitution d'une séquence cible par une séquence d'intérêt, sous réserve que l'utilisation ne comprenne pas un procédé pour la modification de l'identité génétique germinale d'êtres humains et que ladite utilisation ne soit pas une méthode pour le traitement du corps humain ou animal par une chirurgie ou une thérapie.

14. Méthode de remplacement d'une région cible d'une molécule d'acide nucléique par une région d'intérêt d'une autre molécule d'acide nucléique, de sorte à obtenir une molécule d'acide nucléique hybride, ladite méthode comprenant :
- la mise en contact d'un ensemble tel que défini dans la revendication 10, avec l'acide nucléique comprenant la région cible,
ledit ensemble étant tel que
la séquence A de ladite première molécule comprend une séquence complémentaire de la région immédiatement en 5' de la région cible,
la séquence B de ladite première molécule comprend une séquence complémentaire de la région immédiatement en 3' de la région cible, et
la troisième molécule comprend ladite région d'intérêt dans la région située entre séquence complémentaire de ladite deuxième séquence de reconnaissance de ladite transposase de la première molécule et la séquence complémentaire de ladite première séquence de reconnaissance de ladite transposase de la deuxième molécule, afin d'obtenir un complexe de remplacement,
- la mise en présence du complexe de remplacement avec une transposase reconnaissant les sites double brin de liaison de ladite transposase contenus dans ledit ensemble, pour obtenir un complexe de recombinaison, et
- la recombinaison du complexe de combinaison pour obtenir la molécule d'acide nucléique hybride comprenant la région d'intérêt à la place de la région ciblesous réserve que ladite méthode ne soit pas un procédé pour la modification de l'identité génétique germinale d'êtres humains et que ladite méthode ne soit pas une méthode pour le traitement du corps humain ou animal par une chirurgie ou une thérapie.

15. Méthode d'édition du génome d'une cellule, permettant de remplacer un fragment spécifique de l'ADN double brin dudit génome de ladite cellule par un autre fragment d'ADN double brin d'intérêt, pour obtenir un génome hybride recombiné comprenant l'autre fragment d'ADN double brin d'intérêt à la place du fragment spécifique de l'ADN double brin, ladite méthode comprenant :
- la préparation d'un premier ensemble tel que défini dans la revendication 10,
où la séquence A de la première molécule comprend une séquence complémentaire de la région adjacente en 5' du fragment spécifique ;
où la séquence B de la deuxième molécule comprend une séquence complémentaire de la région adjacente en 3' du fragment spécifique ; et
où la troisième molécule comprend, entre la région située entre séquence complémentaire de ladite deuxième séquence de reconnaissance de ladite transposase de la première molécule et la séquence complémentaire de ladite première séquence de reconnaissance de ladite transposase de la deuxième molécule, une séquence d'un des brins dudit fragment spécifique ;
- et éventuellement la préparation d'un deuxième ensemble tel que défini dans la revendication 10,
où la séquence A de ladite région complémentaire de la première molécule du deuxième ensemble comprend une séquence complémentaire de la région adjacente en 5' du fragment spécifique ;
où la séquence B de ladite région complémentaire de la deuxième molécule du deuxième ensemble comprend une séquence complémentaire de la région adjacente en 3' du fragment spécifique ; et
où la troisième molécule du deuxième ensemble comprend, entre la région située entre séquence complémentaire de ladite deuxième séquence de reconnaissance de ladite transposase de la première molécule du deuxième ensemble et la séquence complémentaire de ladite première séquence de reconnaissance de ladite transposase de la deuxième molécule du deuxième ensemble, la séquence du brin complémentaire dudit fragment spécifique contenu dans la troisième séquence du premier ensemble ;
où la séquence complémentaire de la région adjacente en 5' du fragment spécifique contenue dans la région A de la première molécule du premier ensemble est au plus complémentaire à 95% de la séquence complémentaire de la région adjacente en 5' du fragment spécifique contenue dans la région A de la première molécule du deuxième ensemble ; et
où la séquence complémentaire de la région adjacente en 3' du fragment spécifique contenue dans la région B de la première molécule du premier ensemble est au plus complémentaire à 95% de la séquence complémentaire de la région adjacente en 3' du fragment spécifique contenue dans la région B de la première molécule du deuxième ensemble ;
pour obtenir un complexe de recombinaison ;
- la mise en contact de ladite cellule avec ledit complexe de recombinaison, pour obtenir une cellule prête à être éditée,
- l'expression dans ladite cellule prête à être éditée de ladite transposase, pour obtenir une cellule éditée,
- la sélection de la cellule éditée, où le génome de ladite cellule éditée comprend à la place du fragment spécifique de l'ADN double brin l'autre fragment d'ADN double brin d'intérêtsous réserve que ladite méthode ne soit pas un procédé pour la modification de l'identité génétique germinale d'êtres humains et que ladite méthode ne soit pas une méthode pour le traitement du corps humain ou animal par une chirurgie ou une thérapie.

## Patentansprüche

1. Komplex, umfassend
- ein erstes einzelsträngiges Nukleinsäuremolekül, das eine Sequenz A umfasst oder im Wesentlichen daraus besteht, die die Insertion einer zu einer Nukleinsäure von Interesse komplementären Sequenz ermöglicht,
wobei die Sequenz A am 5'-Ende mit einer an A/T reichen, insbesondere an T reichen, ersten Sequenz mit einer Länge von 40 bis 60 Nukleotiden verbunden ist und am 3'-Ende mit einer an A/T reichen, insbesondere an T reichen, zweiten Sequenz mit einer Länge von 40 bis 60 Nukleotiden verbunden ist, wobei die an A/T reiche, insbesondere an T reiche, erste und zweite Sequenz jeweils eine an G/C reiche erste und eine zweite Domäne von 6 bis 12 Nukleotiden umfassen, wobei die Sequenz der ersten Domäne komplementär zur Sequenz der zweiten Domäne ist, wobei die erste und die zweite Domäne 15 bis 52 Nukleotide von der Sequenz A entfernt positioniert sind, wobei das erste Molekül an seinem 5'-Ende eine erste Erkennungssequenz einer Transposase mit einer Ausrichtung 5'-3' und an seinem 3'-Ende mindestens eine zweite Erkennungssequenz der Transposase umfasst; und
- ein zweites einzelsträngiges Nukleinsäuremolekül, das an seinem 5'-Ende mindestens eine Sequenz umfasst oder im Wesentlichen daraus besteht, die zu der zweiten Erkennungssequenz der Transposase komplementär ist,
wobei der Komplex so beschaffen ist, dass das erste und das zweite einzelsträngige Nukleinsäuremolekül entsprechend der von Watson und Crick definierten Basenkomplementarität derart gepaart sind, dass sie zwei doppelsträngige Bindungsstellen für die Transposase definieren.

2. Komplex nach Anspruch 1, wobei die Sequenz A eine zu einer Nukleinsäure von Interesse komplementäre Sequenz umfasst.

3. Komplex nach einem der Ansprüche 1 bis 2, wobei das erste Molekül an seinem 5'-Ende eine erste Erkennungssequenz einer Transposase mit einer 5'-3'-Ausrichtung und an seinem 3'-Ende eine zweite Erkennungssequenz der Transposase mit einer 5'-3'-Ausrichtung umfasst und
wobei das zweite Molekül an seinem 5'-Ende eine erste Sequenz, die komplementär zur ersten Erkennungssequenz der Transposase ist, gefolgt von einer zweiten Sequenz, die komplementär zur zweiten Erkennungssequenz der Transposase ist, umfasst.

4. Komplex nach einem der Ansprüche 1 bis 3, wobei das erste Molekül an seinem 5'-Ende eine erste Erkennungssequenz einer Transposase mit einer 5'-3'-Ausrichtung und an seinem 3'-Ende eine zweite Erkennungssequenz der Transposase, gefolgt von einer ersten Sequenz, die zur ersten Erkennungssequenz der Transposase komplementär ist, umfasst und
wobei das zweite Molekül an seinem 5'-Ende eine Sequenz umfasst, die komplementär zu der zweiten Erkennungssequenz der Transposase ist.

5. Komplex nach einem der Ansprüche 1 bis 4, wobei die Transposase eine bakterielle Transposase, insbesondere eine aus Tn5, Tn9, Tn10 oder Tc1/Mariner ausgewählte Transposase, ist.

6. Komplex nach einem der Ansprüche 1 bis 5, wobei das erste Molekül, insbesondere über ein modifiziertes Nukleotid, an ein Enzym gekoppelt ist.

7. Komplex nach einem der Ansprüche 1 bis 6, wobei das erste Molekül eine der folgenden Sequenzen umfasst: SEQ ID NO: 436, SEQ ID NR: 440, SEQ ID NR: 443, SEQ ID NR: 444, SEQ ID NR: 448, SEQ ID NR: 449, SEQ ID NR: 450, SEQ ID NR: 454, SEQ ID NR: 455, SEQ ID NR: 456, SEQ ID NR: 457, SEQ ID NR: 458, SEQ ID NR: 462, SEQ ID NR: 463, SEQ ID NR: 464, SEQ ID NR: 465, SEQ ID NR: 466 und SEQ ID NR: 641.

8. Komplex nach einem der Ansprüche 1 bis 7, wobei der Komplex ein Paar aus einem ersten und einem zweiten Molekül umfasst, wobei das erste und das zweite Molekül die in Tabelle 2 definierten Sequenzen umfassen.

9. Komplex nach einem der Ansprüche 1 bis 6, wobei der Komplex eines der in Tabelle 4 definierten 300 Paare aus dem ersten und dem zweiten Molekül umfasst.

10. Gruppe, umfassend
- ein erstes einzelsträngiges Nukleinsäuremolekül, das eine Sequenz A umfasst oder im Wesentlichen daraus besteht, die die Insertion einer zu einer Nukleinsäure von Interesse komplementären Sequenz ermöglicht, oder das eine zu einer Nukleinsäure von Interesse komplementäre Sequenz umfasst, wobei die komplementäre Sequenz am 5'-Ende mit einer an A/T reichen, insbesondere an T reichen, ersten Sequenz mit einer Länge von 40 bis 60 Nukleotiden verbunden ist und am 3'-Ende mit einer an A/T reichen, insbesondere an T reichen, zweiten Sequenz mit einer Länge von 40 bis 60 Nukleotiden verbunden ist, wobei die an A/T reiche, insbesondere an T reiche, erste und zweite Sequenz jeweils eine an G/C reiche erste und eine zweite Domäne von 6 bis 12 Nukleotiden umfassen, wobei die Sequenz der ersten Domäne komplementär zur Sequenz der zweiten Domäne ist, wobei die erste und die zweite Domäne 15 bis 52 Nukleotide von der Sequenz A entfernt positioniert sind, wobei das erste Molekül an seinem 5'-Ende mindestens eine erste Erkennungssequenz einer Transposase mit einer Ausrichtung 5'-3' und an seinem 3'-Ende eine zweite Erkennungssequenz der Transposase umfasst,
- ein zweites einzelsträngiges Nukleinsäuremolekül, das eine Sequenz B umfasst oder im Wesentlichen daraus besteht, die die Insertion einer zu einer Nukleinsäure von Interesse komplementären Sequenz ermöglicht, oder das eine zu einer Nukleinsäure von Interesse komplementäre Sequenz umfasst, wobei die komplementäre Sequenz B am 5'-Ende mit einer an T reichen, dritten Sequenz mit einer Länge von 40 bis 60 Nukleotiden verbunden ist, und am 3'-Ende mit einer an T reichen, vierten Sequenz mit einer Länge von 40 bis 60 Nukleotiden verbunden ist, wobei die an T reiche dritte und die vierte Sequenz jeweils eine an G/C reiche, dritte und vierte Domäne von 6 bis 12 Nukleotiden umfassen, wobei die Sequenz der dritten Domäne komplementär zur Sequenz der vierten Domäne ist, wobei die dritte und die vierte Domäne 15 bis 52 Nukleotide von der Sequenz B entfernt positioniert sind, wobei das zweite Molekül an seinem 5'-Ende mindestens die erste Erkennungssequenz einer Transposase mit der Ausrichtung 5'-3' und an seinem 3'-Ende die zweite Erkennungssequenz der Transposase umfasst,
wobei die Sequenz B eine zu der Nukleinsäure von Interesse komplementäre Sequenz ist, die Sequenz A am 5'-Ende von einer Region von Interesse der Nukleinsäure von Interesse positioniert ist und die Sequenz B am 3'-Ende von der Region von Interesse der Nukleinsäure von Interesse positioniert ist, und
- ein drittes einzelsträngiges Molekül, umfassend
mindestens eine Sequenz in seinem 5'-Bereich, die komplementär zu der zweiten Erkennungssequenz der Transposase des ersten Moleküls ist,
mindestens eine Sequenz in seinem 3'-Bereich, die komplementär zu der ersten Erkennungssequenz der Transposase des zweiten Moleküls ist, und eine Region, die sich zwischen der Sequenz, die komplementär zu der zweiten Erkennungssequenz der Transposase des ersten Moleküls ist, und der Sequenz, die komplementär zu der ersten Erkennungssequenz der Transposase des zweiten Moleküls ist, befindet, wodurch die Insertion eines einzelsträngigen Austauschnukleinsäuremoleküls ermöglicht wird, wobei das erste und das dritte einzelsträngige Nukleinsäuremolekül gemäß der von Watson und Crick definierten Basenkomplementarität derart gepaart sind, dass sie zwei doppelsträngige Bindungsstellen der Transposase definieren, und das zweite und das dritte einzelsträngige Nukleinsäuremolekül gemäß der von Watson und Crick definierten Basenkomplementarität derart gepaart sind, dass sie zwei doppelsträngige Bindungsstellen der Transposase definieren.

11. Gruppe nach Anspruch 10, wobei die Gruppe eines der in Tabelle 5 definierten 300 Paare aus dem ersten und dem dritten Molekül umfasst.

12. Kit, umfassend mindestens einen Vektor, der die Expression einer Rekombinase ermöglicht, und das erste, zweite und dritte Molekül der Gruppe, wie sie in Anspruch 10 definiert wird.

13. Verwendung der Gruppe, wie sie in Anspruch 10 definiert wird, für die Nukleinsäuretechnik, insbesondere für die Substitution einer Zielsequenz durch eine Sequenz von Interesse, vorausgesetzt, dass die Verwendung keinen Prozess zur Veränderung der genetischen Keimbahnidentität von Menschen umfasst und dass die Verwendung kein Verfahren zur chirurgischen oder therapeutischen Behandlung des menschlichen oder tierischen Körpers ist.

14. Verfahren zum Austauschen einer Zielregion eines Nukleinsäuremoleküls durch eine Region von Interesse eines anderen Nukleinsäuremoleküls, um derart ein hybrides Nukleinsäuremolekül zu erhalten, wobei das Verfahren umfasst:
- Inkontaktbringen einer Gruppe, wie sie in Anspruch 10 definiert wird, mit der Nukleinsäure, die die Zielregion umfasst,
wobei die Gruppe derart ist, dass
die Sequenz A des ersten Moleküls eine Sequenz umfasst, die komplementär zu der Region unmittelbar am 5'-Ende der Zielregion ist,
die Sequenz B des ersten Moleküls eine Sequenz umfasst, die komplementär zu der Region unmittelbar am 3'-Ende der Zielregion ist, und
das dritte Molekül die Region von Interesse in der Region umfasst, die sich zwischen der Sequenz, die komplementär zu der zweiten Erkennungssequenz der Transposase des ersten Moleküls ist, und der Sequenz, die komplementär zu der ersten Erkennungssequenz der Transposase des zweiten Moleküls ist, befindet, um derart einen Austauschkomplex zu erhalten,
- Zusammenbringen des Austauschkomplexes mit einer Transposase, die die in der Gruppe enthaltenen doppelsträngigen Bindungsstellen der Transposase erkennt, um derart einen Rekombinationskomplex zu erhalten, und
- Rekombination des Kombinationskomplexes, um derart das hybride Nukleinsäuremolekül zu erhalten, das die Region von Interesse anstelle der Zielregion umfasst, vorausgesetzt, dass es sich bei dem Verfahren nicht um einen Prozess zur Veränderung der genetischen Keimbahnidentität von Menschen handelt und dass es sich bei dem Verfahren nicht um ein Verfahren zur chirurgischen oder therapeutischen Behandlung des menschlichen oder tierischen Körpers handelt.

15. Verfahren zum Editieren des Genoms einer Zelle, das es ermöglicht, ein spezifisches doppelsträngigen DNA-Fragment des Genoms der Zelle durch ein anderes doppelsträngiges DNA-Fragment von Interesse zu ersetzen, um derart ein rekombinantes Hybridgenom zu erhalten, das anstelle des spezifischen doppelsträngigen DNA-Fragments das andere doppelsträngige DNA-Fragment von Interesse umfasst, wobei das Verfahren umfasst:
- Herstellen einer ersten Gruppe, wie sie in Anspruch 10 definiert wird,
wobei die Sequenz A des ersten Moleküls eine Sequenz umfasst, die komplementär zur benachbarten Region am 5'-Ende des spezifischen Fragments ist;
wobei die Sequenz B des zweiten Moleküls eine Sequenz umfasst, die komplementär zur benachbarten Region am 3'-Ende des spezifischen Fragments ist; und
wobei das dritte Molekül zwischen der Region, die sich zwischen der Sequenz, die komplementär zu der zweiten Erkennungssequenz der Transposase des ersten Moleküls ist, und der Sequenz, die komplementär zu der ersten Erkennungssequenz der Transposase des zweiten Moleküls ist, befindet, eine Sequenz eines der Stränge des spezifischen Fragments umfasst;
- und gegebenenfalls Herstellen einer zweiten Gruppe, wie sie wie in Anspruch 10 definiert wird,
wobei die Sequenz A der komplementären Region des ersten Moleküls der zweiten Gruppe eine Sequenz umfasst, die zu der benachbarten Region am 5'-Ende des spezifischen Fragments komplementär ist;
wobei die Sequenz B der komplementären Region des zweiten Moleküls der zweiten Gruppe eine Sequenz umfasst, die zu der benachbarten Region am 3'-Ende des spezifischen Fragments komplementär ist; und
wobei das dritte Molekül der zweiten Gruppe zwischen der Region, die sich zwischen der Sequenz, die komplementär zu der zweiten Erkennungssequenz der Transposase des ersten Moleküls der zweiten Gruppe ist, und der Sequenz, die komplementär zu der ersten Erkennungssequenz der Transposase des zweiten Moleküls der zweiten Gruppe ist, befindet, die Sequenz des komplementären Strangs des spezifischen Fragments umfasst, das in der dritten Sequenz der ersten Gruppe enthalten ist;
wobei die Sequenz, die komplementär zu der benachbarten Region am 5'-Ende des spezifischen Fragments ist, das in Region A des ersten Moleküls der ersten Gruppe enthalten ist, höchstens zu 95 % komplementär zur Sequenz ist, die komplementär zu der benachbarten Region am 5'-Ende des spezifischen Fragments ist, das in Region A des ersten Moleküls der zweiten Gruppe enthalten ist; und
wobei die Sequenz, die komplementär zu der benachbarten Region am 3'-Ende des spezifischen Fragments, das in Region B des ersten Moleküls der ersten Gruppe enthalten ist, höchstens zu 95 % komplementär zur Sequenz ist, die komplementär zu der benachbarten Region am 3'-Ende des spezifischen Fragments ist, das in Region B des ersten Moleküls der zweiten Gruppe enthalten ist;
um derart einen Rekombinationskomplex zu erhalten;
- Inkontaktbringen der Zelle mit dem Rekombinationskomplex, um derart eine zum Editieren bereite Zelle zu erhalten,
- Expression der Transposase in der zum Editieren bereiten Zelle, um derart eine editierte Zelle zu erhalten,
- Auswählen der editierten Zelle, wobei das Genom der editierten Zelle anstelle des spezifischen doppelsträngigen DNA-Fragments das andere doppelsträngige DNA-Fragment von Interesse umfasst, vorausgesetzt, dass es sich bei dem Verfahren nicht um einen Prozess zur Veränderung der genetischen Keimbahnidentität von Menschen handelt und dass es sich bei dem Verfahren nicht um ein Verfahren zur chirurgischen oder therapeutischen Behandlung des menschlichen oder tierischen Körpers handelt.

## Claims

1. A complex comprising
- a first single-stranded nucleic acid molecule comprising or consisting essentially of an A sequence allowing the insertion of a complementary sequence of a nucleic acid of interest,
said A sequence being linked at its 5'-end to a first A/T-rich, preferably T-rich, sequence of 40 to 60 nucleotides in length and at its 3'-end to a second A/T-rich, preferably T-rich, sequence of 40 to 60 nucleotides in length, sid first and second A/T-rich, preferably T-rich, sequences respectively comprising a first and a second G/C-rich domain of 6 to 12 nucleotides, the sequence of the first domain being complementary to the sequence of the second domain, the first and the second domains being positioned 15 to 52 nucleotides from said A sequence, said first molecule comprising at its 5'-end the first sequence oriented 5'-to-3' for recognizing a transposase and at its 3'-end at least the second sequence for recognizing the transposase; and
- a second single-stranded nucleic acid molecule comprising or consisting essentially at its 5' end of at least the complementary sequence of the second sequence for recognizing the transposase
the complex being such that the first and second single-stranded nucleic acid molecules are paired according to the base complementarity defined by Watson and Crick so as to define two double-stranded binding sites of a transposase.

2. The complex according to claim 1, wherein the A sequence comprises a complementary sequence of the nucleic acid of interest.

3. The complex according to claim 1 or 2, wherein the first molecule comprises at its 5' end the first sequence oriented 5'-to-3' for recognizing the transposase and at its 3' end the second sequence oriented 5'-to-3' for recognizing the transposase and
wherein the second molecule comprises its 5' end the first complementary sequence of the first sequence for recognizing the transposase followed by the second complementary sequence of the second sequence for recognizing the transposase.

4. The complex according to anyone of claims 1 to 3, wherein said firstmolecule comprises at its 5' end a first sequence oriented 5'-to-3' for recognizing the transposase and at its 3' end the second sequence for recognizing the transposase, followed by the first complementary sequence of the first sequence for recognizing the transposase and
wherein the second molecule comprises at its 5' end the complementary sequence of the second sequence for recognizing the transposase.

5. The complex according to anyone of claims 1 to 4, wherein the transposase is a bacterial transposase, preferably a transposase selected from the group consisting of Tn5, Tn9, Tn10 or Tc1/mariner.

6. The complex according to anyone of claims 1 to 5, wherein the first molecule is coupled with an enzyme, preferably through a modified nucleotide.

7. The complex according to anyone of claims 1 to 6, wherein the first molecule comprises one of the following sequences : SEQ ID NO: 436, SEQ ID NO: 440, SEQ ID NO: 443, SEQ ID NO: 444, SEQ ID NO: 448, SEQ ID NO: 449, SEQ ID NO: 450, SEQ ID NO: 454, SEQ ID NO: 455, SEQ ID NO: 456, SEQ ID NO: 457, SEQ ID NO: 458, SEQ ID NO: 462, SEQ ID NO: 463, SEQ ID NO: 464, SEQ ID NO: 465, SEQ ID NO: 466 and SEQ ID NO: 641.

8. The complex according to anyone of claims 1 to 7, the complex comprising a pair of first and second molecules, the first and the second molecules comprising the sequences as defined in table 2.

9. The complex according to anyone of claims 1 to 8, the complex comprising one of the 300 pairs of the first and the second molecules as defined in table 4.

10. An ensemble comprising
- a first single-stranded nucleic acid molecule comprising or consisting essentially of the A sequence allowing the insertion of a complementary sequence of a nucleic acid of interest, or comprising a complementary sequence of a nucleic acid of interest, the complementary sequence binding at 5' to a first T-rich sequence of 40 to 60 nucleotides in length and at 3' to a second T-rich sequence of 40 to 60 nucleotides in length, the first and second T-rich sequences respectively comprising a first and a second G/C-rich domain of 6 to 12 nucleotides, the sequence of the first domain being complementary to the sequence of the second domain, the first and second domains being positioned 15 to 52 nucleotides from the A sequence, the first molecule comprising at its 5' end at least the first sequence oriented 5'-to-3' for recognizing the transposase and at its 3' end the second sequence for recognizing the transposase,
- a second single-stranded nucleic acid molecule comprising or consisting essentially of a B sequence allowing the insertion of a complementary sequence of the nucleic acid of interest, or comprising a complementary sequence of a nucleic acid of interest, the complementary B sequence binding at 5' to a third T-rich sequence of 40 to 60 nucleotides in length and at 3' to a fourth T-rich sequence of 40 to 60 nucleotides in length, the third and fourth T-rich sequence respectively comprising a third and a fourth G/C-rich domain of 6 to 12 nucleotides, the sequence of the third domain being complementary to the sequence of the fourth domain, the third and fourth domains being positioned 15 to 52 nucleotides from the B sequence, the second molecule comprising at its 5' end at least the first sequence oriented 5'-to-3' for recognizing the transposase and at its 3' end the second sequence for recognizing said transposase,
the B sequence being a complementary sequence of the nucleic acid of interest, the A sequence being positioned at 5' end of a region of interest of the nucleic acid of interest and the B sequence being positioned at 3' end of the region of interest of the nucleic acid of interest, and
- a third single-stranded molecule comprising
in its 5' part, at least the complementary sequence of said second sequence for recognizing the transposase of the first molecule,
in its 3' part, at least the complementary sequence of said first sequence for recognizing the transposase of the second molecule, and
a region located between complementary sequence of the second sequence for recognizing the transposase of the first molecule and the complementary sequence of the first sequence for recognizing the transposase of the second molecule allowing the insertion of a single-stranded replacement nucleic acid molecule
the first and third single-stranded nucleic acid molecules are partially paired according to the base complementarity defined by Watson and Crick so as to define two double-stranded binding sites of a transposase.

11. The ensemble according to claim 10, the ensemble comprising one of the 300 pairs of first and third molecules as defined in table 5.

12. A kit comprising at least one vector allowing the expression of a recombinase and the first, second, and third molecules of the ensemble as defined in claim 10.

13. Use of the ensemble as defined in claim 10, for engineering nucleic acid molecules, in particular

14. A method for replacement of a target region of a nucleic acid molecule with a region of interest of another nucleic acid molecule, so as to obtain a hybrid nucleic acid molecule, said method comprising:
- bringing an ensemble as defined in claim 10 into contact with the nucleic acid comprising the target region,
said ensemble being such that
the A sequence of the first molecule comprises a complementary sequence of the region immediately at 5' end of the target region,
the B sequence of said second molecule comprises a complementary sequence of the region immediately at 3' end of the target region, and
the third molecule comprises the region of interest in the region located between complementary sequence of the second sequence for recognizing the transposase of the first molecule and the complementary sequence of the first sequence for recognizing the transposase of the second molecule, in order to obtain a replacement complex,
- placing the replacement complex in the presence of a transposase recognizing the double-stranded binding sites the said transposase contained in the ensemble, to obtain a recombination complex, and
- recombining the combination complex in order to obtain a hybrid nucleic acid molecule comprising the region of interest instead of the target region, provided that said method is not a method for modifying the genetic identity of human beings and that said method is not a method for treatment of the human or animal body by surgery or therapy.

15. A method for editing the genome of a cell, making it possible to replace a specific fragment of the double-stranded DNA of said genome of said cell with another double-stranded DNA fragment of interest, in order to obtain a recombinant hybrid genome comprising the other double-stranded DNA fragment of interest instead of the specific fragment of double-stranded DNA, said method comprising:
- preparing a first ensemble as defined in claim 10,
wherein the A sequence of the first molecule comprises a complementary sequence of the adjacent region at 5' of the specific fragment;
wherein the B sequence of the second molecule comprises a complementary sequence of the adjacent region at 3' of the specific fragment; and
wherein the third molecule comprises, between the region located between complementary sequence of said second sequence for recognizing said transposase of the first molecule and the complementary sequence of said first sequence for recognizing said transposase of the second molecule, a sequence of one of the strands of said specific fragment;
- and possibly preparing a second ensemble,
wherein the A sequence of said complementary region of the first molecule of the second ensemble comprises a complementary sequence of the adjacent region at 5' of the specific fragment;
wherein the B sequence of said complementary region of the second molecule of the second ensemble comprises a complementary sequence of the adjacent region at 3' of the specific fragment; and
wherein the third molecule of the second ensemble comprises, between the region located between complementary sequence of said second sequence for recognizing said transposase of the first molecule of the second ensemble and the complementary sequence of said first sequence for recognizing said transposase of the second molecule of the second ensemble, the sequence of the complementary strand of said specific fragment contained in the third sequence of the first ensemble;
wherein the complementary sequence of the adjacent region at 5' of the specific fragment contained in the A region of the first molecule of the first ensemble is at most 95 % complementary to the complementary sequence of the adjacent region at 5' of the specific fragment contained in the A region of the first molecule of the second ensemble; and
wherein the complementary sequence of the adjacent region at 3' of the specific fragment contained in the B region of the first molecule of the first ensemble is at most 95 % complementary to the complementary sequence of the adjacent region at 3' of the specific fragment contained in the B region of the first molecule of the second ensemble;
in order to obtain a recombination complex;
- bringing said cell into contact with said recombination complex, in order to obtain a cell ready to be edited,
- expressing the transposase in the cell ready to be edited, in order to obtain an edited cell,
selecting the edited cell, wherein the genome of the edited cell comprises, instead of the specific double-stranded DNA fragment, the other double-stranded DNA fragment of interest, provided that said method is not a method for modifying the genetic identity of human beings and that said method is not a method for treatment of the human or animal body by surgery or therapy.
